(11)  **EP 1 606 389 B1**

(12)  # EUROPEAN PATENT SPECIFICATION

(45) Date of publication and mention
of the grant of the patent:
**12.11.2008 Bulletin 2008/46**

(21) Application number: **04718119.3**

(22) Date of filing: **05.03.2004**

(51) Int Cl.:
***C12Q 1/68*** (2006.01)

(86) International application number:
**PCT/US2004/006948**

(87) International publication number:
**WO 2004/081182 (23.09.2004 Gazette 2004/39)**

(54) **METHODS FOR POLYNUCLEOTIDE SEQUENCE DETECTION**

VERFAHREN ZUM NACHWEIS VON POLYNUKLEOTIDSEQUENZEN

METHODES DE DETECTION DE SEQUENCES POLYNUCLEOTIDIQUES

(84) Designated Contracting States:
**AT BE BG CH CY CZ DE DK EE ES FI FR GB GR
HU IE IT LI LU MC NL PL PT RO SE SI SK TR**

(30) Priority: **06.03.2003 US 452481 P
12.05.2003 US 436231**

(43) Date of publication of application:
**21.12.2005 Bulletin 2005/51**

(73) Proprietor: **Stratagene California
La Jolla, CA 92037 (US)**

(72) Inventors:
• **SORGE, Joseph, A.
Wilson, WY 83014 (US)**
• **FIRMIN, Andrew
Jackson, WY 83002 (US)**

(74) Representative: **De Clercq, Ann G. Y.
De Clercq, Brants & Partners c.v.
Edgard Gevaertdreef 10a
9830 Sint-Martens-Latem (BE)**

(56) References cited:
WO-A2-01/94546        US-B1- 6 255 083
US-B1- 6 263 286      US-B1- 6 461 817

• **PASTINEN T ET AL: "A system for specific, high-
throughput genotyping by allele-specific primer
extension on microarrays" GENOME
RESEARCH, COLD SPRING HARBOR
LABORATORY PRESS, WOODBURY, NY, US, vol.
10, no. 7, July 2000 (2000-07), pages 1031-1042,
XP008013561 ISSN: 1088-9051**
• **GERMER S ET AL: "HIGH-THROUGHPUT SNP
ALLELE-FREQUENCY DETERMINATION IN
POOLED DNA SAMPLES BY KINETIC PCR"
GENOME RESEARCH, COLD SPRING HARBOR
LABORATORY PRESS, WOODBURY, NY, US, vol.
10, no. 2, February 2000 (2000-02), pages 258-266,
XP000927195 ISSN: 1088-9051**
• **HEID C A ET AL: "REAL TIME QUANTITATIVE
PCR" GENOME RESEARCH, COLD SPRING
HARBOR LABORATORY PRESS, WOODBURY,
NY, US, vol. 6, no. 10, October 1996 (1996-10),
pages 986-994, XP000642795 ISSN: 1088-9051**

**EP 1 606 389 B1**

SEQUENCE LISTING

<110> Stratagene
Sorge, Joseph A
Firmin, Andrew

<120> COMPOSITIONS AND METHODS FOR POLYNUCLEOTIDE SEQUENCE DETECTION

<130> 25436/2392

<140> US 10/436,231
<141> 2003-05-12

<150> US 60/452,481
<151> 2003-03-06

<160> 29

<170> PatentIn version 3.2

<210> 1
<211> 18
<212> DNA
<213> Artificial Sequence

<220>
<223> Example Allele A comprising tandem repeats

<400> 1

aaacagcagc agcagcag        18


<210> 2
<211> 18
<212> DNA
<213> Artificial Sequence


<220>
<223> Example Allele A comprising tandem repeats

<400> 2
ctgctgctgc tgctgttt        18


<210> 3
<211> 12
<212> DNA
<213> Artificial Sequence


<220>
<223> Example Allele B comprising tandem repeats

<400> 3
aaacagcagc ag        12


<210> 4
<211> 12
<212> DNA
<213> Artificial Sequence


<220>
<223> Example Allele B comprising tandem repeats

<400> 4
ctgctgctgt tt        12


<210> 5
<211> 18
<212> DNA
<213> Artificial Sequence


<220>
<223> Example Allele A comprising tandem repeats

<400> 5
cagcagcagc agcagccc        18


<210> 6
<211> 18
<212> DNA
<213> Artificial Sequence


<220>
<223> Example Allele A comprising tandem repeats

<400> 6
gggctgctgc tgctgctg        18


<210> 7

<211> 12
<212> DNA
<213> Artificial Sequence

<220>
<223> Example Allele B comprising tandem repeats

<400> 7
cagcagcagc cc          12

<210> 8
<211> 12
<212> DNA
<213> Artificial Sequence

<220>
<223> Example Allele B comprising tandem repeats

<400> 8
gggctgctgc tg          12

<210> 9
<211> 9
<212> DNA
<213> Artificial Sequence

<220>
<223> Example Allele 1 upstream primer

<400> 9
cctaggact          9

<210> 10
<211> 19
<212> DNA
<213> Artificial Sequence

<220>
<223> Example Allele 1

<400> 10
cctaggacta ccggcaagt          19

<210> 11
<211> 19
<212> DNA
<213> Artificial Sequence

<220>
<223> Example Allele 1

<400> 11
acttgccggt agtcctagg          19

<210> 12
<211> 10
<212> DNA
<213> Artificial Sequence

<220>
<223> Example Allele 1 downstream primer

<400> 12
acttgccggt          10

<210> 13
<211> 19
<212> DNA
<213> Artificial Sequence

<220>
<223> Example Allele 2

<400> 13
cctaggactt ccggcaagt          19

<210> 14
<211> 19
<212> DNA
<213> Artificial Sequence

<220>
<223> Example Allele 2

<400> 14
acttgccgga agtcctagg          19

<210> 15
<211> 10
<212> DNA
<213> Artificial Sequence

<220>
<223> Example Allele 2 downstream primer

<400> 15
acttgccgga          10

<210> 16
<211> 9
<212> DNA
<213> Artificial Sequence

<220>
<223> Example Allele 1 downstream primer

<400> 16
acttgccgg          9

<210> 17
<211> 19
<212> DNA
<213> Artificial Sequence

<220>
<223> Example Allele 2

<400> 17
cctaggactc ccggcaagt          19

<210> 18
<211> 19
<212> DNA
<213> Artificial Sequence

<220>
<223> Example Allele 2

<400> 18
acttgccggg agtcctagg          19

<210> 19
<211> 20
<212> DNA
<213> Artificial Sequence

<220>
<223> Fwd 79bp CCR2 primer

<400> 19
gttcatcttt ggttttgtgg          20

<210> 20
<211> 18
<212> DNA
<213> Artificial Sequence

<220>
<223> Rev 79bp CCR2 primer

<400> 20
gtcagtcaag cacttcag          18

<210> 21
<211> 27
<212> DNA
<213> Artificial Sequence

<220>
<223> Fwd 48bp CCR2 0G Mod Primer

<400> 21
actacttcaa ttttgtgggc aacatgc          27

<210> 22
<211> 34
<212> DNA
<213> Artificial Sequence

<220>
<223> Rev 48bp CCR2 0G Mod Primer

<400> 22
cacttaacac tttttgcagt ttattaagat gagg          34

<210> 23
<211> 27
<212> DNA
<213> Artificial Sequence

<220>
<223> Fwd 48bp CCR2 5G Mod Primer

<400> 23
aggagaggcc ttttgtgggc aacatgc        27

<210> 24
<211> 34
<212> DNA
<213> Artificial Sequence

<220>
<223> Rev 48bp CCR2 5G Mod Primer

<400> 24
cgtgaggtcg tttttgcagt ttattaagat gagg        34

<210> 25
<211> 17
<212> DNA
<213> Artificial Sequence

<220>
<223> FWD 48bp CCR2 Primer

<400> 25
ttttgtgggc aacatgc        17

<210> 26
<211> 24
<212> DNA
<213> Artificial Sequence

<220>
<223> REV 48bp CCR2 Primer

<400> 26
tttttgcagt ttattaagat gagg        24

<210> 27
<211> 27
<212> DNA
<213> Artificial Sequence

<220>
<223> Fwd CCR2 Wildtype 5G Mod Primer

<400> 27
ggatggacag gggcaacatg ctggtcg        27

<210> 28
<211> 27
<212> DNA

<213> Artificial Sequence

<220>
<223> Fwd CCR2 Variant 0G Mod Primer

<400> 28
ccaactctaa gggcaacatg ctggtca        27

<210> 29
<211> 9
<212> DNA
<213> Artificial Sequence

<220>
<223> Nucleotide sequence following the variable nucleotide in the upper strand of allele 2

<400> 29
ccggcaagt        9

SEQUENCE LISTING

<110> Stratagene California

<120> COMPOSITIONS AND METHODS FOR POLYNUCLEOTIDE SEQUENCE DETECTION

<130> STRAC-004-EP

<140> EP 04718119.3
<141> 2004-03-05

<150> US 10/436,231
<151> 2003-05-12

<150> US 60/452,481
<151> 2003-03-06

<160> 29

<170> PatentIn version 3.2

<210> 1
<211> 18
<212> DNA
<213> Artificial Sequence

<220>
<223> Example Allele A comprising tandem repeats

<400> 1
aaacagcagc agcagcag        18

<210> 2
<211> 18
<212> DNA
<213> Artificial Sequence

<220>
<223> Example Allele A comprising tandem repeats

<400> 2
ctgctgctgc tgctgttt          18

<210> 3
<211> 12
<212> DNA
<213> Artificial Sequence

<220>
<223> Example Allele B comprising tandem repeats

<400> 3
aaacagcagc ag          12

<210> 4
<211> 12
<212> DNA
<213> Artificial Sequence

<220>
<223> Example Allele B comprising tandem repeats

<400> 4
ctgctgctgt tt          12

<210> 5
<211> 18
<212> DNA
<213> Artificial Sequence

<220>
<223> Example Allele A comprising tandem repeats

<400> 5
cagcagcagc agcagccc          18

<210> 6
<211> 18
<212> DNA
<213> Artificial Sequence

<220>
<223> Example Allele A comprising tandem repeats

<400> 6
gggctgctgc tgctgctg          18

<210> 7
<211> 12
<212> DNA
<213> Artificial Sequence

<220>
<223> Example Allele B comprising tandem repeats

<400> 7
cagcagcagc cc          12


<210> 8
<211> 12
<212> DNA
<213> Artificial Sequence


<220>
<223> Example Allele B comprising tandem repeats


<400> 8
gggctgctgc tg          12


<210> 9
<211> 9
<212> DNA
<213> Artificial Sequence


<220>
<223> Example Allele 1 upstream primer


<400> 9
cctaggact          9


<210> 10
<211> 19
<212> DNA
<213> Artificial Sequence


<220>
<223> Example Allele 1


<400> 10
cctaggacta ccggcaagt          19


<210> 11
<211> 19
<212> DNA
<213> Artificial Sequence


<220>
<223> Example Allele 1


<400> 11
acttgccggt agtcctagg          19


<210> 12
<211> 10
<212> DNA
<213> Artificial Sequence


<220>
<223> Example Allele 1 downstream primer


<400> 12
acttgccggt          10

```
<210> 13
<211> 19
<212> DNA
<213> Artificial Sequence

<220>
<223> Example Allele 2

<400> 13
cctaggactt ccggcaagt          19

<210> 14
<211> 19
<212> DNA
<213> Artificial Sequence

<220>
<223> Example Allele 2

<400> 14
acttgccgga agtcctagg          19

<210> 15
<211> 10
<212> DNA
<213> Artificial Sequence

<220>
<223> Example Allele 2 downstream primer

<400> 15
acttgccgga          10

<210> 16
<211> 9
<212> DNA
<213> Artificial Sequence

<220>
<223> Example Allele 1 downstream primer

<400> 16
acttgccgg          9

<210> 17
<211> 19
<212> DNA
<213> Artificial Sequence

<220>
<223> Example Allele 2

<400> 17
cctaggactc ccggcaagt          19

<210> 18
<211> 19
<212> DNA
```

<213> Artificial Sequence

<220>
<223> Example Allele 2

<400> 18
acttgccggg agtcctagg          19

<210> 19
<211> 20
<212> DNA
<213> Artificial Sequence

<220>
<223> Fwd 79bp CCR2 primer

<400> 19
gttcatcttt ggttttgtgg        20

<210> 20
<211> 18
<212> DNA
<213> Artificial Sequence

<220>
<223> Rev 79bp CCR2 primer

<400> 20
gtcagtcaag cacttcag        18

<210> 21
<211> 27
<212> DNA
<213> Artificial Sequence

<220>
<223> Fwd 48bp CCR2 0G Mod Primer

<400> 21
actacttcaa ttttgtgggc aacatgc        27

<210> 22
<211> 34
<212> DNA
<213> Artificial sequence

<220>
<223> Rev 48bp CCR2 0G Mod Primer

<400> 22
cacttaacac tttttgcagt ttattaagat gagg        34

<210> 23
<211> 27
<212> DNA
<213> Artificial Sequence

<220>

<223> Fwd 48bp CCR2 5G Mod Primer

<400> 23
aggagaggcc ttttgtgggc aacatgc          27

<210> 24
<211> 34
<212> DNA
<213> Artificial Sequence

<220>
<223> Rev 48bp CCR2 5G Mod Primer

<400> 24
cgtgaggtcg tttttgcagt ttattaagat gagg          34

<210> 25
<211> 17
<212> DNA
<213> Artificial sequence

<220>
<223> FWD 48bp CCR2 Primer

<400> 25
ttttgtgggc aacatgc          17

<210> 26
<211> 24
<212> DNA
<213> Artificial Sequence

<220>
<223> REV 48bp CCR2 Primer

<400> 26
tttttgcagt ttattaagat gagg          24

<210> 27
<211> 27
<212> DNA
<213> Artificial sequence

<220>
<223> Fwd CCR2 wildtype 5G Mod Primer

<400> 27
ggatggacag gggcaacatg ctggtcg          27

<210> 28
<211> 27
<212> DNA
<213> Artificial Sequence

<220>
<223> Fwd CCR2 Variant 0G Mod Primer

<400> 28

ccaactctaa gggcaacatg ctggtca          27

<210> 29
<211> 9
<212> DNA
<213> Artificial sequence

<220>
<223> Nucleotide sequence following the variable nucleotide in the upper strand of allele 2

<400> 29
ccggcaagt          9

**Description**

FIELD OF THE INVENTION

This invention relates to the field of polynucleotide sequence variation determination.

BACKGROUND

There are inherited regions of DNA that can vary from organism to organism, and individual to individual. Variations in DNA sequence between individuals include single polymorphisms (SNPs), mutations, and tandem repeats. Sequences with the highest degree of variations are very useful in the fields of forensics, epidemiology, infectious disease, population characterization, human gene mapping, identification of genes involved in disease, relationship testing, crop and animal breeding and identifying genes of interest. Genetic markers which are sufficiently polymorphic with respect to length or sequence have long been sought for use in identity applications, such as paternity testing and identification of tissue samples collected for forensic analysis.

DNA markers which are simple base substitutions, i.e., simple sequence polymorphisms, have been identified using Southern hybridization assays. For examples of references describing the identification of such markers, designed to be used to analyze restriction endonuclease-digested DNA with radioactive probes, see: Southern, E. M. (1975), J. Mol. Biol. 98(3):503-507; Schumm, et al. (1988), American Journal of Human Genetics 42:143-159; and Wyman, A. and White, R. (1980) Proc. Natl. Acad. Sci, U.S.A. 77:6754-6758.

DNA markers based on size variants, i.e., length polymorphisms, such as "variable number of tandem repeat" (VNTR) markers and polymorphic short tandem repeat (STRs) markers, also have been identified (Nakamura Y., et al. (1987), Science 235: 1616-1622; and U.S. Patent Nos. 4,963,663 and 5,411,859; (Jeffreys et al. (1985a), Nature 314: 67-73; Jeffreys et al. (1985b) Nature 316:76-79.; and U.S. Patent No. 5,175,082). Different individuals in a population may contain different numbers of these repeats. These markers are more highly polymorphic than base substitution polymorphisms, sometimes displaying up to forty or more alleles at a single genetic locus. The discovery and development of VNTRs and STRs as genetic markers have stimulated progress in the development of linkage maps, the identification and characterization of diseased genes, and the simplification and precision of DNA typing (Mizutani et al. (2001), J Hum Genet 46:448-55; Sprecher et al., (1996) Biotechniques, 20:266-76; Haaf et al., (1996) Nat. Genet. 12:183-5; Wooster et al., (1994), Nat. Genet. 6:152-6; Vergnaud (1989) Polynucleotides Res. 17:7623-30).

DNA markers which are polymorphic loci also have been identified by applying polymerase chain reaction (PCR) (U.S. Patent Nos. 4,683,202; 4,800,159; 5,468,613; and 5,604,099 by Mullis, K.) technology to the analysis of polymorphic loci (Pertl et al., (2000) Hum. Genet. 106:45-9; Deng et al., (2000) Biotechniques 29:298-304; Hohoff and Brinkmann, (1999) Mol. Biotechnol. 13:123-136; Sherlock et al., (1998) Ann. Hum. Genet. 62:9-23; Kasai K, et al. (1990) Journal Forensic Science 35(5):1196-1200; ). Amplifiable VNTR, STR or SNP loci were discovered, which could be detected without the need for Southern transfer. The amplified products are separated through agarose or polyacrylamide gels and detected by incorporation of radioactivity during the amplification or by post-staining with silver or ethidium bromide.

Several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A.-C., et al., Genomics 8:684-692 (1990); Kuppuswamy, M.N. et al.. Proc. Natl. Acad. Sci. (U.S.A.) 88: 1143-1147 (1991); Prezant, T.R. et al., Hum. Mutat. 1:159-164 (1992); Ugozzoli, L. et al GATA 9:107-112 (1992); Nyren, P. et al., Anal. Biochem. 208:171-175 (1993)). These methods all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.C., et al. Amer. J. Hum. Genet. 52:46 59, 1993).

Other patents and patent applications using primer extension for variation detection include U.S. Patent No. 6,013,431, PCT Application WO91/02087, and PCT Application WO90/09455 which utilize dideoxynucleotides; PCT Application WO89/10414 using allele specific primers; U.S. Patent No. 6,322,980 using degradation of a fluorescent sequence; U.S. Patent No. 6,287,778 using sequence-coded identity tags; PCT publication WO 00/11221 using a series of nucleotides (ddNTP and dNTPs), followed by identification of the incorporated ddNTP for the detection of SNPs; PCT application WO 96/06187 utilizing differentially labeled ddNTP followed by the separation of amplified products based on size or charge; and PCT publication WO 01/94546A2 utilizing one or more differentially labeled nucleotides and detecting the differential signals generated by the labeled nucleotides incorporated into the amplified products.

SUMMARY OF THE INVENTION

The present invention relates to compositions and methods for the detection of polynucleotide polymorphism and mutation. The embodiments of the invention include methods in which an extension reaction contains a labeled nucleotide. The incorporation frequency of the labeled nucleotide is measured to determine the presence or absence of a sequence variation between the two or more polynucleotides.

In one aspect, the specification describes a method for determining a sequence difference between a region of interest in a polynucleotide and a reference sequence, the method comprising: a) incubating the polynucleotide in a reaction mixture comprising a nucleotide labeled with a detectable label to produce a polynucleotide product from the polynucleotide; b) determining an incorporation frequency of the labeled nucleotide for the polynucleotide product; and c) comparing the incorporation frequency determined in step (b) with a known frequency for a reference sequence, wherein a difference in the two frequencies is indicative of a sequence difference between the region of interest of the polynucleotide and the reference sequence.

In one embodiment, step (b) comprises detecting the incorporation of the labeled nucleotide into the polynucleotide product. In another embodiment, step (b) comprises measuring the signal from incorporated labeled nucleotides and measuring the amount of the polynucleotide product. In a further embodiment, step (b) comprises measuring the signal from incorporated labeled nucleotides, measuring the amount of the polynucleotide product, and expressing the resulting values as a ratio of signal from incorporated nucleotides over the amount of the polynucleotide product. In another embodiment, the amount of the polynucleotide product is measured by polynucleotide staining. In another embodiment, SYBR Green can be used for the polynucleotide staining and ROX can be used to label the labeled nucleotide. A detectable signal can be generated by ROX on the labeled nucleotide incorporated into the polynucleotide product.

In another embodiment, the detectable label is one selected from the group consisting of: a fluorescent label, a fluorescence quencher, a colorimetric label, a chemiluminescent label, an isotope, a quantum dot label, an antigen, and an affinity moiety.

In another embodiment, the labeled nucleotide comprises a signal generating moiety and a signal quenching moiety wherein the signal quenching moiety quenches the signal from the signal generating moiety when both such moieties are present on the labeled nucleotide. In one embodiment, the signal quenching moiety is separated from the labeled nucleotide upon incorporation of the labeled nucleotide into the polynucleotide product.

In another embodiment, the step of determining an incorporation comprises computing the ratio of the signal generated by the incorporated labeled nucleotide and the signal generated by the polynucleotide stain.

In another embodiment, the polynucleotide product is linked to a solid support. In another embodiment, the number of potential linkage sites on each specimen of the solid support is substantially constant and the linked polynucleotide product saturates the potential linkage sites on the solid support. In another embodiment, a relative incorporation frequency is determined by measuring the amount of signal from incorporated nucleotides in polynucleotide product linked to the solid support.

In another embodiment, the reaction mixture further comprises a set of oligonucleotide primers which flank the region of interest.

In another embodiment, the reaction mixture further comprises nucleotides dATP, dGTP, dTTP, and dCTP, at least one of which is labeled with the detectable label.

In another embodiment, the polynucleotide product is an amplified product.

In another embodiment, the reaction mixture comprises a mixture of the nucleotide labeled with a detectable label and the same nucleotide not labeled with a detectable label. In another embodiment, the amount of the labeled nucleotide is 0.01 % to 5% of the total amount of the nucleotide, including labeled and unlabeled nucleotide of that kind. Where the presence of label on the nucleotide does not adversely affect enzyme incorporation of the labeled nucleotide, higher proportions can be used, even up to, for example, 100%.

In another embodiment, the sequence difference is at a predetermined nucleotide position within the region of interest in the polynucleotide.

In another embodiment, the sequence variation comprises a single nucleotide polymorphism or a variable number tandem repeat.

In another embodiment, the detectable label is a chemical label.

In another embodiment, the detectable label is one or more selected from the group consisting of: a fluorescent label, a fluorescence quencher, a colorimetric label, a chemiluminescent label, an isotope, a quantum dot label, an antigen, and an affinity moiety.

In another embodiment, the reaction mixture comprises two different nucleotides labeled with different detectable labels.

In another embodiment, the incorporation frequency for the polynucleotide is determined as a ratio between the level of incorporation of one labeled nucleotide and the level of incorporation of another labeled nucleotide into the same polynucleotide product.

In another embodiment, the reaction is an amplification reaction.

In another aspect, the specification describes a method for determining a sequence difference between a region of interest in a first polynucleotide and a corresponding region of interest in a second polynucleotide, the method comprising: a) incubating the first polynucleotide in a first reaction mixture comprising a nucleotide labeled with a detectable label to produce a first polynucleotide product from the first polynucleotide; b) determining an incorporation frequency of the labeled nucleotide for the first polynucleotide product; and c) comparing the incorporation frequency determined in step (b) with an incorporation frequency for the second polynucleotide, wherein a difference in the two incorporation frequencies is indicative of a sequence difference between the region of interest of the first polynucleotide and the corresponding region of interest of the second polynucleotide.

In one embodiment, the detectable label is one or more selected from the group consisting of: a fluorescent label, a fluorescence quencher, a colorimetric label, a chemiluminescent label, an isotope, a quantum dot label, an antigen, and an affinity moiety.

In another embodiment, the first reaction mixture further comprises nucleotides dATP, dGTP, dTTP, and dCTP, at least one of which is labeled with the detectable label.

In another embodiment, step (b) comprises detecting the incorporation of the labeled nucleotide into the polynucleotide product. In another embodiment, step (b) comprises measuring the signal from labeled nucleotides incorporated into the first polynucleotide product and measuring the amount of the first polynucleotide product. In another embodiment, step (b) comprises measuring the signal from labeled nucleotides incorporated into the first polynucleotide product, measuring the amount of the first polynucleotide product, and expressing the resulting values as a ratio of signal from incorporated nucleotides over the amount of the first polynucleotide product. In another embodiment, the amount of the first polynucleotide product is measured by polynucleotide staining. In another embodiment, SYBR is used for the polynucleotide staining and ROX is used to label the labeled nucleotide. A detectable signal can be generated by ROX on the labeled nucleotide incorporated into the polynucleotide product.

In another embodiment, the incorporation frequency for the second polynucleotide is determined by performing the steps of a)-b) for the second polynucleotide.

In another embodiment, the nucleotide labeled with the detectable label for the first polynucleotide is also used for the second polynucleotide.

In another embodiment, the sequence difference is at a predetermined nucleotide position within the region of interest in the first polynucleotide.

In another embodiment, the first reaction mixture further comprises a set of oligonucleotide primers which flank the region of interest.

In another embodiment, the first polynucleotide product is an amplified product.

In another embodiment, the reaction mixture comprises a mixture of the nucleotide labeled with a detectable label and the same nucleotide not labeled with a detectable label. In another embodiment, the amount of the labeled nucleotide is 0.01% to 25% of the total amount of the nucleotide, including labeled and unlabeled nucleotide of that kind. Where the presence of label on the nucleotide does not adversely affect enzyme incorporation of the labeled nucleotide, higher proportions can be used, even up to, for example, 100%.

In another embodiment, the sequence difference is at a predetermined nucleotide position within the region of interest in the polynucleotide.

In another embodiment, the sequence difference comprises a single nucleotide polymorphism or a tandem repeat.

In another embodiment, the reaction mixture comprises two or more nucleotides, each labeled with a different detectable label.

In another embodiment, the incorporation frequency for the polynucleotide is determined as a ratio between the level of incorporation of one labeled nucleotide and the level of incorporation of another labeled nucleotide into the same polynucleotide product.

In another embodiment, the reaction is an amplification reaction.

In another embodiment, the first polynucleotide product is linked to a solid support. In another embodiment, the number of potential linkage sites for the polynucleotide product on each specimen of the solid support is substantially constant, and the linked polynucleotide product saturates the potential linkage sites on the solid support. In another embodiment, the method further comprises the step of determining a relative incorporation frequency by measuring the amount of signal from incorporated nucleotides in polynucleotide product linked to the solid support. In another embodiment, the step of determining a relative incorporation frequency comprises computing the ratio of the signal generated by the incorporated labeled nucleotide and the signal generated by the polynucleotide stain.

In another embodiment, the labeled nucleotide comprises a signal generating moiety and a signal quenching moiety, wherein the signal quenching moiety quenches the signal from the signal generating moiety when both the moieties are present on the labeled nucleotide. In another embodiment, the signal quenching moiety is separated from the labeled nucleotide upon incorporation of the labeled nucleotide into the polynucleotide product.

In another aspect, the specification describes a method for determining the presence of a mutation in a region of interest in a polynucleotide, the method comprising: a) incubating the polynucleotide in a reaction mixture comprising a nucleotide labeled with a detectable label to produce a polynucleotide product from the polynucleotide; b) determining an incorporation frequency of the labeled nucleotide for the polynucleotide product; and c) comparing the incorporation frequency determined in step (b) with a known frequency for a reference wild-type sequence, wherein a difference in the two frequencies is indicative of the presence of a mutation in a region of interest in a polynucleotide. Each of the particular embodiments described above for the previous aspects can be used in this method where applicable.

The present specification further describes a method for genotyping comprising: a) incubating in a reaction mixture a first nucleic acid sample comprising a region of interest from a multiallelic species, the reaction mixture comprising a nucleotide labeled with a detectable label to produce a polynucleotide product from the nucleic acid sample; and b) measuring the level of incorporation of the labeled nucleotide in the product to determine an incorporation frequency of the labeled nucleotide for the polynucleotide product, where the ascertained incorporation frequency is indicative of the genotype of the multiallelic organism. Each of the particular embodiments described above for the previous aspects can be used in this method where applicable.

In one embodiment, the method further comprises, before step (b), the step of measuring the level of incorporation of the labeled nucleotide in the product.

In another embodiment, the reaction mixture further comprises a set of oligonucleotide primers which flank the region of interest.

In another embodiment, the reaction mixture further comprises nucleotides dATP, dGTP, dTTP, and dCTP, at least one of which is labeled with the detectable label.

In another embodiment, the product is an amplified product.

In another embodiment, the reaction is an amplification reaction.

In another embodiment, the incorporation frequency is indicative of whether the organism has a wild-type or a variant genotype.

In particular, the present invention relates to a method for determining a sequence difference between a region of interest in a first polynucleotide and a corresponding region of interest in a second polynucleotide, said method comprising:

a) incubating said first polynucleotide in a first reaction mixture comprising a primer and a nucleotide labeled with a detectable label to produce a first extended polynucleotide product comprising said primer from said first polynucleotide;

b) determining an incorporation frequency of said labeled nucleotide for said first extended polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency; and

c) comparing said incorporation frequency determined in step (b) with an incorporation frequency for said second polynucleotide,

wherein a difference in the two incorporation frequencies is indicative of a sequence difference between the region of interest of said first polynucleotide and the corresponding region of interest of said second polynucleotide.

The present invention relates also to a method as described herein, wherein said nucleotide labeled with said detectable label for said first polynucleotide is also used for said second polynucleotide.

The present invention relates also to a method as described herein, wherein said first reaction mixture further comprises nucleotides dATP, dGTP, dTTP, and dCTP, at least one of which is labeled with said detectable label.

The present invention relates also to a method as described herein, wherein said first polynucleotide product is linked to a solid support.

The present invention relates also to a method as described herein, wherein said amount of the first and/or second polynucleotide product is measured by polynucleotide staining with a polynucleotide stain.

The present invention relates also to a method as described herein, wherein said first reaction mixture further comprises a set of oligonucleotide primers which flank the region of interest.

The present invention relates also to a method as described herein, wherein said first polynucleotide product is an amplified product.

The present invention relates also to a method as described herein, wherein said sequence difference is at a predetermined nucleotide position within the region of interest in said polynucleotide, or comprises a single nucleotide polymorphism or a tandem repeat.

In addition, the present invention relates to a method for determining a sequence difference between a region of interest in a polynucleotide and a reference sequence, said method comprising:

a) incubating said polynucleotide in a reaction mixture comprising a primer and a nucleotide labeled with a detectable label to produce an extended polynucleotide product comprising said primer from said polynucleotide;
b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency; and

c) comparing said incorporation frequency determined in step (b) with a known frequency for said reference sequence,

wherein a difference in the two frequencies is indicative of a sequence difference between the region of interest of said polynucleotide and said reference sequence.

The present invention relates also to a method as described herein, wherein said sequence difference is at a predetermined nucleotide position within the region of interest in said polynucleotide, or comprises a single polymorphism or a tandem repeat. Moreover, the present invention relates to a method for determining the presence of a mutation in a region of interest in a polynucleotide, said method comprising:

a) incubating said polynucleotide in a reaction mixture comprising a primer and a nucleotide labeled with a detectable label, to produce an extended polynucleotide product comprising said primer from said polynucleotide;
b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency; and

c) comparing said incorporation frequency determined in step (b) with a known frequency for a reference wild-type sequence,

wherein a difference in the two frequencies is indicative of the presence of a mutation in a region of interest in a polynucleotide.

Also, the present invention relates to a method for genotyping comprising:

a) incubating in a reaction mixture a first nucleic acid sample comprising a region of interest, said reaction mixture comprising a primer and a nucleotide labeled with a detectable label to produce an extended polynucleotide product comprising said primer from said nucleic acid sample; and
b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency,

and wherein said incorporation frequency is indicative of the genotype of the organism from which said nucleic acid sample was obtained.

The present invention relates also to a method as described herein, wherein said incorporation frequency is indicative of whether said organism has a wild-type or a variant genotype.

The present invention relates also to a method as described herein, wherein said polynucleotide product is linked to a solid support.

The present invention relates also to a method as described herein, wherein said amount of the polynucleotide product is measured by polynucleotide staining with a polynucleotide stain.

The present invention relates also to a method as described herein, wherein said reaction mixture further comprises a set of oligonucleotide primers which flank the region of interest.

The present invention relates also to a method as described herein, wherein said reaction mixture further comprises nucleotides dATP, dGTP, dTTP, and dCTP, at least one of which is labeled with said detectable label.

The present invention relates also to a method as described herein, wherein said polynucleotide product is an amplified product.

The present invention relates also to a method as described herein, wherein the number of potential linkage sites on said solid support is substantially constant and said linked polynucleotide product saturates the potential linkage sites on said solid support.

The present invention relates also to a method as described herein, wherein said detectable label is one selected from the group consisting of: a fluorescent label, a fluorescence quencher, a colorimetric label, a chemiluminescent label, an isotope, a quantum dot label, an antigen, an affinity moiety, and a chemical label.

The present invention relates also to a method as described herein, wherein said labeled nucleotide comprises a signal generating moiety and a signal quenching moiety, wherein said signal quenching moiety quenches the signal from said signal generating moiety when both said moieties are present on said labeled nucleotide.

The present invention relates also to a method as described herein, wherein said signal quenching moiety is separated from said labeled nucleotide upon incorporation of said labeled nucleotide into said polynucleotide product.

The present invention relates also to a method as described herein, wherein SYBR is used for said polynucleotide staining, and ROX is used to label said labeled nucleotide.

The present invention relates also to a method as described herein, wherein a detectable signal is generated by ROX on said labeled nucleotide incorporated into the polynucleotide product.

The present invention relates also to a method as described herein, wherein said reaction mixture comprises a mixture of said nucleotide labeled with a detectable label and the same nucleotide not labeled with a detectable label.

The present invention relates also to a method as described herein, wherein the amount of said labeled nucleotide is 0.01% to 25%, preferably 0.5% to 25% of the total amount of said nucleotide, including labeled and unlabeled said nucleotide.

The present invention relates also to a method as described herein, wherein said reaction mixture comprises two different nucleotides labeled with different detectable labels.

The present invention relates also to a method as described herein, wherein said reaction is an amplification reaction.

BRIEF DESCRIPTION OF THE DRAWINGS

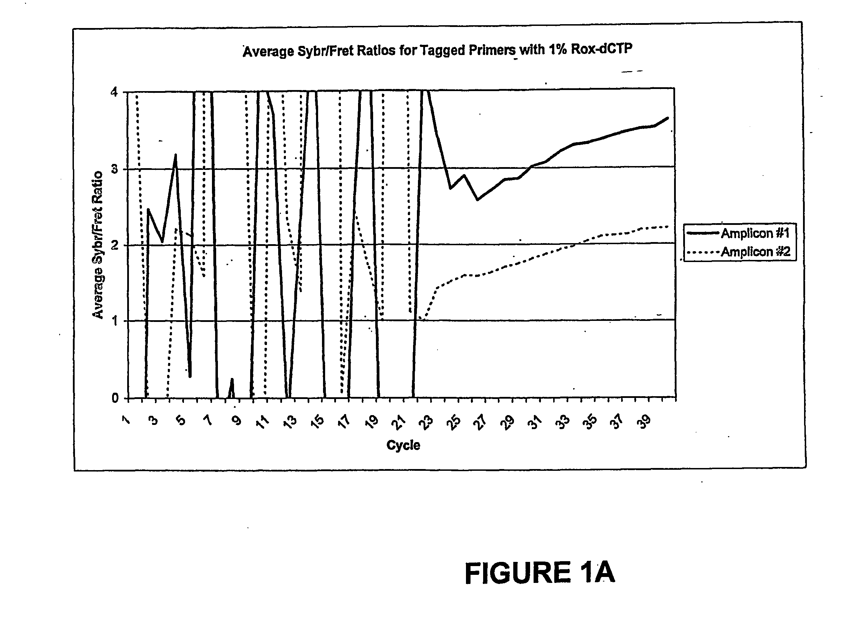

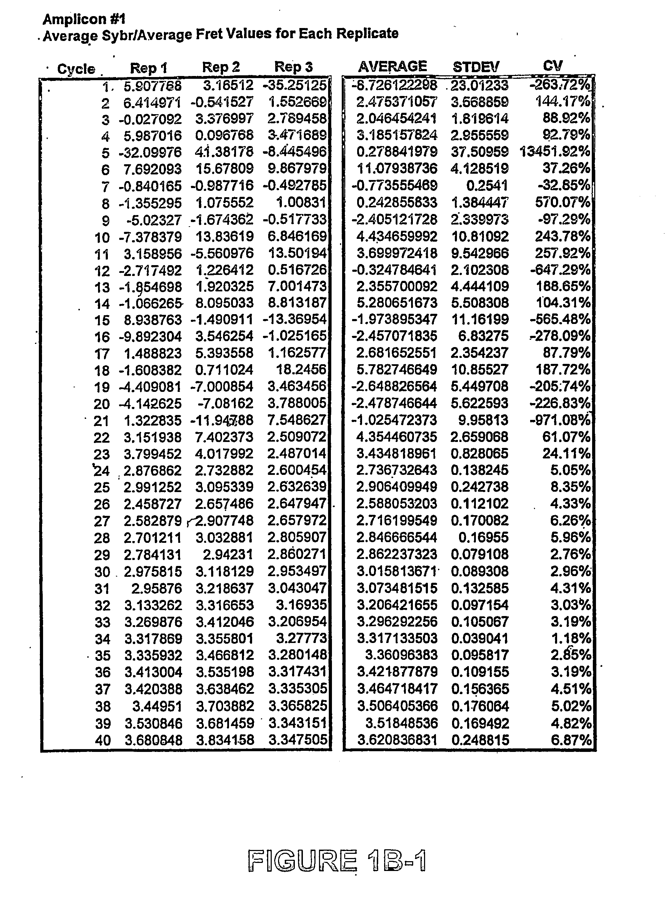

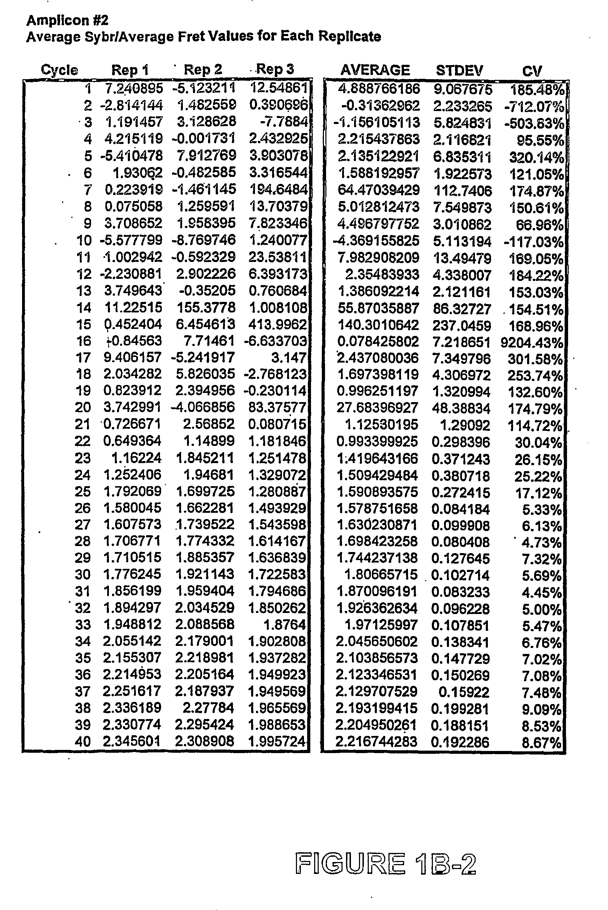

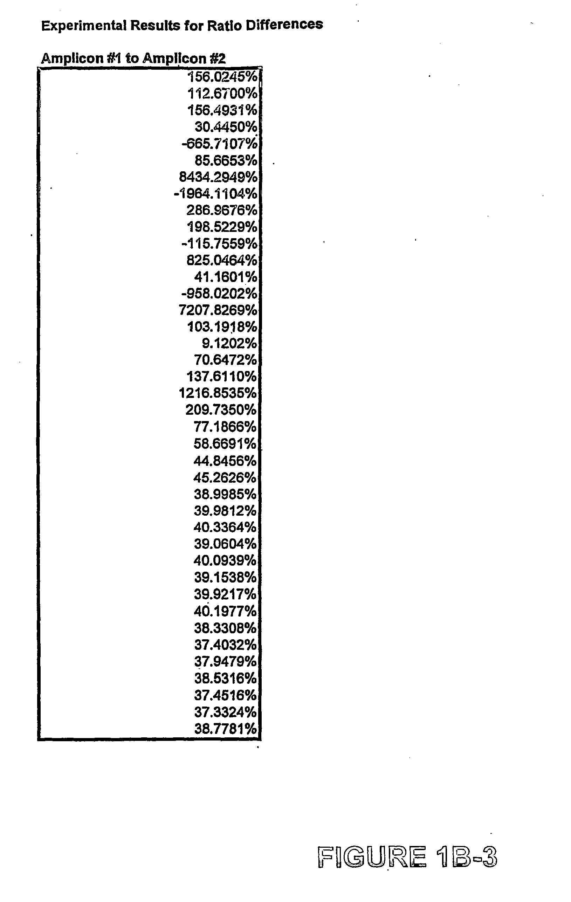

Figure 1, A and B, shows the results of experiments examining the correlation between differences in color ratios and differences in nucleotide incorporation ratios. Figure 1A shows a graph of the averaged values of the SYBR/FRET ratios for two experimental amplicons, termed Amplicon #s 1 (solid line) and 2 (dashed line), versus PCR cycle number. Labels were SYBR Green and 1% Rox-dCTP. Fluorescence data were obtained from three replicates during PCR cycling. Amplicon #1 corresponds to the product with 15 dCTP incorporation sites. Amplicon #2 corresponds to a product with 25 dCTP incorporation sites. The 79 bp CCR2 purified PCR product was used as template. The primers used were modified with 10bp tags that are used to artificially incorporate either 15 or 25 dCTP sites respectively. Amplicon #2 will incorporate more Rox-dCTP molecules than Amplicon #1, thus having a greater FRET signal and a lower SYBR Green/FRET ratio, as confirmed in the graph. Figure 1B shows the data graphed in Figure 1A.

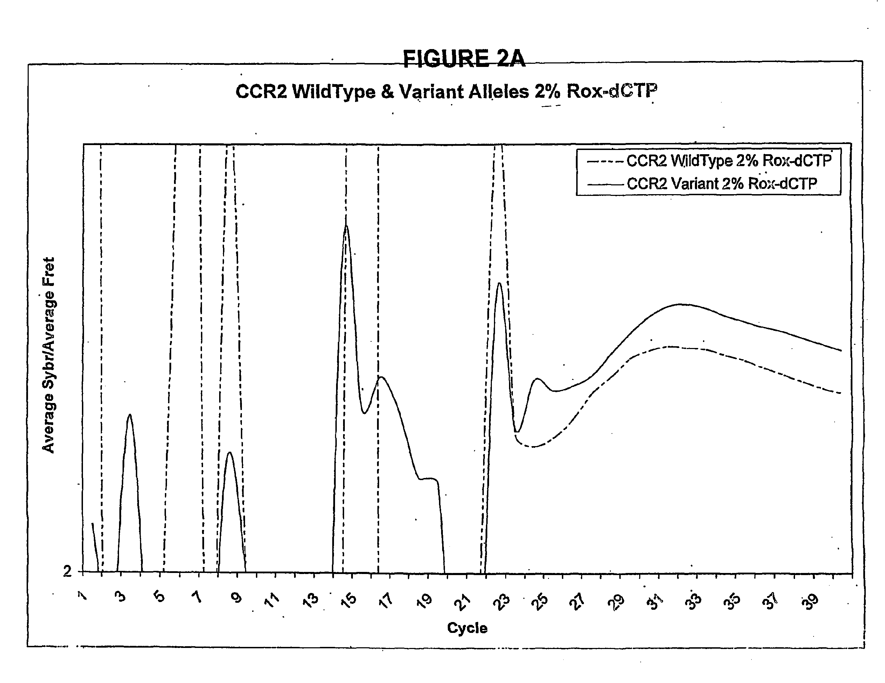

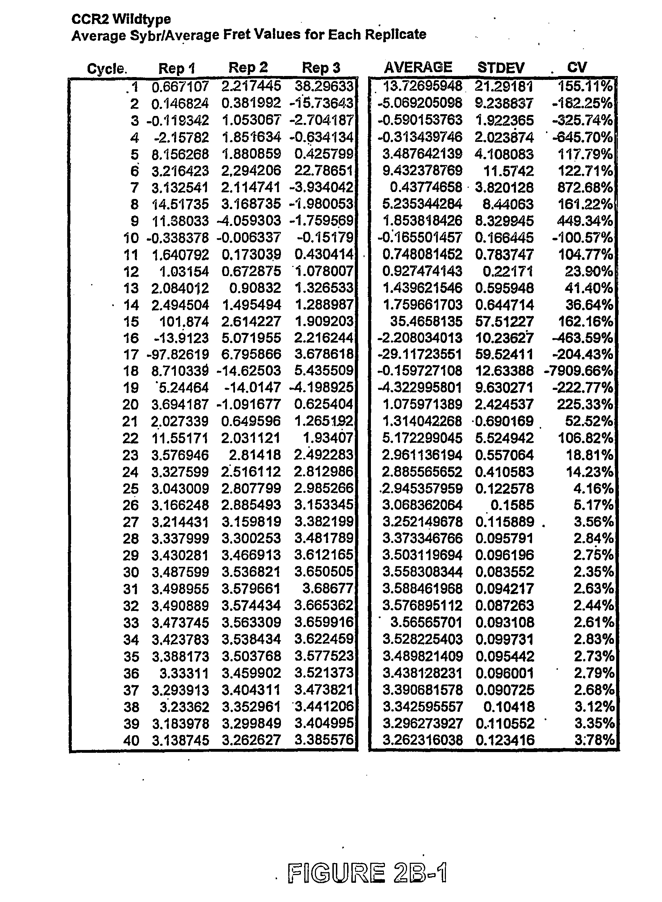

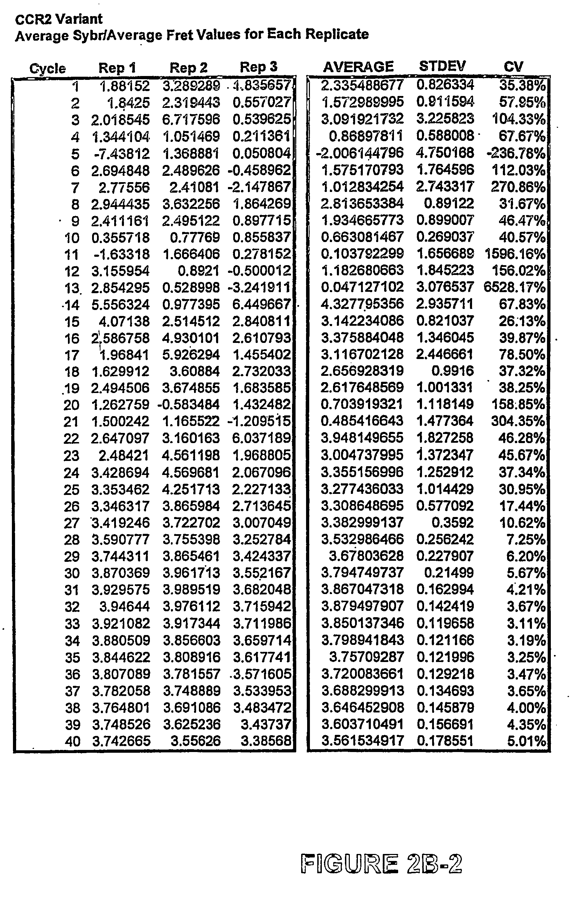

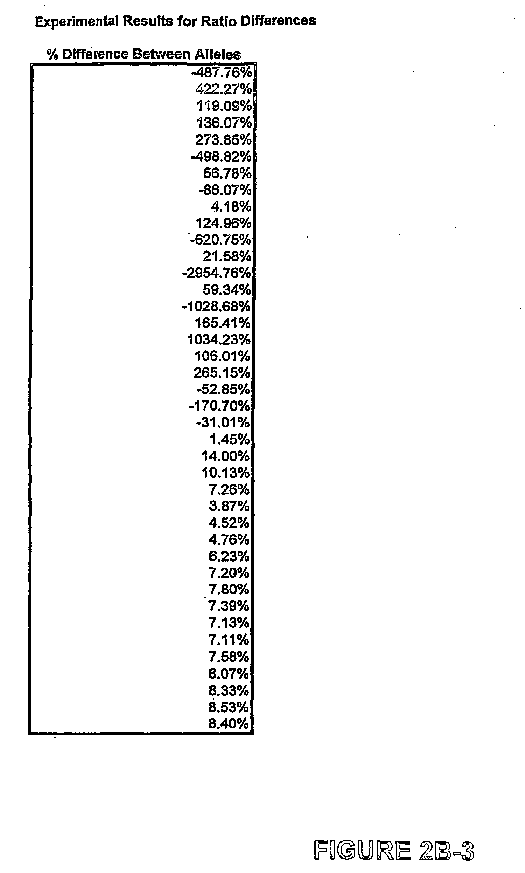

Figure 2, A and B, shows the SYBR/FRET ratio results of experiments using two templates with a single base difference (i.e., a SNP). Average SYBRGreen/FRET ratios for three replicate reactions were determined during amplification for CCR2 wildtype and variant alleles using Forward 48bp CCR2, and Reverse 48bp CCR2 primers with the purified 79bp PCR amplicon (wild-type or SNP variant) as template. Labels were SYBR Green and 2% Rox-dCTP. Figure 2A shows a graph of SYBR Green/FRET ratio for wildtype (dashed line) and variant (solid line) alleles versus cycle number. The ratio of SYBR Green/FRET fluorescence is higher for the variant allele. Figure 2B shows the data graphed in Figure 2A.

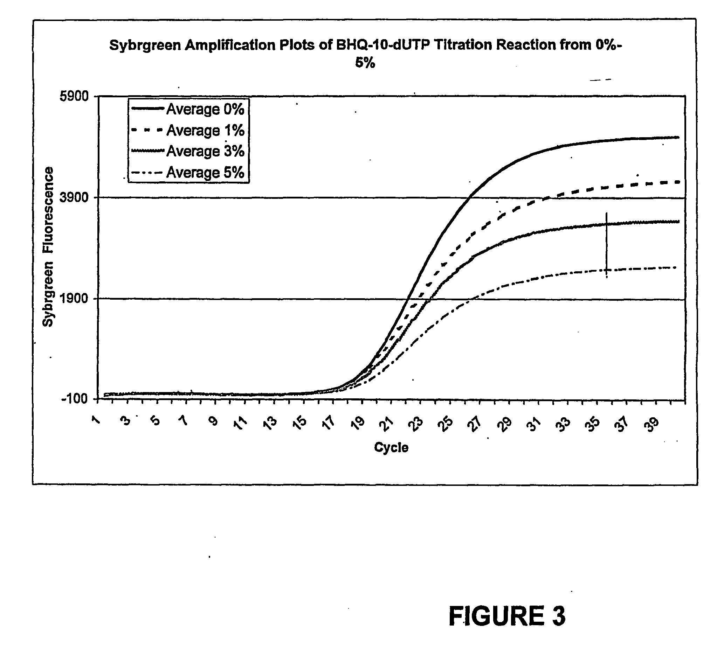

Figure 3 shows the results of experiments examining the effect of BHQ-10-dUTP on SYBR Green fluorescence. Four different PCR reactions were performed using the purified 79bp PCR product of the CCR2 variant allele with the Fwd 48 and Rev 48 primers used in Example 2 (Figure 2). SYBR Green concentration was held constant. The amount of BHQ-10-dUTP in the PCR reactions varied from 0% (0uM) to 5% (1uM). SYBR Green fluorescence is graphed versus cycle number. The data shown are averages of data from three replicates of each reaction.

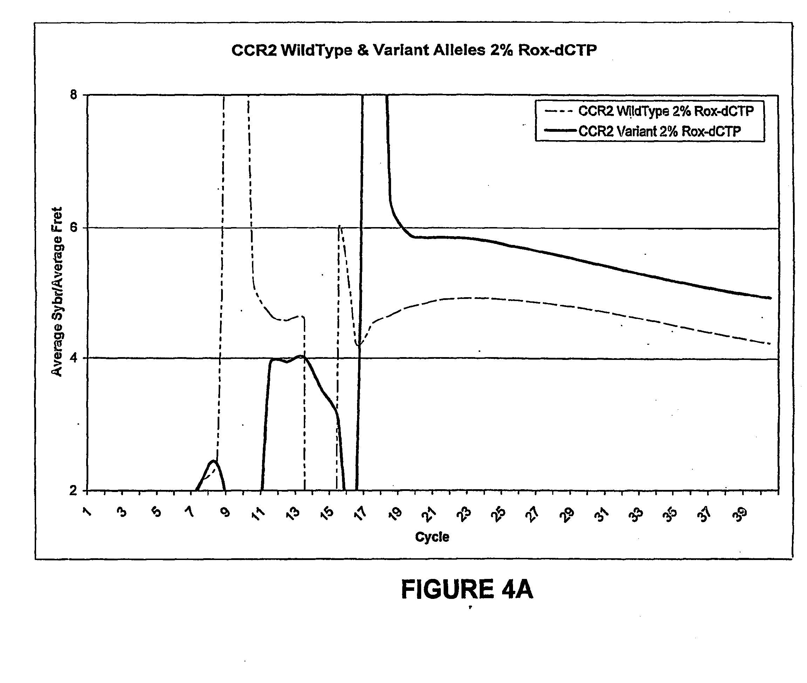

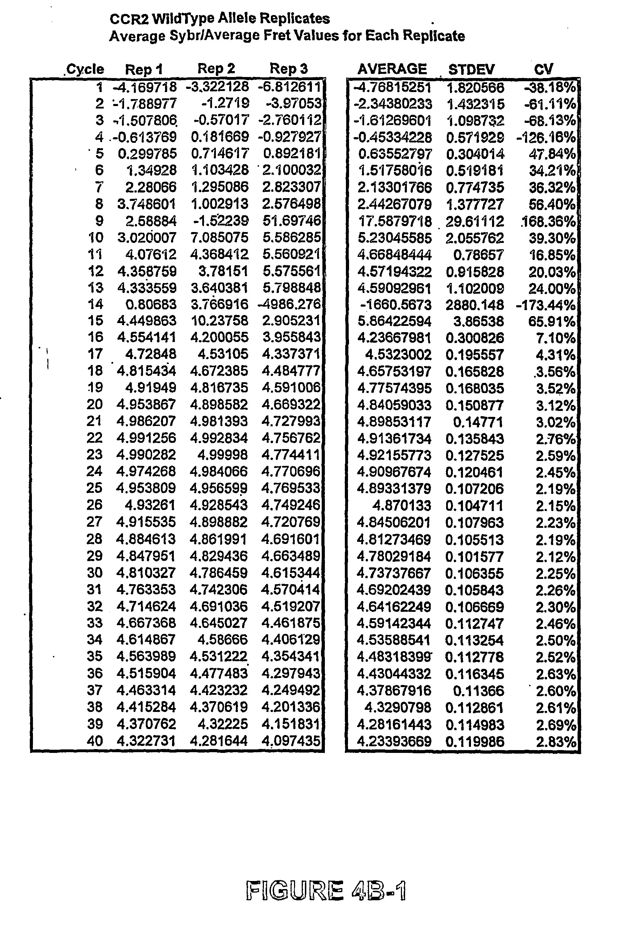

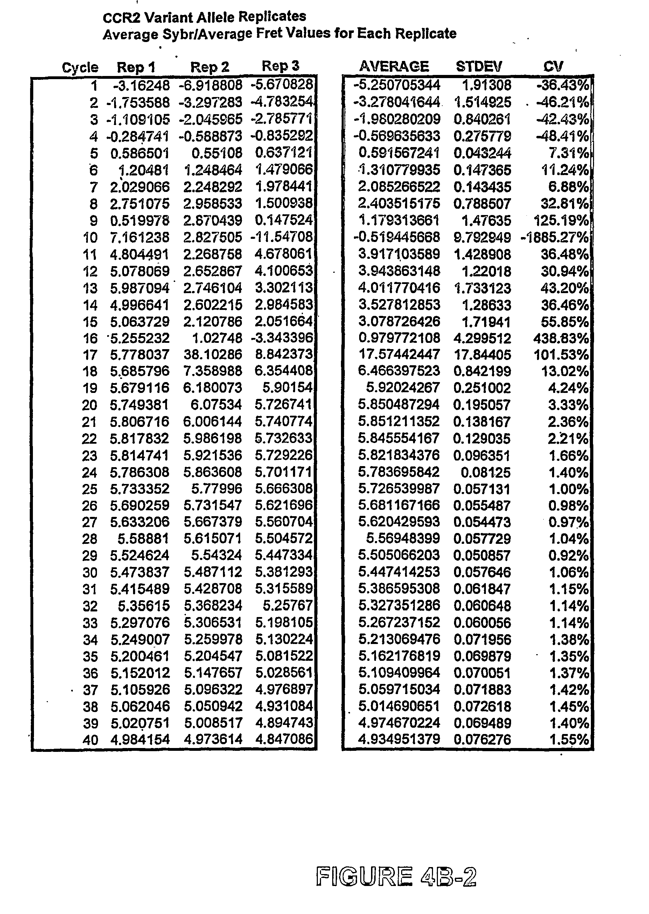

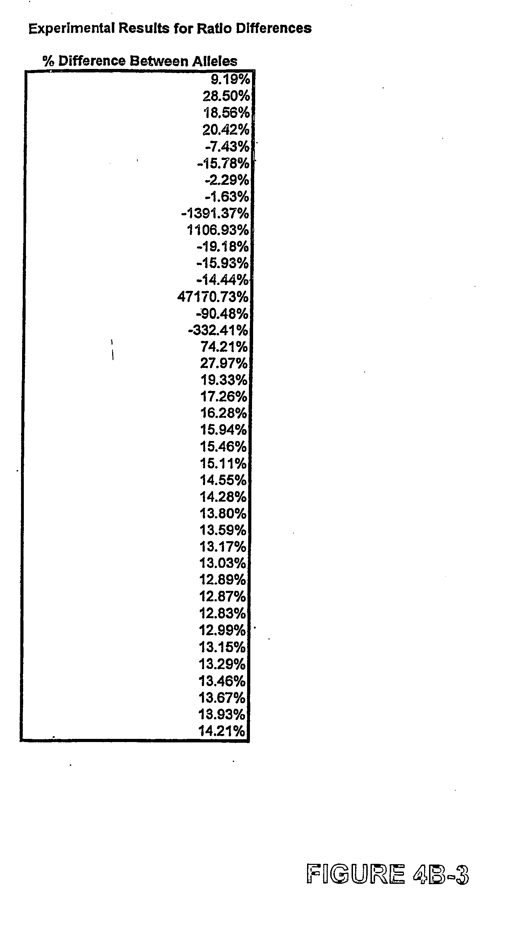

Figure 4, A and B, shows the results of experiments examining the use of tagged allele specific primers in the determination of a single nucleotide difference. The average SYBR Green/FRET ratios obtained during PCR amplification of CCR2 wildtype and SNP variant alleles as in Example 2 (Figure 2) are shown. For the amplification, the Forward CCR2 Wildtype 5G Mod (5 G tag), Forward CCR2 Variant 0G Mod (0 G tag), and Reverse 48bp CCR2 primers were used with the purified 79bp PCR (wildtype or variant) amplicon as template. All reactions contained 2% Rox-dCTP and were performed in triplicate. Figure 4A shows a graph of the average SYBR Green/FRET ratios for the wildtype (dashed line) and variant (solid line) allele versus cycle number. Figure 4B shows the data graphed in Figure 4A.

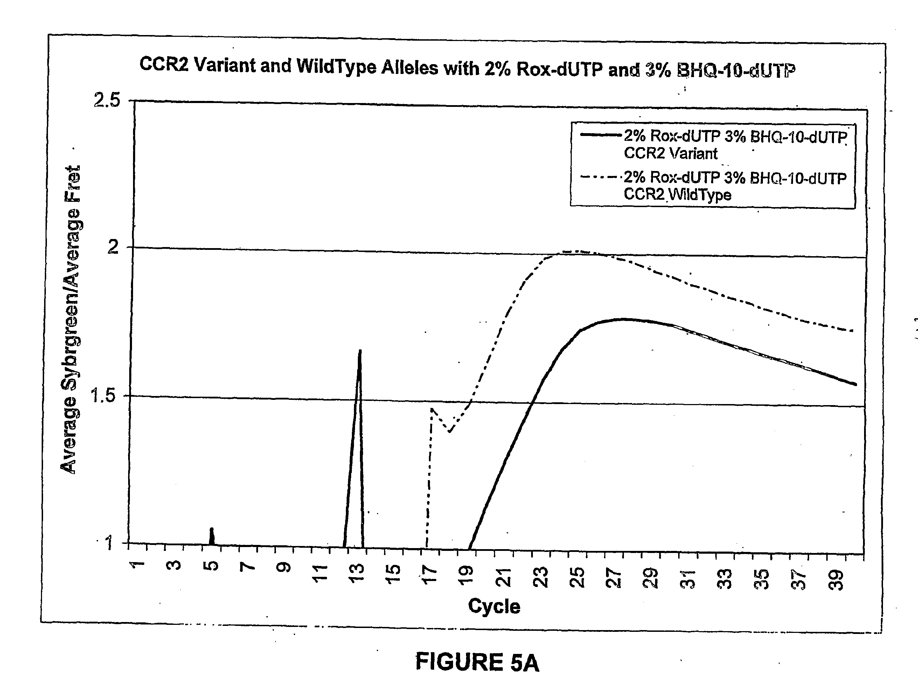

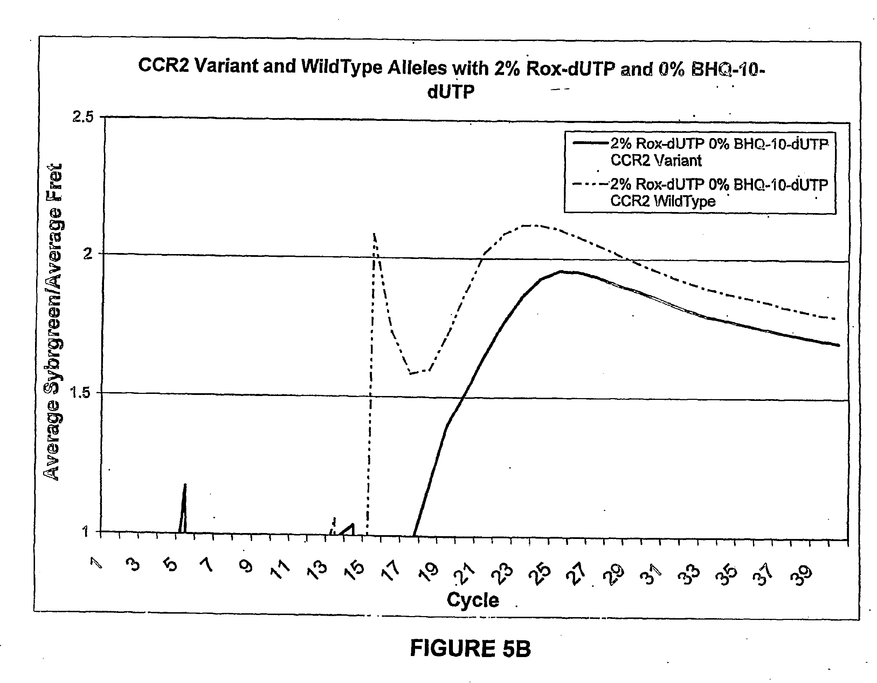

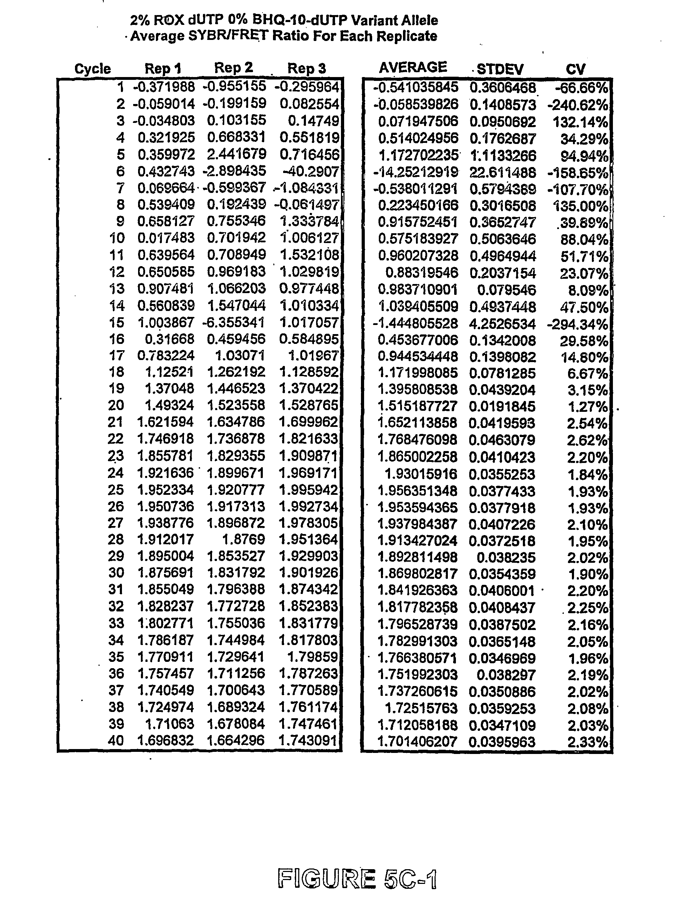

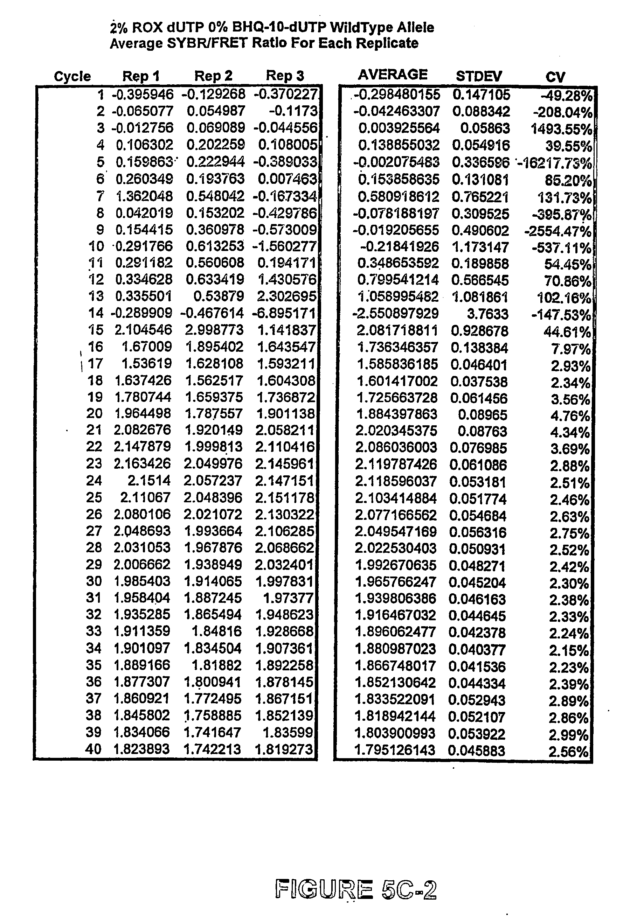

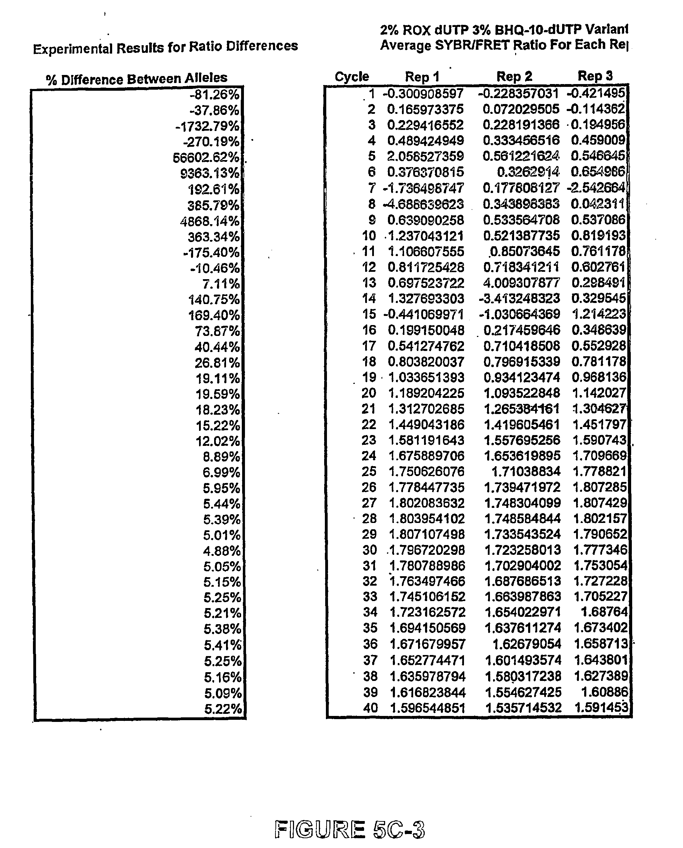

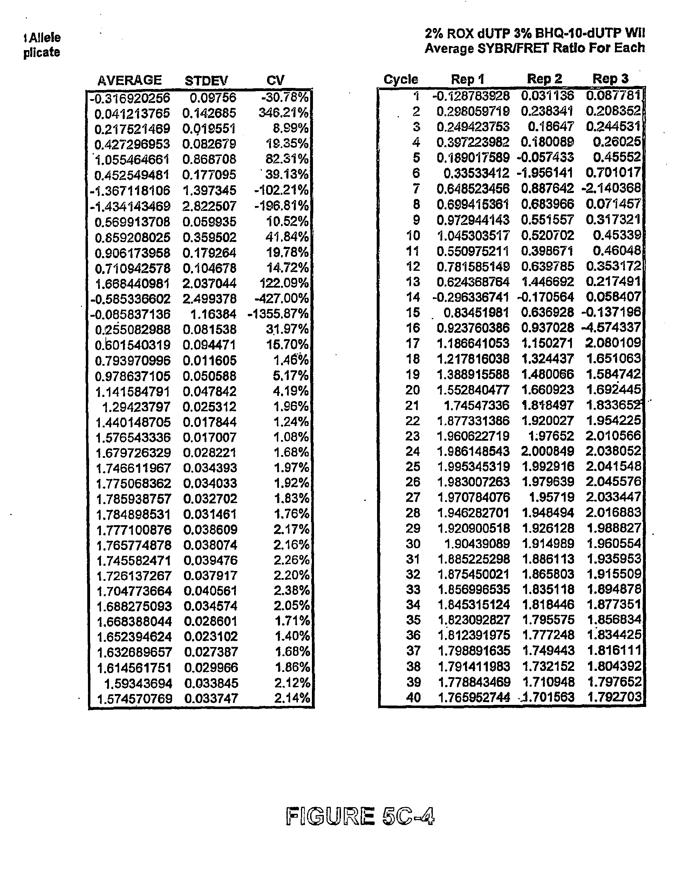

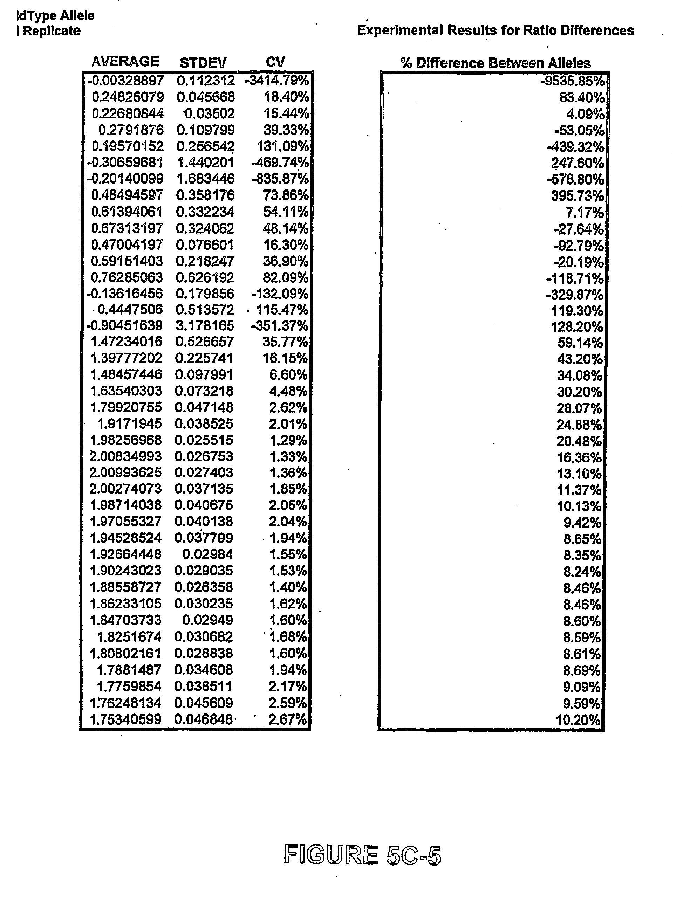

Figure 5, A-C, shows the results of experiments examining the effect of quencher dNTP on color ratio in an SNP model. The wildtype and SNP variant CCR2 templates used in Example 2 (Figure 2) were used in amplifications with and without 3% BHQ-10-dUTP. All reactions were performed in triplicate and contained 2% Rox-dUTP. Figure 5A shows a graph of average SYBR/FRET ratio versus cycle number for reactions with wildtype (dashed line) and variant allele (solid line) templates in reactions including 3% BHQ-10-dUTP. Figure 5B shows a graph of average SYBR/FRET ratio versus cycle number for reactions with wildtype (dashed line) and variant allele (solid line) templates in reactions without (0%) BHQ-10-dUTP. Figure 5C shows the data graphed in Figures 5A and 5B.

## DETAILED DESCRIPTION OF THE INVENTION

The present invention is predicated on the concept of synthesizing a polynucleotide using an enzyme capable of extending a polynucleotide in the presence of a labeled nucleotide whose incorporation frequency into the growing nucleotide strand is indicative of the presence or absence of a sequence variation in the template polynucleotide.

## Definitions

As used herein, the term "sequence difference", "sequence variation" or "variation" refers to nucleotide sequence that is different between two polynucleotide molecules. The sequence difference may exist over a single nucleotide or up to thousands of nucleotides in length, for example, 1 nucleotide, 5 nucleotides, 10 nucleotides, 50 nucleotides, 100 nucleotides, 500 nucleotides, 1000 nucleotides, 5000 nucleotides, or more, in length. Preferably, the "sequence difference" of the subject disclosure refers to a nucleotide sequence that is different between two or more otherwise closely homologous polynucleotide molecules, for example, a polymorphism between two or more alleles of a multiallelic organism.

As used herein, a "region of interest" refers to a stretch of one or more nucleotides which comprises a potential sequence difference between two or more polynucleotides. A "region" should be understood to also include a plurality of discontinuous sequences on the same polynucleotide. A region of interest, according to the present disclosure, is at least 10 nucleotides in length, for example, at least 35 nucleotides, at least 50 nucleotides in length, for example, at least 100 nucleotides, or at least 200 nucleotides, or at least 300 nucleotides, or at least 500 nucleotides, or more, in length. A region of interest may have the same length as a region containing the sequence difference or may be longer. A polynucleotide containing a region of interest, according to the present disclosure, also contains at least 5 nucleotides, for example, at least 10 nucleotides, at least 15 nucleotides, at least 25 nucleotides, or at least 50 nucleotides flanking the region of interest.

As used herein, a "corresponding region of interest" refers to a region on one polynucleotide which is an analog of a region of interest on another polynucleotide. A "region of interest" on a first polynucleotide and its "corresponding region of interest" on a second polynucleotide may be completely identical in sequences or be different only by comprising a sequence difference as defined herein above.

The term "known region of sequence of interest" refers to a region of sequence containing a known sequence variation as defined herein above.

"Polymorphism", according to the present disclosure, is used in its broadest sense and includes a sequence difference involving one or more nucleotides (e.g., 1, 2, 5, 10, 50, 100, 1000, or more nucleotides). A polymorphism includes a "single nucleotide polymorphism (SNP)," as well as "tandem repeats", insertions, deletions, inversions, and mutations.

A polymorphism may be also referred to as "allelic," in that, due to the existence of the polymorphism, some members of a species may have the invariant sequence (i.e., the original "allele") whereas other members may have a variant sequence (i.e., the variant or mutant "allele"). In the simplest case, only one variant sequence may exist, and the polymorphism is said to be diallelic. The occurrence of alternative variations can give rise to triallelic polymorphisms, etc.

Some allelic polymorphisms are referred to herein as "single nucleotide polymorphisms," or "SNPs." SNP is a single nucleotide sequence variation from the most frequently occurring base at a particular polynucleotide position. SNPs are defined by the following attributes. A central attribute of such a polymorphism is that it contains a polymorphic site, which for ease of reference is referred to herein as "X," which is the site of variation between allelic sequences. A second characteristic of a SNP is that its polymorphic site "X" is frequently preceded by and followed by "invariant" sequences of the allele. The polymorphic site of the SNP thus lies "immediately" 3' to a "5'-proximal" invariant sequence, and "immediately" 5' to a "3'-distal" invariant sequence. Such invariant sequences may flank the polymorphic site. The term "single" of single nucleotide polymorphisms refers to the number of nucleotides of the specific polymorphism (i.e. one nucleotide); it is unrelated to the number of polymorphisms present in the target nucleic acid (which may range from one to many). While "polymorphism" typically refers to alleles having a 1% or greater frequency in the population, it is used herein in the broadest sense to encompass mutations that may be present in only a single individual or in a single cell.

As used herein, a "tandem repeat" in polynucleotide is two or more adjacent, approximate copies of a stretch of nucleotides. It may exist as multiple copies of the same base sequence on a polynucleotide which may be used as a marker in physical mapping when the number of repeats varies in the population. Tandem repeats are usually classified among satellites (spanning megabases of DNA, associated with heterochromatin), minisatellites (repeat units in the range 6-100 bp, spanning hundreds of base-pairs) and microsatellites (repeat units in the range 1-5 bp, spanning a few tens of nucleotides). The minisatellites are also called "various number tandem repeats" or VNTRs. The microsatellites are also called "short tandem repeats" or STRs. Both VNTR and STR markers, contain regions of nearly identical sequences repeated in tandem fashion. The core repeat sequence is typically 10 to 70 bases in length, with shorter core repeat sequences referred to as STRs and longer repeats referred to as VNTRs. Both repeats may be used to identify individuals genetically.

As used herein, an organism comprising at least one varied allele, in addition to the original allele, is referred to as a "multiallelic organism." A "multiallelic organism," therefore, includes diallelic, triallelic organisms, etc.

The term "homozygote," as used herein, refers to a multiallelic organism with the same allele at a region of interest (e.g., a gene locus) on homologous chromosomes. The term "heterozygote," as used herein, refers to a multiallelic organism with different alleles at a region of interest (e.g., a gene locus) on homologous chromosomes. For a diploid organism (e.g., human), there are two types of homozygote compositions: in one homozygote composition, both alleles contain the same wild-type sequence at the region of interest (herein referred to as a" wild-type" homozygote); in the other composition, both alleles contain the same variant sequence at the region of interest (herein referred to as a "variant" homozygote). The two alleles of a diallelic heterozygote contain sequence variations at the region of interest between them, e.g., one allele contains the wild-type sequence, and the other contains a variant sequence.

As used herein, a sequence is an "invariant" sequence of an allele if the sequence does not vary in the population of the species, and if mapped, would map to a "corresponding" sequence of the same allele in the genome of every member of the species population. It should be noted that two or more sequence differences may be very close in proximity to each other. As used herein, the term "conventional nucleotide" refers to one of the deoxynucleotides (dNTPs), including dATP, dTTP, dCTP, and dGTP.

The term "nucleotide" as used herein refers to a phosphate ester of a nucleoside, e.g., mono, di, tri, and tetraphosphate esters, wherein the most common site of esterification is the hydroxyl group attached to the C-5 position of the pentose (or equivalent position of a non-pentose "sugar moiety"). The term "nucleotide" also includes a modified nucleotide which includes phosphorothioate, phosphite, ring atom modified derivatives, and the like. The term "nucleotide" also includes a labeled or an unlabeled nucleotide.

As used herein, "level" of a detectable label in an amplified product refers to an amount or intensity of the label incorporated into the amplified product during or after a polynucleotide synthesis reaction (e.g., PCR). Such level can be measured by nucleotide incorporation assays well known in the art (e.g., in Innis et al., (1990) Academic Press, Inc.; Molecular Cloning, A Laboratoy Manual (2d Edition, Sambrook, et al. (1989); and Current Protocols in Molecular Biology (1997, Ausubel et al., John Weley & Sons, Inc.).

As used herein, the term "substantially constant" means that the number of potential linkage sites for a polynucleotide product on a solid support per unit area vary by less than 0.5% between samples of that solid support.

The term "ascertain the incorporation frequency," as used herein, refers to the determination of an incorporation frequency for a polynucleotide template, e.g., with the presence of at least one labeled nucleotide in the extension reaction.

As used herein, the term "incorporation frequency of a nucleotide" refers to the level of the incorporation of a labeled nucleotide into an extended product as measured by the level of the detectable label in the extended product. According to some embodiments of the disclosure, the incorporation frequency of a labeled nucleotide is measured as a ratio between the level of the detectable label incorporated into the extended product and the total amount of the extended product, or the levels of incorporation between two or more labeled nucleotides into the same extended product.

The phrase "difference in the incorporation frequency" refers to a statistically significant difference (increase or decrease) in the value of the incorporation frequency percentage of a detectable label incorporated into one amplified product, compared to the value of the incorporation frequency percentage of a detectable label incorporated into another amplified product or a control polynucleotide. While one of skill in the art would generally consider a difference of two standard deviations to be significant, a statistically significant difference is one that the user of the method relies upon as being significant.

The phrase "same incorporation frequency" refers to the situation in which the value of the incorporation frequency percentage of a detectable label incorporated into at first amplified product is identical to or does not differ in a statistically significant manner from the value of the incorporation frequency percentage of a detectable label incorporated into a second amplified product.

As used herein, the term "amount of an amplified product" refers to an amount of an amplified product as measured by methods known in the art, for example, measured in $\mu$g, $\mu$mol or copy number.

A "polynucleotide" is a covalently linked sequence of nucleotide bases (i.e., ribonucleotides for RNA and deoxyribonucleotides for DNA) typically in which the 3' position of the pentose of one nucleotide is joined by a phosphodiester group to the 5' position of the pentose of the next. "Polynucleotide" includes, without limitation, single- and double-stranded polynucleotide. According to the disclosure, a nucleotide can be modified, biotinylated, radiolabeled, and the like and also include phosphorothioate, phosphite, ring atom modified derivatives, and the like. The term "nucleotide" includes the derivatives and analogs thereof and includes dNTPs. The term "polynucleotide" therefore embraces chemically, enzymatically or metabolically modified forms of polynucleotide. "Polynucleotide" also embraces a short polynucleotide, often referred to as an oligonucleotide.

A polynucleotide typically has a "5'-terminus" (5' end) and a "3'-terminus" (3' end) because polynucleotide phosphodiester linkages occur at the 5' carbon and 3' carbon of the pentose ring of the substituent mononucleotides. The end of a polynucleotide at which a new linkage would be to a 5' carbon is its 5' terminal nucleotide. The end of a polynucleotide at which a new linkage would be to a 3' carbon is its 3' terminal nucleotide. A "terminal nucleotide", as used herein, is the nucleotide at the end position of the 3'- or 5'-terminus. As used herein, a polynucleotide sequence, even if internal to a larger polynucleotide (e.g., a sequence region within a polynucleotide), also can be said to have 5'- and 3'- ends.

A "target polynucleotide" refers to a polynucleotide comprising a region of interest. A target polynucleotide may serve as a polynucleotide template for a polynucleotide synthesis reaction.

As used herein, a "template" refers to a polynucleotide of specific identity which can serve as a template for the synthesis of a complementary molecule. A polynucleotide template may be single- or double- stranded, and it may be DNA, RNA, a polynucleotide comprising both deoxyribo- and ribonucleotides, or a polynucleotide comprising deoxyribonucleotides, ribonucleotides, and/or analogs and derivatives thereof. In the context of PCR, a "polynucleotide template" may refer to a fragment or fraction of the polynucleotides from which a complementary molecule is to be synthesized, i.e., the sequence between and including the two primers, or it may refer to the entire polynucleotide comprising the fragment or fraction.

As used herein, an "oligonucleotide primer" or a "primer" is an oligonucleotide comprising a sequence complementary to a polynucleotide template and is able to hybridize to the template. A primer, according to the disclosure, hybridizes to a polynucleotide template through base pairing so as to initiate an elongation (extension) reaction to incorporate a nucleotide into the oligonucleotide primer. A primer of the present disclosure may be between 10 to 100 nucleotides in length, preferably between 15-50 nucleotides in length.

As used herein, the term "a set of primers" includes at least two, may be three or more primers according to the present disclosure.

A set of primers which "flank" a region of interest refers to two primers with opposite orientation, where the 3' terminal nucleotide of each primer hybridizes to the 3' terminal nucleotide of a double stranded region of interest or to a nucleotide located at 3' of the region of interest. The nucleotide located at 3' of the region of interest may be located immediately 3' of the 3' terminal nucleotide of the region of interest, or it may be one, or two, or three, or more nucleotides away from the 3' terminal nucleotide of the region of interest.

As used herein, the term "opposite orientation", when refers to primers, means that one primer comprises a nucleotide sequence complementary to the sense strand of a polynucleotide template, and another primer comprises a nucleotide sequence complementary to the antisense strand of the same polynucleotide template. Primers with opposite orientations may generate an amplified product from the polynucleotide template to which they complement.

As used herein, the term "same orientation", means that both or all primers comprise nucleotide sequences complementary to the same strand of a target polynucleotide template. Primers with the same orientation will not generate an amplified product from the polynucleotide template to which they complement.

As used herein, a "primer which hybridizes immediately 3' of nucleotide X" is an oligonucleotide having a 3' terminal nucleotide complementary to the nucleotide next to the 3' end of nucleotide X of a polynucleotide template, with no nucleotides in between the position of the 3' terminal nucleotide of the oligonucleotide and the position of the 3' end of nucleotide X. The position of nucleotide X, according to the disclosure, may be predetermined, for example, as a site containing a sequence difference, e.g., a polymorphism. The hybridization of the oligonucleotide to the immediately 3' of nucleotide X of the polynucleotide allows the incorporation of one or more nucleotides into the oligonucleotides starting by incorporating a nucleotide complementary to nucleotide X.

"Complementary" refers to the broad concept of sequence complementarity between regions of two polynucleotide strands or between two regions of the same polynucleotide strand. It is known that an adenine base of a first polynucleotide region is capable of forming specific hydrogen bonds ("base pairing") with a base of a second polynucleotide region which is antiparallel to the first region if the base is thymine or uracil. Similarly, it is known that a cytosine base of a first polynucleotide strand is capable of base pairing with a base of a second polynucleotide strand which is antiparallel to the first strand if the base is guanine. A first region of a polynucleotide is complementary to a second region of the same or a different polynucleotide if, when the two regions are arranged in an antiparallel fashion, at least one nucleotide base of the first region is capable of base pairing with a base of the second region. A first polynucleotide that is 100% complementary to a second polynucleotide forms base pair at every nucleotide position. A first polynucleotide that is not 100% complementary (e.g., 90%, or 80% or 70% complementary) contains mismatched nucleotides at one or more nucleotide positions. An oligonucleotide primer, according to the present disclosure, is complementary (i.e., having more than 70%, 80%, 90%, or up to 100% sequence identity) to a polynucleotide template.

As used herein, a "detectable label" refers to a molecule capable of generating a detectable signal. A "detectable label" may be detected directly or detectable through a specific binding reaction that generates a detectable signal. The label can be isotopic or non-isotopic, usually non-isotopic, and can be a catalyst, such as an enzyme (also referred to as an enzyme label), a polynucleotide coding for a catalyst, a promoter, dye, fluorescent molecule (also referred to as a fluorescent label), fluorescent quencher, fluorescence resonance energy transfer pair, chemiluminescer (also referred to as a chemiluminescent label), coenzyme, enzyme substrate, radioactive group (also referred to as a radiolabel), a small organic molecule, amplifiable polynucleotide sequence, a particle such as latex or carbon particle, metal sol, crystallite, liposome, cell, etc., which may or may not be further labeled with a dye (also referred to as a colorimetric label), catalyst or other detectable group, and the like. The label may be a directly detectable label or may be a member of a signal generating system, and thus can generate a detectable signal in context with other members of the signal generating system, e.g., a biotin-avidin signal generation system. The label can be bound directly to a nucleotide or a polynucleotide sequence or indirectly via a linker.

As used herein, the term "hybridize" is used in reference to the pairing of complementary polynucleotide strands. Hybridization and the strength of hybridization (i.e., the strength of the association between polynucleotide strands) is impacted by many factors well known in the art, including the degree of complementarity between the polynucleotides, stringency of the conditions involved, such as the concentration of salts, the Tm (melting temperature) of the formed hybrid, the presence of other components (e.g., the presence or absence of polyethylene glycol), the molarity of the hybridizing strands and the G:C content of the polynucleotide strands.

As used herein, "polynucleotide synthesis enzyme" refers to an enzyme that catalyzes the polymerization of nucleotides. Generally, the enzyme will initiate synthesis at the 3'-end of the primer hybridized to a polynucleotide template sequence, and will proceed toward the 5' end of the template strand. "DNA polymerase" catalyzes the polymerization of deoxynucleotides.

The term "sample" as used herein is used in its broadest sense to refer to a material containing a polynucleotide. A sample may comprise a cell, a biological fluid, chromosomes isolated from a cell (e.g., a spread of metaphase chromosomes), genomic DNA, RNA, cDNA and the like.

"Primer extension reaction" or "chain elongation reaction" means a reaction between a template-primer hybrid and a nucleotide which results in the addition of the nucleotide to a 3'-end of the primer such that the incorporated nucleotide is complementary to the corresponding nucleotide of the template polynucleotide. Primer extension reagents typically include (i) a polymerase enzyme; (ii) a buffer; and (iii) one or more extendible nucleotides, e.g., dNTPs, but may use enzymes such as ligases, terminal transferases, or reverse transcriptases.

As used herein, "polymerase chain reaction" or "PCR" refers to an in vitro chain elongation reaction method for amplifying a specific polynucleotide template sequence. The PCR reaction involves a repetitive series of temperature cycles and is typically performed in a volume of 25-100 µl. The reaction mix generally comprises dNTPs (e.g., each of the four deoxynucleotides dATP, dCTP, dGTP, and dTTP), primers, buffers, DNA polymerase, and at least one polynucleotide template. One PCR reaction may consist of 5 to 100 "cycles" of denaturation and synthesis of a polynucleotide molecule.

As used herein, the term "linked" when used in relation to a polynucleotide and a solid support means that the polynucleotide is physically associated with or bound to the solid support. The association or binding can be direct or indirect (e.g., mediated by physical association of the polynucleotides with another moiety, e.g, an affinity moiety or polynucleotide bound to the surface of the support), covalent or non-covalent.

The present disclosure describes method(s) for determining a sequence variation between a region of interest of a first polynucleotide and a corresponding region of sequence of a second polynucleotide (or relative to a known reference sequence).

The subject method of the present disclosure includes incubating the first polynucleotide in a reaction mixture comprising a polynucleotide synthesis enzyme, a set of primers flanking the region of interest, and a nucleotide labeled with a detectable label (e.g., a labeled dCTP). The incubation allows the amplification of the region of interest, therefore, the labeled nucleotide (e.g., labeled dCTP) is incorporated into the primers during the amplification process. After the amplification, the incorporation frequency of the labeled nucleotide (e.g., labeled dCTP) is determined. The above process can be repeated for as many polynucleotides of interest which comprises a corresponding region of interest (e.g., a second, a third, or a fourth polynucleotide) as possible. After the process has been performed for each polynucleotide, the incorporation frequency of the labeled nucleotide (e.g., labeled dCTP) for each polynucleotide of interest (e.g., the first, second, third, or fourth polynucleotide) is determined. The presence or absence of a difference in the incorporation frequency of the labeled nucleotide, either as compared between two or more polynucleotides of interest, or as compared to one or more control polynucleotides, is indicative of the presence or absence of a sequence variation within the region of interest of the first polynucleotide or the corresponding region of sequence of the second, or third, or fourth polynucleotide.

Samples And Polynucleotide Templates Of The Present Disclosure

The methods of the present disclosure can be utilized to determine the identity of a nucleotide at a variety of different types of variant sites including, but not limited to, SNPs and mutations such as transitions, transversions, insertions and deletions, as well as tandem repeats.

The polynucleotide template can be only a fraction of a larger polynucleotide or can be present initially as a purified and discrete molecule. The polynucleotide template can be synthesized enzymatically in vivo, synthesized enzymatically in vitro, or synthesized non-enzymatically.

A polynucleotide of interest useful as template for the present disclosure may be single- or double- stranded, and it may be DNA (e.g., genomic or cDNA), RNA, a polynucleotide comprising both deoxyribo- and ribonucleotides, or a polynucleotide comprising deoxyribonucleotides, ribonucleotides, and/or analogs and derivatives thereof. Preferably, the polynucleotide template is a double-stranded DNA molecule.

The polynucleotide templates (i.e., a target polynucleotide or a polynucleotide containing a region of interest) according to the disclosure are preferably polynucleotides comprising polymorphisms, e.g., alleles, or mutations. The polynucleotide template may also be a polynucleotide containing a potential mutation in its nucleotide sequence.

One preferred polymorphism according to the present disclosure is a SNP. If a polynucleotide template is double-stranded (e.g., a genomic DNA, or a cDNA), each SNP can be defined in terms of either the plus strand or the minus strand, the sense or the antisense strand, the upper or the lower strand. In a preferred embodiment, where each SNP's polymorphic site, "X," is a single nucleotide, each strand of the double-stranded DNA of the SNP will contain invariant sequences flanking X, i.e., invariant sequences on both the 5' and 3' ends of X. It is also possible, however, that a SNP may only have invariant sequence on one of the 5' and 3' ends, e.g., when the SNP locates at the end of a polynucleotide.

Although the preferred SNPs of the present disclosure involve a substitution of one nucleotide for another at the SNP's polymorphic site, SNPs can also be more complex, and may comprise a deletion of a nucleotide from, or an insertion of a nucleotide into, one of two corresponding sequences. For example, a particular gene sequence may contain an "X" in a particular polymorphic site in some organisms, whereas in other organisms a single or multiple base deletion might be present at that site.

Another preferred polynucleotide variation of the present disclosure is referred to as Tandem repeat (both STRs and VNTRs). Since there may be mutations in sequence replication, exact matching is not required in finding the short tandem repeats.

The SNP sites or tandem repeats of the present disclosure can be used to analyze the DNA of any plant, animal, or microbe. Such sites are suitable for analyzing the genome of mammals, including humans, nonhuman primates, domestic animals (such as dogs, cats, etc.), farm animals (such as cattle, sheep, etc.) and other economically important animals. They may, however, be used with regard to other types of animals, plants, and microorganisms, including viruses having RNA or DNA genomes.

Therefore, the templates used in the methods of this disclosure can be obtained from any source that potentially contains a polynucleotide of interest. Such sources include those from any animal, including humans and other mammals, as well as plants, fungi, bacteria, and archaebacteria. Templates can be prepared from any material containing cells or polynucleotides. In the case of an animal, such material includes, e.g. tissue biopsy, blood, hair, buccal scrapes, etc. In the case of plants, such materials include seeds, spores, embryos, flowers, ovules, leafs, stems, etc.

Before the polynucleotide synthesis reaction, the polynucleotide template may be obtained in suitable quantity and quality for the chosen amplification method to be used. For example, in some instances, the samples contain such a low level of polynucleotide templates that it is useful to conduct a pre-amplification reaction to increase the concentration of the polynucleotide templates. If samples are to be amplified, amplification is typically conducted using the polymerase chain reaction (PCR) according to known procedures. See generally, PCR Technology : Principles and Applications for DNA Amplificatioh (H. A. Erlich, Ed.) Freeman Press, NY, NY (1992); PCR Protocols : A Guide to Methods and Applications (Innis, et al., Eds.) Academic Press, San Diego, CA (1990); Mattila et al., Nucleic Acids Res. 19: 4967 (1991) ; Eckert et al., PCR Methods and Applications 1: 17 (1991) ; PCR (McPherson et al. Ed.), IRL Press, Oxford; and U. S. Patent Nos. 4,683,202 and 4,683,195.

Other suitable amplification methods include: (a) ligase chain reaction (LCR) (see Wu and Wallace, Genomies 4 : 560 (1989) and Landegren et al., Science 241: 1077 (1988)); (b) transcription amplification (Kwoh et al., Proc. Natl. Acad. Sci. USA 86 : 1173 (1989)); (c) self-sustained sequence replication (Guatelli et al., Proc. Natl. Acad. Sci. USA, 87 : 1874 (1990)); and (d) nucleic acid based sequence amplification (NABSA) (see, Sooknanan, R. and Malek, L., Bio Technology 13: 563-65 (1995)), rolling circle replication (Lizardi et al., Nature Genet. 19: 225-232 (1998); Schweitzer et al., Proc. Nat'l. Acad. Sci. USA 97: 10113-10119 (2000); random primer amplification (Williams, et al, Nucl. Acids. Res. 18: 6531-6535 (1990); Welsh & McClelland, Nucl. Acids. Res. 18: 7213-7218 (1990).

Further guidance for the preparation of templates can be found in a multitude of sources, including PCR Protocols, A Guide to Methods and Applications (Innis et al., supra; Sambrook, et al. supra; Ausubel et al., supra). Any such method can be used in the present disclosure. Typically, these methods involve cell lysis, followed by purification of polynucleotides by methods such as phenol/chloroform extraction, electrophoresis, and/or chromatography. Often, such methods include a step wherein the polynucleotides are precipitated, e.g. with ethanol, and resuspended in an appropriate buffer for addition to a PCR or similar reaction.

Some suitable samples can be purchased from suppliers such as the American Type Culture Collection, Rockville, MD, US or Coriell Institute for Medical Research, Camden, NJ, US. Additionally, commercially available kits for obtaining suitable polynucleotide samples from various sources are available from Stratagene (La Jolla, CA); Qiagen Inc. (Chatsworth, CA); Invitrogen Corporation (Carlsbad, CA); and 5'-3' Prime Inc. (Boulder, CO), among other suppliers. Further, general methods for obtaining polynucleotides from various sources for amplification methods including PCR and RT-PCR are well known to those with skill in the art.

The choice of the template used in the present disclosure will depend on the particular application used. Any polynucleotide desirably synthesized may be used in the present disclosure. Such applications include, but are not limited to: mutation identification, allele discrimination, genotyping, and diagnostic procedures where the presence or absence of a particular polynucleotide provides information regarding the existence or state of a biological condition, such as a disease.

In certain embodiments, a plurality of polynucleotide templates from one or more sample sources are used in the present disclosure. For example, a single polynucleotide from a multitude of sources may be synthesized to screen for the presence or absence of a particular sequence difference. In other applications, a plurality of polynucleotides may be amplified from a single sample or individual, thereby allowing the assessment of a variety of polynucleotides in a single individual, e.g., to simultaneously screen for a multitude of disease markers in an individual. Any of the above applications can be easily accomplished using the method of the present disclosure.

A reaction mixture may comprise one polynucleotide template, or it may comprise more than one polynucleotide template. The present method allows for simultaneous analysis of polynucleotides obtained from a plurality of samples.

Typically, one or more control polynucleotides are provided for a polynucleotide template. A control polynucleotide, according to the present disclosure, may be a reference polynucleotide for which the incorporation frequency of a labeled nucleotide can be calculated based on the sequence of the region of interest. Alternatively, a positive control polynucleotide may be a polynucleotide which can be extended in identical reaction condition as that for the polynucleotide of interest and therefore provides an incorporation frequency for a labeled nucleotide which can be compared with the frequency calculated for the polynucleotide of interest.

Control polynucleotides may be different according to the specific application of the subject method. For example, if the subject method is applied to mutation identification, the control polynucleotide may be a polynucleotide containing the wild-type sequence at the region of interest. If the subject disclosure is applied to allele discrimination or genotyping, the control polynucleotide template may be a polynucleotide representing the sequence of any possible allele. Alternatively, two or more control polynucleotide templates may be employed for a single extension reaction; for example, in genotyping using genomic DNA template, the control polynucleotide templates may represent the wild-type alleles from a homozygote, or the variant alleles from a homozygote, or the alleles from a heterozygote (a copy of each of the wild-type and variant alleles).

For generating control templates, one of three common methods may be used: (a) the respective polynucleotides (e.g., alleles) are cloned and the purified, linearized plasmid serves as a control; (b) gDNA that has been sequence verified to belong to one of the three groups (i.e., wild-type homozygote, variant homozygote, and heterozygote) is used; or (c) synthetic oligonucletides with the corresponding SNP are used.

Selection Of One Or More Regions Of Interest For Analysis

One or more specific regions of interest may be selected where the presence, location or identity of at least one sequence difference (e.g., a polymorphism) is to be determined. Region selection can be based upon known sequence information for the same or related polynucleotides, or can be based upon the region of interest of a reference polynucleotide which is sequenced using techniques well known to those with skill in the art.

In a preferred embodiment, the region selected comprises or potentially comprises at least one sequence difference (e.g., a polymorphism including a SNP or a tandem repeat). Preferably, the selected region is at least 20 nucleotides in length, for example, at least 35 nucleotides, at least 50 nucleotides, at least 100 nucleotides, or at least 200 nucleotides, or at least 300 nucleotides, or at least 500 nucleotides, or more, in length. Also preferably, there are at least 15 nucleotides, for example, at least 25 nucleotides, or at least 50 nucleotides flanking a polymorphism site.

Signal Generation

The level of a labeled nucleotide incorporation according to the present disclosure may be detected by any polynucleotide detecting method known in the art. In preferred embodiments, signals for detection of dNTP incorporation are generated by detectable labels. The detection of the amount of amplified product may also be performed by any techniques known in the art, such as polynucleotide staining or through a detectable label by using a labeled primer for the amplification.

The detectable label of the present disclosure includes a label that is either directly or indirectly detectable. The label can be any compound or molecule that can be detected and that does not significantly interfere with the extension reaction (e. g., interfering sufficiently such that an undetectable amount of amplified product is formed and/or causing elevated rates of misincorporation such that an accurate determination of the identity of the nucleotide at the variant site is not possible).

Detectable labels may be compounds or elements detectable by techniques that include fluorescent labels, fluorescent quenchers, polynucleotide tag labels, radioisotopes (e.g., $^3$H, $^{125}$I, $^{35}$S, $^{14}$C, $^{32}$P, $^{33}$P, etc.), enzymes (e.g. horse-radish peroxidase, alkaline phosphatase etc.), chemiluminescent compounds, spin labels, immunologically detectable haptens, colorimetric labels such as colloidal gold or colored glass or plastic (e.g. polystyrene, polypropylene, latex, etc.) beads. Such labels can be detected by spectroscopic, photochemical, biochemical, immunochemical, electrical, optical or chemical means. As indicated above, a wide variety of labels are used, with the choice of label depending on sensitivity required, ease of conjugation with the compound, stability requirements, available instrumentation, and disposal provisions.

In a preferred embodiment of the disclosure, the level of a labeled nucleotide incorporation is detected by fluorescent signals generated by fluorescent dyes (e.g., fluorophores).

Fluorescent dyes useful as detectable labels (e.g., fluorescein isothiocyanate, Texas red, rhodamine, and the like), are well known to those skilled in the art and numerous examples can be found in the Handbook of Fluorescent Probes and Research Chemicals 6th Edition, Richard Haugland, Molecular Probes, Inc., 1996 (ISBN 0-9652240-0-7).

Available fluorophores include, but are not limited to, coumarin, fluorescein, tetrachlorofluorescein, hexachlorofluorescein, Lucifer yellow, rhodamine, BODIPY, SYBR Green tetramethylrhodamine, Cy3, Cy5, Cy7, eosine, Texas red, FAM, TAMRA, ROX, R6G, R110, Texas Red™ (TR), Lissamine™ rhodamine B, Oregon Green™ 488 (2',7' - difluorofluorescein), carboxyrhodol and carboxyrhodamine, Oregon Green™ 500, 6 - JOE (6 - carboxy - 4',5' - dichloro - 2',7' - dimethoxyfluorescein), eosin F3S (6 - carobxymethylthio - 2',4',5',7' - tetrabromo - trifluorofluorescein), cascade blue™ (CB), aminomethylcoumarin (AMC), pyrenes, dansyl chloride (5 - dimethylaminonaphthalene - 1 - sulfonyl chloride) and other napththalenes, PyMPO, ITC (1 - (3 - isothiocyanatophenyl) - 4 - (5 - (4 - methoxyphenyl)oxazol - 2 - yl)pyridinium bromide). Combination fluorophores such as fluorescein-rhodamine dimers, described, for example, by Lee et al. (1997), Polynucleotides Research 25:2816, are also suitable. Fluorophores may be chosen to absorb and emit in the visible spectrum or outside the visible spectrum, such as in the ultraviolet or infrared ranges. Suitable fluorescent dye labels are commercially available from Molecular Probes, Inc., Eugene, OR, US and Research Organics, Inc., Cleveland, OH, US, among other sources.

Fluorescent dye-labeled nucleotides can be purchased from commercial sources, or they may be prepared by a number of approaches. The nucleotides of the disclosure may contain primary and secondary amines, hydroxyl, nitro and carbonyl groups. Methods that can be used to make fluorescent nucleotides are described below.

Useful nucleotides that can be labeled for the present disclosure may be any nucleotide, including nucleotide analog which can be incorporated into a primer extension reaction. There are conventional nucleotides, i.e., dATP, dTTP, dCTP, dGTP, and dUTP. "Nucleotide Analog" refers to a nucleotide in which the pentose sugar and/or one or more of the phosphate esters is replaced with its respective analog. Exemplary pentose sugar analogs are those previously described in conjunction with nucleoside analogs. Exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., including any associated counterions, if present.

Also included within the definition of "nucleotide analog" are nucleobase monomers which can be polymerized into polynucleotide analogs in which the DNA/RNA phosphate ester and/or sugar phosphate ester backbone is replaced with a different type of linkage.

Nucleotides containing amine groups that are appropriate for the introduction of fluorescent dyes include but are not limited to those listed in Table 1. A number of chemical reactions can be applied to the fluorescent labeling of amines including but not limited to the following, where the fluorescent dye is conjugated to the indicated reactive group:

**Table** 1

| Functional Group | Reaction | Product |
|---|---|---|
| Amine | dye - isothiocyanates | Thiourea |
| Amine | dye - succinimidyl ester | Carboxamide |
| Amine | dye - sulfonyl chloride | Sulphonamide |
| Amine | dye - aldehyde | Alkylamine |

Nucleotides containing ketone groups that are appropriate for the introduction of fluorescent dyes include but are not limited to those listed in Table 2. A number of chemical reactions can be applied to the fluorescent labeling of ketone groups including but not limited to the following, where the fluorescent dye is conjugated to the indicated reactive group:

**Table 2**

| Functional Group | Reaction | Product |
|---|---|---|
| Ketone | dye - hydrazides | Hydrazones |
| Ketone | dye - semicarbazides | Hydrazones |
| Ketone | dye - carbohydrazides | Hydrazones |
| Ketone | dye - amines | Alkylamine |

Nucleotides containing aldehyde groups that are appropriate for the introduction of fluorescent dyes include but are not limited to those listed in Table 3. A number of chemical reactions can be applied to the fluorescent labeling of aldehyde groups including but not limited to the following, where the fluorescent dye is conjugated to the indicated reactive group:

**Table 3**

| Functional Group | Reaction | Product |
|---|---|---|
| Aldehyde | dye - hydrazides | Hydrazones |
| Aldehyde | dye - semicarbazides | Hydrazones |
| Aldehyde | dye - carbohydrazides | Hydrazones |
| Aldehyde | dye - amines | Alkylamine |

Nucleotides containing dehydroalanine groups that are appropriate for the introduction of fluorescent dyes include but are not limited to those listed in Table 4. Dehydrobutyrene and dehydroalanine moieties have characteristic reactions that can be utilized to introduce fluorophores, as illustrated but not limited to the following, where the fluorescent dye is conjugated to the indicated reactive group:

**Table 4**

| Functional Group | Reaction | Product |
|---|---|---|
| Dehydrobutyrine | dye - sulphydryl | Methyl lanthionine |
| Dehydroalanine | dye - sulphydryl | Lanthionine |

Further label systems include two-component systems where a signal is created or abolished when the two components are brought into close proximity with one another. Alternatively a signal is created or abolished when the two components are separated, e.g., following the incorporation of the labeled nucleotide into the amplified product.

Convenient two-component systems may be based on the use of energy transfer, for example between a fluorophore and a quencher. In a particular aspect of the disclosure the detection system comprises a fluorophore/ quencher pair. Convenient and preferred attachment points for energy transfer partners may be determined by routine experimentation. A number of convenient fluorophore/quencher pairs are detailed in the literature (for example Glazer et al, Current Opinion in Biotechnology, 1997, 8, 94-102) and in catalogues such as those from Molecular Probes, Glen Research and Applied Biosystems (ABI). Any fluorescent molecule is suitable for signaling provided it may be detected on the instrumentation available. The quencher must be able to quench the dye and this may be via a Fluorescence Resonance Energy Transfer (FRET) mechanism involving a second, receptor fluorophore, or more preferably via a collisional mechanism involving a non-fluorogenic quencher such as DABCYL, which is a "universal" quencher of fluorescence. The selected fluorophores and quenchers are readily incorporated into the oligonucleotides by one of skill in the art. Suitable quenchers and energy transfer pairs are commercially available, such as Big Dyes™ from Perkin-Elmer Corporation.

The donor and acceptor groups may independently be selected from suitable fluorescent groups, chromophores and quenching groups. Donors and acceptors useful according to the disclosure include but are not limited to: 5 - FAM (also called 5 - carboxyfluorescein; also called Spiro(isobenzofuran - 1(3H), 9' - (9H)xanthene) - 5 - carboxylic acid,3', 6' - dihydroxy - 3 - oxo - 6 - carboxyfluorescein); 5 - Hexachloro - Fluorescein ([4,7,2',4',5',7' - hexachloro - (3',6' - dipivaloyl - fluoresceinyl) - 6 - carboxylic acid ]); 6 - Hexachloro - Fluorescein ([4,7,2',4',5',7' - hexachloro - (3',6' - dipivaloylfluoresceinyl) - 5 - carboxylic acid ]); 5 - Tetrachloro - Fluorescein ([4,7,2',7' - tetra - chloro - (3',6' - dipivaloylfluoresceinyl) - 5 - carboxylic acid]); 6 - Tetrachloro - Fluorescein ([4,7,2',7' - tetrachloro - (3',6' - dipivaloylfluoresceinyl) - 6 - carboxylic acid]); 5 - TAMRA (5 - carboxytetramethylrhodamine; Xanthylium, 9 - (2,4 - dicarboxyphenyl) - 3,6 - bis (dimethyl - amino); 6 - TAMRA (6 - carboxytetramethylrhodamine; Xanthylium, 9 - (2,5 - dicarboxyphenyl) - 3, 6 - bis (dimethylamino); EDANS (5 - ((2-aminoethyl) amino)naphthalene - 1 - sulfonic acid); 1,5 - IAEDANS (5 - ((((2 - iodoacetyl) amino)ethyl) amino)naphthalene - 1 - sulfonic acid); DABCYL (4 - ((4 - (dimethylamino)phenyl) azo)benzoic acid) Cy5 (Indodicarbocyanine - 5) Cy3 (Indo - dicarbocyanine - 3); and BODIPY FL (2,6 - dibromo - 4,4 - difluoro - 5,7 - dimethyl - 4 - bora - 3a,4a - diaza - s - indacene - 3 - proprionic acid), ROX (Carboxy-X-rhodamine) and SYBR Green, as well as suitable derivatives thereof. Further guidance regarding the selection of donor and acceptor pairs that can effectively be used with the methods of the present disclosure includes: Fluorescence Spectroscopy (Pesce et al., Eds.) Marcel Dekker, New York, (1971) ; White et al., Fluorescence Analysis : A Practical Approach, Marcel Dekker, New York, (1970); Berlman, Handbook of Fluorescence Spectra of Aromatic Molecules, 2nd ed., Academic Press, New York, (1971) ; Griffiths, Colour and Constitution of Organic Molecules, Academic Press, New York, (1976); Indicators (Bishop, Ed.). Pergamon Press, Oxford, 19723; and Haugland, Handbook of Fluorescent Probes and Research Chemicals, Molecular Probes, Eugene (1992).

Preferred combinations of donors and acceptors are listed as, but not limited to, the donor/acceptor pairs shown in Tables 5 and 6 (which includes values for $R_o$ - the distance at which 50% of excited donors are deactivated by FRET).

**Table 5.** Typical values of $R_o$

| Donor | Acceptor | Ro (Å)* |
|---|---|---|
| Fluorescein | Tetramethylrhodamine | 55 |
| IAEDANS | Fluorescein | 46 |
| EDANS | DABCYL | 33 |
| Fluorescein | Fluorescein | 44 |
| BODIPY FL | BODIPY FL | 57 |
| *$R_o$ is the distance at which 50% of excited donors are deactivated by FRET. Data from Haugland, RP. 1996. Handbook of Fluorescent Probes and Research Chemicals, 6th edition. Molecular Probes, Inc. Eugene OR, USA. | | |

**Table 6.** FRET-pairs suitable for use in the method of this disclosure.

| Donor | Acceptor |
|---|---|
| **(a) Fluorescent donors** | |
| Fluorescein | Tetramethylrhodamine |
| Fluorescein | Cy-3 |
| Fluorescein | ROX |

(continued)

| Donor | Acceptor |
|---|---|
| **(a) Fluorescent donors** | |
| EDANS | DABCYL |
| Dansyl | Fluorescein |
| Cy3 | Cy-5 |
| Tryptophan | AEDANS |
| Fluorescein | Tetramethyl rhodamine |
| Tetramethyl rhodamine | DABCYL |
| Fluorescein | DABCYL |
| DABCYL | Cy-3 |
| Fluorescein | Hexachlorofluorescein |
| Tetrachlorofluorescein | Cy-5 |
| SYBR Green | Rox |
| **(b) Luminescent donors** | |
| Europium | Cy-5 |
| Terbium | Tetramethyl rhodamine |
| Terbium | Cy-3 |

Both elements of the two component system may be provided on the same or different molecules.

The detectable label of the present disclosure may be joined directly to the nucleotide or the primer, or it may be joined through a linker. Examples of suitable linkers are described in U.S. Patent No. 5,770,716. Preferably, the detectable label is joined to the nucleotide or the primer so as not to prevent the incorporation of the labeled nucleotide in a DNA extension reaction.

Further, custom-made primers with attached fluorescent labels can be obtained from Amersham Pharmacia Biotech, Inc., among other suppliers.

In another embodiment, a fluorescent donor is placed on the extension product, e.g., by staining or covalent attachment. The detectable label on the labeled nucleotide is a fluorescent acceptor which can be activated by the fluorescent donor. Therefore, the unincorporated nucleotide will remain "silent", while it will be activated to generate a detectable signal after being incorporated into the amplified product.

In some embodiments, a polynucleotide stain is used due to its preferential staining for double stranded DNA. Thus the amount of extension product is reflected by the amount of stain signal produced. The use of such stains may decrease the cost and complexity of the extension reactions. The present disclosure can work well with such stains, since the objective is to measure the incorporation level of the labeled nucleotide relative to a standard. The simplicity and low cost of such stains could make the present disclosure the preferred method for SNP and tandem repeat detection in certain settings.

While fluorescent stains are preferred stains for the present disclosure, any polynucleotide stain (including chemiluminescence or phosphorescence) is also useful.

Useful polynucleotide stain may be a phenanthridinium dye, including monomers or homo- or heterodimers thereof, that give an enhanced fluorescence when complexed with polynucleotides. Examples of phenanthridinium dyes include ethidium homodimer, ethidium bromide, propidium iodide, and other alkyl-substituted phenanthridinium dyes.

Useful polynucleotide stain may be or may incorporate an acridine dye, or a homo- or heterodimer thereof, such as acridine orange, acridine homodimer, ethidium-acridine heterodimer, or 9-amino-6-chloro-2-methoxyacridine.

Useful polynucleotide stain may also be an indole or imidazole dye, such as Hoechst 33258, Hoechst 33342, Hoechst 34580 (BIOPROBES 34, Molecular Probes, Inc. Eugene, Oreg., (May 2000)) DAPI (4',6-diamidino-2-phenylindole) or DIPI (4',6-(diimidazolin-2-yl)-2-phenylindole).

Useful polynucleotide stain may also be a cyanine dye or a homo- or heterodimer of a cyanine dye that gives an enhanced fluorescence when associated with polynucleotides. Any of the dyes described in U.S. Patent No. 4,883,867 to Lee (1989), U.S. Patent No. 5,582,977 to Yue et al. (1996), U.S. Patent No. 5,321,130 to Yue et al. (1994), and U.S. Patent No. 5,410,030 to Yue et al. (1995) (all four patents incorporated by reference) may be used, including polynucleotide stains commercially available under the trademarks TOTO, BOBO, POPO, YOYO, TO-PRO, BO-PRO, PO-PRO and YO-PRO from Molecular Probes, Inc., Eugene, Oreg. Any of the dyes described in U.S. Patent No. 5,436,134 to Haugland et al. (1995), U.S. Patent No. 5,658,751 to Yue et al. (1997), and U.S. Patent No. 5,863,753 to Haugland et al. (1999) (all three patents incorporated by reference) may be used, including polynucleotide stains commercially available under the trademarks SYBR, SYTO, SYTOX, PICOGREEN, OLIGREEN, and RIBOGREEN from Molecular Probes, Inc. (Eugene, Oreg).

Still, useful polynucleotide stain may be a monomeric, homodimeric or heterodimeric cyanine dye that incorporates an aza- or polyazabenzazolium heterocycle, such as an azabenzoxazole, azabenzimidazole, or azabenzothiazole, that gives an enhanced fluorescence when associated with polynucleotides. This includes, but is not limited to, polynucleotide stains commercially available under the trademarks SYTO, SYTOX, JOJO, JO-PRO, LOLO, LO-PRO from Molecular Probes, Inc., (Eugene, Oreg).

Other useful polynucleotide stains include, but are not limited to, 7-aminoactinomycin D, hydroxystilbamidine, LDS 751, selected psoralens (furocoumarins), styryl dyes, metal complexes such as ruthenium complexes, and transition metal complexes (incorporating Tb3+ and Eu3+, for example). A preferred stain used in some embodiments of the disclosure is SYBR Green (by Molecular Probes Inc. Eugene, OR).

The polynucleotide stain is selected to have the desired relative polynucleotide binding affinity and spectral characteristics, according to methods well known in the art.

In a preferred embodiment, a nucleotide which is complementary to a potential variable nucleotide within the variable site of a polynucleotide is labeled with ROX. ROX's FRET donor SYBR Green is used to stain the amplified product. If the amplification permits the incorporation of the ROX-labeled nucleotide into the amplified product, ROX is then brought into close proximity to SYBR. Upon the excitement of SYBR, e.g., by a wavelength of light that excites SYBR, SYBR can serve as a FRET donor to excite ROX.

When ROX and SYBR are used as described above, the reaction well is illuminated with a wavelength of light that excites SYBR green dye. The emission of light from SYBR green is measured in the instrument with a filter that allows SYBR green emitted light to pass through, but blocks the wavelength of the excitation light. Measurement of the light emitted from ROX is measured in the instrument using a filter that permits the passage of light emitted from ROX, but blocks the wavelength of light used to excite and the wavelength emitted from SYBR green. The SYBR green dye does not emit a significant amount of light unless the SYBR green molecule is bound to double stranded DNA. The ROX molecules are not excited by the wavelength of light that is being used to illuminate the reaction vessel, so ROX does not emit a significant amount of light unless it is excited by light that has been emitted from SYBR green. Thus significant ROX signal is seen only when ROX has been incorporated into a DNA polymer that is also stained by SYBR green molecules. Since SYBR green is a non-specific double stranded DNA stain, the ROX molecules on the labeled nucleotide are stimulated upon the incorporation of the labeled nucleotide into the amplified product. The incorporation frequency is measured as a ratio of the SYBR green emitted light to the ROX emitted light. In PCR reactions, the ratio may vary with the cycle number, but is reproducible when measured at a defined number of cycles after the "Ct cycle" of the PCR reaction. Since the frequency of incorporated ROX labeled nucleotide will vary with the number of such nucleotide residue in the polymerase-synthesized portion of the DNA molecule, small differences in the polynucleotide sequences can be detected by measuring the ROX to SYBR signal ratio.

In another embodiment, one of the oligonucleotide primers used to amplify the region of interest is labeled with a fluorescent donor, and a nucleotide is labeled with a fluorescent acceptor. Once the labeled nucleotide gets incorporated into the extended product, the donor and acceptor are brought into close proximity so that a detectable signal is generated for measuring the incorporation frequency.

Quenching Nucleotide

The present disclosure provides a quenching nucleotide. Such quenching nucleotide does not emit a detectable signal (inactive) when not incorporated into the amplified product, but may emit a detectable signal (active), either directly or indirectly (e.g., by FRET) upon its incorporation into the amplified product. Alternatively, the quenching nucleotide may emit a first detectable signal when not incorporated into the amplified product, but emit a different (second) detectable signal upon its incorporation into the amplified product.

In one embodiment, a labeled nucleotide is quenched prior to its incorporation into the amplified product, but becomes active, i.e., detectable, upon incorporation into the amplified product. For example, this may be done by using a labeled nucleotide where a quencher is attached to the β or γ phosphate of the triphosphate region and the detectable label (e.g., a dye) is attached to other region of the nucleotide or the alpha phosphate. Upon incorporation into the amplified product, the beta and gamma phosphates are cleaved away by the extension enzyme and released into the reaction mixture solution, whereas the rest of the nucleotide is attached to the 3' end of the growing strand. Such a nucleotide would be quenched in its free substrate state, but would become active after being incorporated into the amplified product. This way, the reaction can take place in a single tube and the signal generated from the detectable label can be monitored without the need to remove the unincorporated nucleotides prior to measuring the incorporation frequency. Two different such labeled nucleotides could be used, and the ratio of incorporated label could be used to measure the relative incorporation frequency.

A nucleotide can alternatively incorporate a moiety capable of quenching the signal from a moiety used to quantitate polynucleotide product, e.g., the polynucleotide stain. Examples of quenchers useful according to this embodiment include the "Black Hole Quenchers" (Biosearch Technologies, Inc.). As an example, BHQ-10™ is capable of quenching the fluorescence signal from fluorescent molecules including SYBR Green. Without being bound to any one theory as to a mechanism of action, nucleotides modified with such quenchers, e.g., BHQ-10-dUTP, can be used in the methods according to the disclosure to increase the sensitivity of the assays.

Calculating The Incorporation Frequency

Amplified product is generally detected by detecting the incorporation of label into the primer by various direct or indirect methods. In some instances, amplified product is initially separated from unreacted reactants in the reaction mixture before it is detected. However, such separation is not required according to some embodiments of the disclosure and the methods can be performed in a homogenous format. In preferred embodiments, the amplified product is analyzed in a homogeneous assay without being separated from the unreacted reactants.

Separation of the amplified products from other reaction components can be achieved in a variety of ways. Methods for purifying amplified polynucleotide product are well known in the art, e.g., may be found in Innis et al., supra; Sambrook, et al. supra; and Ausubel et al., supra.

In one approach, the primer includes an attachment moiety that is one component of an affinity pair and that allows for affinity purification of amplified product from other components of the extension reaction. Typically, the attachment moiety is located at or near the 5' end of the primer. Alternatively, the attachment moiety is connected to the nucleotide. The other member of the affinity pair is frequently attached to a solid support such that extended primer bearing label can be bound to the support via the attached member of the affinity pair. Other reaction components can then be washed away. Another option besides the use of affinity purification is to separate extended primer from other reaction components using gel electrophoresis. Yet another option is to selectively inactivate the label associated with unincorporated nucleotide. Another approach is to attach the primer directly to a solid support, or bead, or other easily separatable substance. After primer extension, the extension product is detected and the incorporation frequency determined. If beads are used as a solid support, the beads may be labeled (Luminex) or encoded (Illumina) so that multiple different extension reactions can take place in a single tube. Alternatively multiple extension reactions could be carried out and detected with an array, where either the different primers are distributed at various locations on the array, or complementary capture polynucleotides are distributed at various locations on the array.

A variety of different attachment moieties can be used as part of an affinity pair to achieve purification of the extended primer from other components. In general terms, the attachment moiety and the other component of the affinity pair include two agents that are capable of specifically binding to one another. Examples of such binding pairs include, but are not limited to, polynucleotide/complementary polynucleotide, biotin/avidin, antigen/antibody and heavy metal/thiol group. In some instances, one member of the affinity pair is attached to a solid support or bead (or equivalent). A solution containing (or potentially containing) a primer bearing the complementary member of the affinity pair is then contacted with the support.

After allowing the two components an opportunity to bind and form a complex, other species in the extension reaction mixture can be washed from the support.

Thus, for example, in one suitable arrangement, the attachment moiety is a polynucleotide that serves as a 5' extension to the primer. A complementary nucleotide is attached to a solid support and is capable of selectively binding the extension primer.

Alternatively, an antigen functions as the attachment moiety and an antibody specific thereto is attached to the support. In yet another arrangement, a thiol group is linked to the primer and serves as the attachment moiety. A heavy metal group attached to the solid support can be used to selectively bind the thiolated primer.

The attachment moiety can be attached at any point of the primer where it does not interfere with the extension reaction. Most typically, the attachment moiety is attached at or near the 5' end of the primer. However, in some instances, the attachment moiety is connected to a more internal nucleotide.

Instead of attaching the attachment moiety to the primer, in some methods the attachment moiety is part of the nucleotide. The attachment moiety can be selected from the group of affinity pairs described above, for example (see, also, U. S. Patent No. 5,710,028). Alternatively, one can obtain antibodies specific to a fluorescent dye label on the nucleotide (e. g., an antibody elicited to fluorescein as a hapten). Such antibodies have been discussed (see, Voss, E. W., Jr. (Ed.) Fluorescein Hapten: An Immunological Probe).

A variety of different types of supports can be utilized in methods employing affinity-binding pairs. Suitable supports include, but are not limited to, beads, microparticles, the surface of a microtiter well, a filter, silicon and its derivatives and a glass slide. Similarly, the supports can be formed from any material stable to the binding and washing conditions including, for example, glass, polystyrene, cellulose, latex, nitrocellulose, nylon, polyacrylamide, dextran and agarose.

As an alternative to the use of affinity binding pairs, extended primers can be separated from other reaction components by a variety of size based separation techniques such as gel electrophoresis and size exclusion chromatography (e. g., HPLC). In some methods, separation of components by gel electrophoresis and the detection step (typically detection of fluorescence from a fluorescent label attached to the extended primer via the incorporated nucleotide) is performed using a single integrated instrument, such as the PrizmDNA Sequencer from Applied Biosystems, and MegaBACE from Amersham Biosciences.

Preferably, the subject method(s) of the present disclosure are conducted in a homogenous assay format in which extension products do not need to be separated from other extension reaction components (e. g., unincorporated nucleotide). In some methods, this is accomplished using donor and acceptor fluorophores, including fluorescence resonance energy transfer pairs. The fluorophores are chosen so that the emission spectrum of one fluorophore (i. e., the donor fluorophore) overlaps the excitation spectrum of the other fluorophore (i. e., the acceptor fluorophore).

The present disclosure employs different ways for the determination of incorporation frequency of a labeled nucleotide. Preferably, the frequency is measured as a ratio in the present disclosure.

In one embodiment, the incorporation frequency is calculated as a ratio between the level of a labeled nucleotide incorporated into the amplified product and the amount of the amplified product. Any known method known in the art for measuring the amount of polynucleotide may be used in determining the amount of the amplified product, for example, as described herein above and as in Current Protocols in Molecular Biology (1997, Ausubel et al., John Weley & Sons, Inc.). Any instruments available for measuring the amount of PCR amplicon (i.e., yield) during the amplification reaction may be used for measuring the amount of the amplified product or fluorescent intensity in the present disclosure. A non-limiting example of such an instrument is the Mx4000 Multiplex Quantitative PCR System by Stratagene (La Jolla, California).

In another embodiment, the incorporation frequency is calculated as a ratio of the levels of two or more labeled nucleotides incorporated into the amplified product. The labeled nucleotides preferably present in different numbers in different alleles or variants and are labeled differentially. A reference ratio may be calculated without performing an amplification reaction if the sequence variation is known between two alleles. For example, for an allele A which contains 5 Ts and 4 Cs in its sense strand, the ratio would be 5/4 if differentially labeled dTTP and dCTP are used in the extension reaction; while for an allele B which contains the identical sequence as allele A except that one A-T base pair is replaced by one G-C base pair, the ratio would be 4/5 if differentially labeled dTTP and dCTP are used in the extension reaction. These ratios can be obtained without performing an amplification reaction and be used as reference ratios to determine whether a polynucleotide template (e.g., a target polynucleotide) contains the sequence of allele A or allele B. Due to factors related to the sensitivity of the measuring instrument, the ratio of label signal emitted by each allele may not be in exact proportion to the ratio of labeled nucleotides in the allele. For example, the "gain" or sensitivity of the instrument may be different for each label used, thus changing the ratio by the factor by which the sensitivity is different. This is accommodated by calibrating the instrument against known standard alleles. Also, the "ratio of ratios" (one allele's ratio divided by the other allele's ratio) will always be a predictable ratio regardless of the instrument's sensitivity for each label. For example, if the theoretical ratio for allele A is 5/4, and the theoretical ratio for allele B is 4/5, the ratio of ratios will be A/B=5/4 ÷ 4/5=25/16. Regardless of the instrument's specific sensitivity to one label or another, the ratio of ratios will be 25/16. If two unknown samples both contain only allele A or allele B, the ratio of ratios will be 1. But if unknown sample 1 is allele A and unknown sample 2 is allele B, the ratio of ratios will be 25/16. If the alleles in the samples are reversed, the ratio of ratios will be 16/25.

In some embodiments, the labeled nucleotide bears one member of the donor/acceptor dye pair and the other member is attached to the amplified product. Upon the incorporation of the labeled nucleotide the donor and acceptor are brought into an energy transfer relationship, wherein fluorescence energy can be transferred from the donor to the acceptor. By measuring fluorescence changes that occur as a consequence of energy transfer (e. g., a decrease in the fluorescence intensity of the donor or an increase in the fluorescence intensity of the acceptor), one can detect the incorporation of label into the amplified product without having to separate the amplified product from unreacted reactants. Specific labels suitable for use in such methods are discussed infra in the section on labeled nucleotides. Further guidance on such methods is described in U. S. Patent No. 5,945,283, incorporated hereby by reference.

In one embodiment, the reaction mixture for polynucleotide amplification contains a labeled nucleotide (e.g.; a dCTP) and other non-labeled nucleotides that are not the labeled nucleotide (e.g., dATP, dTTP and dGTP).

In a preferred embodiment of the disclosure, polynucleotide amplification is carried out in the presence of three non-labeled nucleotides (e.g., dATP, dTTP, and dGTP) and a mixture of labeled nucleotide and non-labeled fourth nucleotide (e.g., a dCTP). For each of the nucleotides added as a mixture, typically the labeled nucleotide is at least 0.001% (e.g., 0.1%, 0.5%, 1%, 2%, 3%, 4%, 5%, or 10%, 20%, 30%, 40%, 50% or more, and up to 100%) of the total concentration of the nucleotide, i.e., a percentage of the sum of the labeled and non-labeled forms of that nucleotide on

a molar or μg basis.

The optimal relative amount of the labeled nucleotide and the same non-labeled nucleotide (e.g., labeled dCTP and non-labeled dCTP) may be determined by one skilled in the art according to the specific reaction conditions, including the sequence of the region of interest. The amount is to be selected to provide both sensitivity and accuracy required for the method. Once selected the relative amount of the labeled nucleotide and the non-labeled nucleotide should remain the same for different polynucleotides of interest to be compared.

If two polynucleotides (i.e., the first and the second polynucleotides) are different at a single nucleotide position X (such as with a SNP) and the labeled nucleotide is incorporated during the synthesis of the first polynucleotide but not the second polynucleotide (as a result of their sequence difference), then the incorporation frequency of the labeled nucleotide into synthesis product from the second polynucleotide may be 0 (e.g., as measured as the level of incorporated level against the amount of amplified product) and the incorporation frequency of the labeled nucleotide into amplified product from the first polynucleotide is greater than 0. Therefore, by comparing the incorporation frequencies for the two polynucleotides, one can conclude that there is a sequence variation within the synthesized sequence between the first and the second polynucleotides. It is understood that such comparison may be performed between a polynucleotide of interest (e.g., the first or the second polynucleotide) with a control polynucleotide.

For example, suppose the existence of two alleles that comprise either an A/T or a G/C base pair at a predetermined position X in their otherwise identical nucleotide sequences. If PCR primers are designed to flank the A/T or G/C variable sequence and PCR is carried out in the presence of all four dNTPs plus a labeled dCTP, then the A/T allele would be amplified with one fewer potential labled dCTP incorporated into the amplified product, while the G/C allele would be amplified with one or more potential labeled dCTP incorporated into the amplified product. An incorporation frequency of the labeled dCTP may be determined as the level of the detectable label. Thus there would be different incorporation frequency of the labeled dCTP between two alleles.

Also for example, suppose the A/T allele contains 5 T's and 4 C's on the sense strand within the region of interest replicated downstream of the primer (the primer sequence itself is supplied in the assay and is not enzymatically synthesized - however its complementary strand will be enzymatically synthesized in a PCR reaction), and the G/C allele contains the same sequence except that one A/T basepair has been replaced with a G/C basepair. Thus the G/C allele would contain 4 Ts and 5 Cs in the enzymatically synthesized region of the sense strand within the region of the interest. During PCR of the double strand A/T allele to produce a double stranded amplified product, a total of 5 Ts and 4 Cs would be incorporated each time the sense strand of the allele was replicated during PCR. In contrast, during PCR of the G/C allele, a total of 4 Ts and 5 Cs would be incorporated each time the sense strand of the allele was replicated during PCR. If PCR is carried out in the presence of labeled dCTP, the G/C allele would have a greater labeled dCTP incorporation frequency in the sense strand than that of the A/T allele. Similar changes could occur in the antisense strand, depending on the position of the antisense primer. The presence of each allele would therefore be indicated by the incorporation frequency measured.

The incorporation frequency may be measured as a ratio between the level of the incorporated labeled nucleotide (e.g., dCTP) and the total amount of amplified product generated. In one embodiment, the total amount of amplified product is determined by SYBR green staining and the labeled nucleotide is labeled with a ROX dye. The incorporation of the labeled nucleotide into SYBR stained amplified product leads to the generation of a detectable FRET signal representing the level of incorporation (e.g., as described above herein).

Alternatively, in the above example, if we carry out PCR in the presence of labeled dCTP and distinguishably labeled dTTP, the resulting allelic amplicons would contain different ratios of the labels associated with dTTP and dCTP. For example suppose we label dTTP with Cy3 and labeled dCTP with Cy5, then the A/T allele (5 Ts, 4 Cs) would contain a 5/4 ratio of Cy3/Cy5 and the G/C allele (4Ts, 5Cs) would contain a 4/5 ratio of Cy3/Cy5.

The level of the detectable label, the accuracy and sensitivity of the incorporation frequency, may be adjusted by the relative concentration of the labeled nucleotide placed in the reaction mixture. The higher the concentration of the labeled nucleotide, the greater the difference in the absolute incorporation amount in the amplification of the different alleles.

The disclosure can be used as well for the differentiation of tandem repeat alleles. Different individuals can have different numbers of the tandem repeats. The exact number of copies of the repeated sequence can be used as an allele to distinguish a particular genetic region, chromosome, or person. Until now the number of repeat units in the allele has been measured by determining the length of a PCR amplicon containing the repeat region. The measurement has typically been carried out using electrophoresis. In the present disclosure, PCR amplification of alleles containing different numbers of the repeat sequence will occur in the presence of at least one labeled nucleotide which may be incorporated into the amplified product of the region of interest containing the tandem repeat. The incorporation frequency of the labeled nucleotide can be determined by calculating the ratio of the level of label incorporation against the total amount of amplified product produced, as described above. When an increase in tandem repeat units between flanking primers results in an increase in the number of nucleotides corresponding to a labeled nucleotide, the ratio of labeled nucleotide incorporation to total amplified DNA will increase. For example, if the primers contain few G:C basepairs, but the repeats are rich in G:C base pairs, the frequency of G or C incorporation will increase with higher numbers of G:C rich repeat units in the amplicon.

For example, if two polynucleotides (i.e., A and B) comprise a different number of tandem repeats, the labeled nucleotide is then incorporated at a different number of nucleotide locations during the synthesis of polynucleotide A compared to polynucleotide B (as a result of the frequency of a particular nucleotide present in their sequences).

Suppose there are two alleles as follows:

*Allele A*:    >>>>>>>>AAA CAG CAG CAG **CAG CAG**>>>>>>>>>
(SEQ ID NO: 1)
>>>>>>>>TTT GTC GTC GTC **GTC GTC**>>>>>>>>>
(SEQ ID NO: 2)

*Allele B*:    >>>>>>>>AAA CAG CAG CAG>>>>>>>> (SEQ ID NO: 3)
>>>>>>>>TTT GTC GTC GTC>>>>>>>> (SEQ ID NO: 4)

If a labeled dCTP is used in the reaction mix for the amplification of both alleles, in producing a double stranded amplified product, allele A has 10 instances of C in the repeated region; and the allele B has 6 instances of C in the repeated region. If all other reaction conditions were equal, one would expect the incorporation frequency of C for the polynucleotide comprising a greater number of tandem repeats to be higher than that for the polynucleotide comprising less number of tandem repeats. Thus, if there are no other Cs to be incorporated in the amplicon, when we calculate the incorporation frequency, e.g., as a ratio between the level of labeled dCTP incorporated against the total amount of amplified product, the incorporation frequency for allele A will be 10/6 (i.e., about 1.667) times of that for allele B.

When, on the other hand, there are other nucleotides corresponding to the labeled nucleotide in the amplified region but outside the variable repeat unit, the incorporation frequency will differ between alleles, but the numerical relationship between labeling intensity will not be simply the ratio of the number of labeled nucleotides in the repeat units. That is, the numerical relationship will be influenced by the presence of the labeled nucleotides incorporated outside the repeat unit. This is exemplified below.

*Allele A*:    >>>>>>>>CAG CAG CAG **CAG CAG**>>>C>C>C>>>> 8 Cs
(SEQ ID NO: 5)
>>>>>>>>GTC GTC GTC **GTC GTC**>>>G>G>G>>>> 5 Cs
(SEQ ID NO: 6)

*Allele B*:    >>>>>>>>CAG CAG CAG>>>C>C>C>>>> 6 Cs (SEQ ID NO: 7)
>>>>>>>>GTC GTC GTC>>>G>G>G>>>> 3 Cs (SEQ ID NO: 8)

If labeled dCTP is used in the reaction mix for the amplification of both alleles, the replication of Allele A will result in the incorporation of 13 Cs and the replication of Allele B will result in the incorporation of 9 Cs, and Allele A will exhibit 1.44X the label of Allele B (compare with 1.667 in the example above). Thus, while the incorporation frequency will increase as the number of repeat units increases, the frequency differs from the ratio of Cs in the repeat units alone. Knowledge of the number of positions for incorporation of label outside the repeat unit, which stays constant as repeats are added or removed, will permit the calculation of differences in the number of repeats.

In the preferred embodiments, these PCR reactions generate quantifiable signals, and are performed either separately or in multiplexed fashion.

Instruments are available for measuring the level of the incorporation and the amount of PCR amplicon during (e.g., in real time) and/or after the amplification reaction (QPCR reactions and instruments), for example, the MX4000 (Stratagene) detects the quantity and rate of synthesis of PCR amplicons in real time, and may detect multiple light wavelenths in the same amplification reaction

DNA sequencing methods known in the art may further determine the sequence identity of a polynucleotide template of the present disclosure.

General Criteria For Designing Oligonucleotide primers

Useful oligonucleotide primers of the disclosure can be obtained by biological synthesis or by chemical synthesis. For short sequences (up to about 100 nucleotides) chemical synthesis is frequently more economical as compared to biological synthesis. For longer sequences standard replication methods employed in molecular biology can be used such as the use of M13 for single stranded DNA as described by Messing, 1983, Methods Enzymol. 101: 20 - 78. Chemical methods of oligonucleotide synthesis include phosphotriester and phosphodiester methods (Narang, et al., Meth. Enzymol. (1979) 68:90) and synthesis on a support (Beaucage, et al., Tetrahedron Letters. (1981) 22:1859 - 1862) as well as the phosphoramidate technique, Caruthers, M. H., et al., Methods in Enzymology (1988)154:287 - 314 (1988), and others described in "Synthesis and Applications of DNA and RNA," S. A. Narang, editor, Academic Press, New York, 1987, and the references contained therein.

Oligonucleotides for use as primers, e.g., in PCR or non-thermal amplification reactions, are typically synthesized chemically according to the solid phase phosphoramidite triester method described by Beaucage and Caruthers (1981), Tetrahedron Letts., 22(20):1859-1862, e.g., using an automated synthesizer, as described in Needham-VanDevanter et al. (1984) Polynucleotides Res., 12:6159-6168. Oligonucleotides can also be custom made and ordered from a variety of commercial sources known to persons of skill. Purification of oligonucleotides, where necessary, is typically performed by either native acrylamide gel electrophoresis or by anion-exchange HPLC as described in Pearson and Regnier (1983) J. Chrom. 255:137-149. The sequence of the synthetic oligonucleotides can be verified using the chemical degradation method of Maxam and Gilbert (1980) in Grossman and Moldave (eds.) Academic Press, New York, Methods in Enzymology 65:499-560.

While primers can hybridize to any of a number of sequences, selecting optimal primers may be done using computer assisted consideration of available sequences and excluding potential primers which do not have desired hybridization characteristics, and/or including potential primers which meet selected hybridization characteristics. This is done by determining all possible polynucleotide primers, or a subset of all possible primers with selected hybridization properties (e.g., those with a selected length, G:C ratio, uniqueness in the given sequence, etc.) based upon the known sequence. The selection of the hybridization properties of the primer is dependent on the desired hybridization and discrimination properties of the primer. In general, the longer the primer, the higher the melting temperature. As noted above, any desired primer can be synthesized using standard methods.

In general, it is expected that one of skill is thoroughly familiar with the theory and practice of polynucleotide hybridization and primer selection. Gait, ed. Oligonucleotide Synthesis: A Practical Approach, IRL Press, Oxford (1984); W. H. A. Kuijpers Polynucleotides Research 18(17), 5197 (1994); K. L. Dueholm J. Org. Chem. 59, 5767-5773 (1994); S. Agrawal (ed.) Methods in Molecular Biology, volume 20; and Tijssen (1993) Laboratory Techniques in biochemistry and molecular biology--hybridization with polynucleotide probes, e.g., part I chapter 2 "overview of principles of hybridization and the strategy of polynucleotide probe assays", Elsevier, N.Y., provide a basic guide to polynucleotide hybridization. Innis supra provides an overview of primer selection.

One of skill will recognize that there are a variety of possible ways of performing the primer selection steps, and that variations on the steps are appropriate. Most typically, selection steps are performed using simple computer programs to perform the selection as outlined above; however, all of the steps are optionally performed manually. Available computer programs for primer selection include, but are not limited to, Accelrys (San Diego, CA), Lab Tools (Stratagene website labetools, stratagene.com), oligo 6 by Molecular Biology Insights (Cascade, CO). An alternate program is the MFOLD program (developed by Dr. Michael Zuker, see world wide web mfold.burnet.edu.au) which predicts secondary structure of, e.g., single-stranded polynucleotides. In addition to programs for primer selection, one of skill can easily design simple programs for any or all of the preferred selection steps.

In one embodiment, at least one oligonucleotide primer in a polynucleotide amplification reaction hybridizes immediately 5' of the region of interest on one strand.

In another embodiment, two oligonucleotide primers each hybridize immediately flanking the region of interest on each.

In another embodiment, the primers are not hybridized immediately adjacent to the region of interest, leaving at least one nucleotide between the hybridization sites and the region of interest.

In some applications, the primers used in the present disclosure may be specifically designed.

In one embodiment, two SNP alleles are transversions of each other, for example A/T versus T/A. In this case, if primers are used that flank the region of interest, e.g., the transversion site, a labeled nucleotide, e.g., dTTP, would be incorporated into the replica of the "upper" strand in the first allele, but would also be incorporated into the replica of the "lower" stand in the second allele. Thus both alleles would have the same total dTTP incorporation frequency so that the allele present in the amplification reaction would not be determined. This can be addressed by designing one of the PCR primers to hybridize with the variable region. For example, the two alleles below contain a single nucleotide transversion:

Upstream primer

5'>>>>>>CCTAGGACT3' (SEQ ID NO: 9)

Allele 1       5'>>>>>>CCTAGGACTACCGGCAAGT>>>>>>3' (SEQ ID NO: 10)
3'>>>>>>GGATCCTGATGGCCGTTCA>>>>>>5' (SEQ ID NO: 11)

3'TGGCCGTTCA>>>>>>5' (SEQ ID NO: 12)

Downstream primer

Upstream primer

5'>>>>>>CCTAGGACT3' (SEQ ID NO: 9)

Allele 2       5'>>>>>>CCTAGGACTTCCGGCAAGT>>>>>>3' (SEQ ID NO: 13)
3'>>>>>>GGATCCTGAAGGCCGTTCA>>>>>>5' (SEQ ID NO: 14)

3'AGGCCGTTCA>>>>>>5' (SEQ ID NO: 15)

Downstream primer

There is a single nucleotide difference in both strands at the bolded position. Primers are shown as both "upstream" and "downstream" PCR primers above and below each double stranded allele. In this embodiment, the "upstream" primer is designed to not hybridize with the variable nucleotide, but the downstream primer does. In addition, the same upstream primer serves to prime both alleles, but different downstream primers are used for the two different alleles. If all three of these primers are put into a single PCR reaction together with a labeled nucleotide, e.g., dTTP, then it will be incorporated into the amplified product of allele 2 in higher frequency, while if a labeled dATP is used in the reaction mixture instead of the labeled dTTP, the labeled dATP will be incorporated into the amplified product of allele 1 in higher frequency. In this way, a differential incorporation frequency of a labeled nucleotide will be detected for allele 1 and allele 2, therefore the presence of either allele 1 or allele 2 in the reaction mixture can be determined. The two different downstream primers can each be designed with 5' tails that do not hybridize to the target nucleic acid. These 5' tails may contain a high proportion of the complement of a labeled nucleotide, thus driving the incorporation frequency far in one direction. If the 5' tails of the two downstream primers contain different nucleotide frequencies, the incorporation frequencies of two different alleles can be driven in opposite directions by the choice of nucleotide composition in the 5' tails. For example, if the downstream primer specific for allele 1 contains a non-hybridizing 5' region (tail) that is rich in T nucleotides (for example 5 of 10 non-hybridizing nucleotides are T and there are no As in this 5' tail) then when this tail is replicated during subsequent PCR cycles the replication enzyme will place 5 additional As into the amplicon in the upper strand complementary to the 5' tail. If the downstream primer specific for allele 2 contains a 5' tail containing 5 A nucleotides but no T nucleotides, then when this tail is replicated during subsequent PCR cycles the replication enzyme will place 5 additional Ts into the amplicon. Thus the non-hybridizing 5' tail of the downstream primer specific for allele 1 will produce a more A-rich amplicon; and the non-hybridizing 5' tail of the downstream primer specific for allele 2 will product a more T-rich amplicon. This will enhance the difference in incorporation frequencies of A and T nucleotides in these two alleles.

In an embodiment where the alleles do not conserve the same nucleotides, then such a primer design is not needed. For example,

Upstream primer

5'>>>>>>CCTAGGACT  (SEQ ID NO: 9)

Allele 1      5'>>>>>>CCTAGGACTACCGGCAAGT>>>>>>3'  (SEQ ID NO: 10)
3'>>>>>>GGATCCTGATGGCCGTTCA>>>>>>5'  (SEQ ID NO: 11)

3'GGCCGTTCA>>>>>>5'  (SEQ ID NO: 16)

Downstream primer

Upstream primer

5'>>>>>>CCTAGGACT3'  (SEQ ID NO: 9)

Allele 2      5'>>>>>>CCTAGGACTCCCGGCAAGT>>>>>>3'  (SEQ ID NO: 17)
3'>>>>>>GGATCCTGAGGGCCGTTCA>>>>>>5'  (SEQ ID NO: 18)

3'GGCCGTTCA>>>>>>5'  (SEQ ID NO: 16)

Downstream primer

In the example above only two primers (may be the same for both alleles) are needed and there may be no need to have either primer hybridize with the variable position because the amplified product of allele 1 will be able to incorporate a labeled A or T, while the amplified product of allele 2 will be able to incorporate a labeled G or C. Therefore by including a labeled A or T, or a labeled G or C in the reaction mixture, the two alleles will demonstrate different incorporation frequencies which indicates the presence of either allele 1 or allele 2 (or both) in the reaction.

Where primers not immediately flanking the variable region are used, i.e., leaving at least one nucleotide between the primer hybridization site and the variable region, the labeled nucleotide can be incorporated in a template-dependent manner into the regions that flank the variable regions. In the example shown above, the DNA sequence flanking the variable position has instances of adenine (A). A DNA polymerase can incorporate a labeled dATP at any position where it would normally incorporate a dATP. Since DNA polymerization begins by adding nucleotides to a primer, any sequence "downstream" or 3' relative to the primer will be synthesized by the polymerase and will be susceptible to the incorporation of the labeled nucleotide. In the upper allele shown above, the variable nucleotide in the upper strand is A followed by CCGGCAAGT>>>>>>3' (SEQ ID NO: 29). In this case, a labeled dATP could be incorporated at any of the A positions. Based on the above disclosure, in assessing the incorporation frequency of a labeled nucleotide, it is understood that one must consider all potential sites of incorporation in the 3' direction of each primer. Due to the existence of the variable region, the incorporation frequency between the two alleles will still be different. In allele 1 shown above there are 3 instances of A 3' of the upstream primer in the upper strand and 2 instances of A 3' of the downstream primer in the lower strand. In allele 2 there are only 2 instances of A where a labeled dATP can be incorporated in either strand.

The incorporation of a labeled nucleotide at regions flanking the variable site may reduce the sensitivity of the subject methods by minimizing the difference of incorporation frequency between two alleles. For example, in the allele pair shown above a labeled dATP will give a higher incorporation frequency for allele 1 (with 5 A's incorporated) than for allele 2 (with 4 A's incorporated), i.e., 1.25 times the incorporation frequency of allele 2.

Amplification Reaction

Once the region comprising or potentially comprising a sequence difference is selected, the region may be subjected to a polynucleotide amplification reaction according to techniques known to those with skill in the art, to produce synthesized products. In a polynucleotide amplification reaction, according to the disclosure, one or more polynucleotides of interest would serve as templates for the synthesis using at least a pair of oligonucleotide primers with opposite orientation. The oligonucleotide primers with opposite orientation preferably hybridize to sequences on a template flanking a potential sequence difference.

In a preferred embodiment, the polynucleotide synthesized by this reaction comprises double stranded polynucleotide strands comprising a sequence between the sites to which the two primers with opposite orientation hybridize. In a particularly preferred embodiment, the polynucleotide synthesis method is PCR where the polynucleotide being analyzed is DNA, or is RT-PCR where the polynucleotide being analyzed is RNA, though the templates can be produced by any suitable synthesis method for the polynucleotide being analyzed as will be understood by those with skill in the art with reference to this disclosure. Suitable kits for performing PCR and RT-PCR are available from a number of commercial suppliers, including Amersham Pharmacia Biotech, Inc. (Piscataway, NJ); Life Technologies, Inc. (Gaithersburg, MD); and Perkin-Elmer, Corp. (Norwalk, CT); Stratagene (La Jolla, CA); among other sources.

Although a variety of polynucleotide amplification methods have been developed, the general approach of such methods is nonetheless quite similar. In brief, the methods involve hybridizing a primer that is complementary to a polynucleotide template such that the 3' end of the primer hybridizes adjacent to, but does not span, the region of interest, e.g., the variant site of the polynucleotide template. The hybridization is typically performed in the presence of one or more labeled nucleotides complementary to a nucleotide that potentially occupies the variant site. Hybridization is performed under conditions allowing primer extension if a nucleotide complementary to a base occupying the variant site in the polynucleotide template is present. Extension results in the incorporation of a labeled nucleotide, thereby generating a labeled amplified product.

Amplified products are detected and provide an indication of which base(s) occupy the site of variation in the polynucleotide template. Examples of such primer extension methods include, but are not limited to, U. S. Patent Nos. 5,710,028; 5,856,092; 5,846,710; 5,888,819 and 6,004,744; and PCT publication WO 92/16657, each of which is incorporated by reference in its entirety. The methods of the disclosure are generally applicable to these methods and other related amplification methods.

Typically, the method(s) of the disclosure begin with the treatment of a sample that includes a duplex polynucleotide template to obtain unpaired nucleotides that at least span the variant site of interest or, alternatively, to obtain separate strands. Of course, if the polynucleotide template is already single-stranded, such a step is unnecessary. The term "polynucleotide template" as used herein refers to single-or double-stranded polynucleotide that includes at least one of the variant sites being interrogated. For double-stranded polynucleotides, the variant site includes the nucleotide at the site being examined and the complementary nucleotide in the complementary strand. If a double-stranded polynucleotide template is denatured to form two single strands, each strand can be considered a polynucleotide template and either strand can serve as a template in the methods of the disclosure.

Strand separation can be achieved using various denaturing conditions that are known in the art including, for example, heat, alkali, formamide, urea, glyoxal and combinations thereof. Typically, strand separation is achieved using heat denaturation at temperatures ranging from 80°C to about 105°C for time periods ranging from about 1 to 10 minutes. Numerous protocols teach the performance of an initial, long denaturation step, particularly when using complex polynucleotides as a starting template, e.g. genomic DNA. In certain embodiments, the present disclosure will include such initial, longer denaturation steps due to the use of genomic DNA as template. Alternatively, single-stranded template can be generated through degradation of one strand by exonucleases (see, e. g., Somers et al, Biochimica et Biophysica Acta 1379: 42-52 (1998); Nikiforov et al, PCR Methods and Applications 3: 285-291 (1994); Higuchi and Ochman, Nucleic Acids Research 17: 5865 (1989); and Straus and Zagursky, Biotechniques 10: 376-384 (1991)).

After strand separation, a primer is then annealed under hybridizing conditions to a template strand of the polynucleotide template (annealing). The primer is capable of specifically hybridizing to a segment of the polynucleotide template such that its 3' end is adjacent to the region of interest, e.g., a variant site on the target nucleic acid. As used herein, the term "adjacent to", when used in reference to hybridization between the primer and polynucleotide template typically means that the primer hybridizes to the polynucleotide template so that its 3' end is immediately 5' to the variant site. However, the 3' end can be located several (e.g., 1, 2, 3, 4, 5, or more) or many (e.g., 10, 20, 30, 50, 100 or more) nucleotides 5' to the variant site. As known to those of skill, optimal annealing temperatures depend on the melting temperature for the primer and templates, typically falling in the range from about 40°C to about 65°C. Numerous methods of varying simplicity and precision are known to calculate the melting temperature of polynucleotides. Any such method can be used in the present disclosure.

The nucleotide(s) included in extension reactions can be any of the naturally occurring deoxynucleotides (i. e. dATP, dGTP, dTTP and dCTP) or derivatives, so long as the nucleotide can be incorporated at the 3' end of a primer in a template-dependent fashion.

An extendible nucleotide refers to nucleotides to which another nucleotide can be attached at the 3' position of the sugar (e. g., the hydroxyl group in the naturally occurring deoxynucleotides dATP, dTTP, dCTP and dGTP) during amplification reaction.

Optimal temperatures for the extension step of a PCR are also extensively taught in the prior art. Generally, this temperature is between about 70°C and about 80°C. Often, the temperature of 72°C is cited as the ideal temperature for extension. Other temperatures may, however, be used, e.g., depending on the polymerase used and other factors well known in the art.

When extendible nucleotides are included, a variety of techniques can be used to control the extent of the extension reaction. Such techniques include, controlling polymerase concentration, limiting extension reaction times and conducting extension reactions at low temperatures.

It is understood that the amplification efficiency is intrinsic to the amplification reaction, and depends on many variables such as the polymerase, the primers, the annealing temperature, the buffer, the template, etc. However the amplification efficiency should be approximately the same for each allele if the amplification reaction conditions are kept the same, e.g., same reagent concentrations and same cycling condition.

The subject disclosure measures a differential incorporation frequency of a labeled nucleotide that can be differentially incorporated into copies of two or more polynucleotide template sequences. Amplification schemes that repeat the replication step multiple times will enhance the differential in yield of final amplification product. The process is particularly well suited to PCR and is amenable to quantitative PCR measurements, especially using stains such as SYBR Green. However it will be appreciated by those skilled in the art that there are ways of determining the frequency of incorporation of a labeled nucleotide that do not require the staining of the total nucleic acid, or do not require the use of a second labeled nucleotide. For example, if one or more primers are attached to a solid support ( for example a bead, planar surface, array format, etc) and the extension reactions are run to completion, and one knows the density of primers attached to the solid support, one can determine the incorporation frequency of labeled nucleotide.

In some embodiments of the disclosure, up to four different nucleotides, i.e., one, two, three, or four of the dATP, dTTP, dGTP, dCTP, may be labeled in the same amplification reaction for the subject method of the disclosure. If unnatural nucleotides are used, then more than 4 labels may be used. Each labeled nucleotide comprises a different label and emits a distinguishable signal which correlates with the structure (i.e., identity) of the nucleotide. The labeled nucleotide may be used at a concentration range similar to that of a dNTP used in the PCR reaction.

Reagents for the practice of PCR and related reactions are amply described in the art.

Buffers for PCR and related reactions can be easily made using standard laboratory chemicals according to recipes provided in the above-cited protocols. Alternatively, buffers and additional reagents useful for PCR can be commercially obtained from any of a variety of companies such as BRL, Sigma, Perkin-Elmer, Roche, Boehringer Mannheim, Stratagene, NEB, and others. Such companies and the above references provide substantial guidance for the optimal use of such buffers. Nucleoside triphosphates can also be readily obtained commercially. In addition, guidance for their use can be found in any of a multitude of sources including guides such as Innis, Sambrook, Ausubel, etc. supra. Similarly, other reagents commonly used in cyclic polymerase-mediated reactions such as $Mg^{2+}$ ions, BSA, detergents, etc, can be readily obtained and guidance for their optimal use readily found in any of the above sources.

A wide variety of DNA polymerases maybe used in the subject methods. Suitable DNA polymerases for use in the subject methods may or may not need to be thermostable. Known conventional DNA polymerases include, for example, *Pyrococcus furiosus* (Pfu) DNA polymerase (Lundberg et al., 1991, Gene, 108:1, provided by Stratagene), *Pyrococcus woesei* (Pwo) DNA polymerase (Hinnisdaels et al., 1996, Biotechniques, 20:186-8, provided by Boehringer Mannheim), *Thermus thermophilus* (Tth) DNA polymerase (Myers and Gelfand 1991, Biochemistry 30:7661), *Bacillus stearothermophilus* DNA polymerase (Stenesh and McGowan, 1977, Biochim Biophys Acta 475:32), *Thermococcus litoralis* (Tli) DNA polymerase (also referred to as Vent DNA polymerase, Cariello et al., 1991, Polynucleotides Res, 19: 4193, provided by New England Biolabs), 9°Nm DNA polymerase (discontinued product from New England Biolabs), *Thermotoga maritima* (Tma) DNA polymerase (Diaz and Sabino, 1998 Braz J. Med. Res, 31:1239), *Thermus aquaticus* (Taq) DNA polymerase (Chien et al., 1976, J. Bacteoriol, 127: 1550), *Pyrococcus kodakaraensis* KOD DNA polymerase (Takagi et al., 1997, Appl. Environ. Microbiol. 63:4504), JDF-3 DNA polymerase (from *thermococcus sp*. JDF-3, Patent application WO 0132887), *Pyrococcus GB-D* (PGB-D) DNA polymerase (also referred as Deep-Vent DNA polymerase, Juncosa-Ginesta et al., 1994, Biotechniques, 16:820, provided by New England Biolabs), UITma DNA polymerase (from *thermophile Thermotoga maritima*; Diaz and Sabino, 1998 Braz J. Med. Res, 31:1239; provided by PE Applied Biosystems), Tgo DNA polymerase (from *thermococcus gorgonarius*, provided by Roche Molecular Biochemicals), *E. coli* DNA polymerase I (Lecomte and Doubleday, 1983, Polynucleotides Res. 11:7505), T7 DNA polymerase (Nordstrom et al., 1981, J. Biol. Chem. 256:3112), and archaeal DPI/DP2 DNA polymerase II (Cann et al., 1998, Proc Natl Acad Sci U S A 95:14250-5).

For thermocyclic reactions, the polymerases are thermostable polymerases such as Taq, Deep Vent, Tth, Pfu, Vent, and UITma, each of which are readily available from commercial sources. Similarly, guidance for the use of each of these enzymes can be readily found in any of a number of protocols found in guides, product literature, the Internet (see, for example, www.alkami.com), and other sources.

For non-thermocyclic reactions, and in certain thermocyclic reactions, the polymerase will often be one of many polymerases commonly used in the field, and commercially available, such as DNA pol 1, Klenow fragment, T7 DNA polymerase, and T4 DNA polymerase. In applications for RNA amplification, a number of RNA polymerases are also commercially available, such as T7 RNA polymerase, T3 RNA polymerase and SP6 RNA polymerase. Guidance for the use of such polymerases can readily be found in product literature and in general molecular biology guides such as Sambrook or Ausubel, both supra.

Polymerases can also incorporate labeled (e.g., fluorescent) nucleotides or their analogs during synthesis of polynucleotides. See, e.g. Hawkins et al., U.S. Patent No. 5,525,711, where the use of nucleotide analogs which are incorporatable by Taq is described.

The methods of this disclosure can generally be carried out using standard reaction conditions and reagents unless specified. Such reagents and conditions are well known to those of skill in the art, and are described in numerous references and protocols. See, e.g. Innis supra; Sambrook, supra.; Ausubel, et al., eds. (1996) Current Protocols in Molecular Biology, Current Protocols, a joint venture between Greene Publishing Associates, Inc. and John Wiley & Sons, Inc. Also, see, Mullis et al., (1987) U.S. Patent No. 4,683,202, and Arnheim & Levinson (1990) C&EN 6-47, The Journal Of NIH Research (1991) 3, 81-94; Kwoh et al. (1989) Proc. Natl. Acad. Sci. USA 86, 1173; Guatelli et al. (1990) Proc. Natl. Acad. Sci. USA 87, 1874; Lomell et al. (1989) J. Clin. Chem 35, 1826; Landegren et al., (1988) Science 241, 1077-1080; Van Brunt (1990) Biotechnology 8, 291-294; Wu and Wallace, (1989) Gene 4, 560; Barringer et al. (1990) Gene 89, 117, and Sooknanan and Malek (1995) Biotechnology 13: 563-564.

Mutation Identification, Allele discrimination, And Genotyping

The subject method of the present disclosure can be used for many applications, for example, mutation identification (i.e., determining the presence or absence of a mutation in a polynucleotide of interest), allele discrimination and genotyping.

For example, a diallelic organism contains two copies of each gene. Genotyping involves the determination of whether a diallelic organism contains two copies of the reference allele (a reference-type homozygote), one copy each of the reference and variant allele (i. e., a heterozygote), or contains two copies of the variant allele (i. e., a variant-type homozygote).

When conducting a genotyping analysis, the methods of the disclosure can be utilized to determine a single variant site (e.g., a SNP or a tandem repeat). However, the methods can also be used to determine allelic frequency in a group of individuals, as well as the genotype of an individual in many different DNA loci, either on the same gene, different genes or combinations thereof.

Most typically, SNPs consist of two allelic forms, i. e., the variant site includes one of two different nucleotides. The sample can contain nucleic acids representative of the two copies of the target nucleic acid of interest. Analyses can be conducted with a single labeled nucleotide, but more typically labeled nucleotides complementary to both nucleotides potentially at the site of variation are utilized. When one single labeled nucleotide is used, the amplification of the allele containing a complementary nucleotide at the variation site would produce a labeled amplified product, while the other allele not containing a complementary nucleotide at the variation site would not produce a labeled amplification product. Therefore, if the polynucleotide template is from a homozygote, it will give rise to a determinable level of label incorporation (i.e., if both allele containing a complementary nucleotide at the variation site), or no incorporation at all (i.e., if both allele not containing a complementary nucleotide at the variation site). An intermediate level of label incorporation would indicate that the nucleotide template is derived from a heterozygote. If two labeled nucleotides are used in the reaction mixture (i.e., each labeled nucleotide may be incorporated into a different allele), the formation of a single labeled amplified product (i.e., reflected by the incorporation frequency) indicates that the sample is from a homozygote. The particular label signifies whether the sample is from a reference-type or variant-type homozygote. The existence of two labeled amplified products indicates that the sample is from a heterozygote. Reactions can be conducted separately such that each labeled nucleotide is added to a different reaction mix, or reactions can be conducted in a single reaction mixture containing both labeled nucleotides. If reactions are conducted in a single reaction vessel, the labeled nucleotides are differentially labeled so that the different allelic forms can be distinguished. If different reactions are conducted with each labeled nucleotide, the labels for each labeled nucleotide can be the same or different since the particular nucleotide added to each reaction is tracked.

For polymorphisms that include more than two allelic forms, additional labeled nucleotides can be used. For example, for triallelic polymorphisms, three differentially labeled nucleotides can be used. In like manner, with tetra-allelic polymorphisms, four differentially labeled nucleotides can be employed. Here, too, all the nucleotides can be added to a single reaction mixture or to separate reaction mixtures.

Typically, any additional nucleotides are provided as mixtures of labeled and unlabeled forms.

The ability to use the methods of the disclosure to make rapid genotyping determinations provides a powerful tool in genetic analysis and ascertaining the susceptibility of an individual to a disease. Individuals that are mutant homozygotes for an allele associated with a particular disease are at higher risk of having the disease than a heterozygote or a homozygote for the other allele. The heterozygote, however, is a carrier of the allele associated with the disease. Such knowledge can be useful in prenatal and other types of medical and genetic counseling, for example.

Kits/Compositions

Compositions and kits for conducting the sequence and genotyping determinations described herein are also provided by the disclosure. The compositions include mixtures of labeled and unlabeled nucleotides such as those described above. The concentration of labeled to unlabeled forms for a nucleotide is as indicated above, but most typically the labeled form is 0.01% to 5% of the total concentration of the labeled and unlabeled forms as expressed on a molar basis. The labeled forms can include any of the labels described supra; most typically, however, the label is a fluorophore, especially FAM, ROX, TAMRA, R110, R6G, JOE, TET, HEX, Alexa dyes, Cy3 and Cy 5.

Generally, the reagents and devices described herein are packaged to include many if not all of the necessary components for performing the methods described herein. For example, the kits can include any of target polynucleotides, a labeled nucleotide, a polynucleotide synthesis enzyme, primers, buffer and other chemical agents, nucleotides, sample materials, control materials, devices, or the like. Such kits also typically include appropriate instructions for performing the methods of the present disclosure. Generally, reagents are provided in a stabilized form, so as to prevent degradation or other loss during prolonged storage, e.g., from leakage. A number of stabilizing processes are widely used for reagents that are to be stored, such as the inclusion of chemical stabilizers (i.e., enzymatic inhibitors, microcides/bacteriostats, anticoagulants), the physical stabilization of the material, e.g., through immobilization on a solid support, entrapment in a matrix (i.e., a gel), lyophilization, or the like.

EXAMPLES

The non-limiting examples shown below may help to better understand the present invention.

**Example 1** Color Ratios Correspond to Differences in Incorporation Ratios

Genomic DNA is prepared according to known methods in the art to serve as the polynucleotide template for sequence variation determination. Whole blood is drawn into EDTA-anticoagulated (purple top) tubes and then centrifuged in a clinical centrifuge at 3000 rpm for 20 minutes. The buffy coat is collected and genomic DNA (gDNA) is extracted using a commercially available kit (Stratagene DNA Extraction Kit cat# 200600, Qiagen QIAamp Blood Kit, etc.). The gDNA concentration is spectrophotometrically determined. In general, 50 to 100 ng of gDNA is added to each amplification reaction. In the example that follows, the chemokine receptor 2 (CCR2) allele is used as the SNP target.

No-template controls (NTCs) are prepared by using low-TE buffer (5 mM Tris-HCl, 0.1 mM EDTA, pH 8.0) to replace the template. Positive controls (e.g., homozygote allele 1 pos., homozygote allele 2 pos., or heterozygote) are prepared by any one of three common methods: (a) the respective CCR2 alleles are cloned and the purified, linearized plasmid serves as a control; (b) patient gDNA that has been sequence verified to belong to one of the three groups is used; or (c) synthetic oligonucletides with the corresponding SNP are used.

The template used was purified 79bp PCR Amplicon from the wildtype allele for the CCR2 Gene starting at position 236 and ending at position 313 in the reference sequence Accession # NM_000648.

The forward CCR2-79 and reverse CCR2-79 primers generate a PCR amplicon of 79bp with SNP site located at 15bp from the 3' end of the forward primer.

Fwd 79bp CCR2 primer 5' GTTCATCTTTGGTTTTGTGG 3' (SEQ ID NO: 19)
Rev 79bp CCR2 primer 5' GTCAGTCAAGCACTTCAG 3' (SEQ ID NO: 20)

5 femtograms of this 79bp PCR amplicon template was used in each 50ul PCR amplification reaction. Two sets of primers are used that bind to an interior region of the 79bp CCR2 amplicon that amplify a 48bp region starting at position 248 and ending at position 295 in the CCR2 reference sequence Accession # NM_000648. Each primer has a different 10bp tag region appended to the 5' end. The tag regions are non-complementary to the sequence upstream of the primer binding sites and do not play a role in binding to the CCR2 amplicon template. Once the tagged primers bind and extend the tag regions, the tag regions become part of the amplicon and are replicated in subsequent amplification rounds. The final PCR amplicon is a 68bp product including the incorporated tag regions.

One set of primers has 5 dGTP bases included in each tag region, which will add a total of 10 sites for dCTP incorporation during amplification. The other set of primers has no dGTP bases included in the tag region and will not add any additional sites for dCTP incorporation during amplification.

The primer designs are listed below with the Tag regions in Bold Italic:

**0 G Set (Amplifies Amplicon #1):**

Fwd 48bp CCR2 0G Mod Primer 5'*ACTACTTCAA*TTTTGTGGGCAACATGC 3' (SEQ ID NO: 21)

Rev 48bp CCR2 0G Mod Primer 5'*CACTTAACAC*TTTTTGCAGTTTATTAAGATGAGG 3'

(SEQ ID NO: 22)

**5 G Set (Amplifies Amplicon #2):**

Fwd 48bp CCR2 5G Mod Primer 5'*AGGAGAGGCC*TTTTGTGGGCAACATGC 3' (SEQ ID NO: 23)

Rev 48bp CCR2 5G Mod Primer 5'*CGTGAGGTCG*TTTTTGCAGTTTATTAAGATGAGG 3'

(SEQ ID NO: 24)

The Fwd and Rev 0G set and the Fwd and Rev 5G set were used to obtain amplicons with 15 and 25 dCTP incorporation sites respectively.

Reagents for the amplification reaction are assembled as follows (e.g., 50 μl reactions, 4 replicates each; final concentration are given):

0.5 U UNG

1 x Brilliant Core PCR Buffer (Stratagene Cat# 600530)
2 mM MgCl$_2$
SYBR Green I at a final concentration of 0.33X (1:30,000 dilution of "10,000X" stock from Molecular Probes, Inc.)
2.5 U SureStart Taq
10 uM each dATP, dGTP
20 uM dUTP
10 uM dCTP( 99% dCTP and 1% ROX-dCTP (0.1uM))
150 nM each forward and reverse primer.
0-3% DMSO
5 femtograms purified 79bp PCR product template

The PCR reaction is performed on the Stratagene Mx 4000 for the CCR2 template with the following parameters, but it should be understood that the PCR conditions may be optimized for each amplification reaction:

| 1 cycle | 50°C | 2 min |
|---|---|---|
| 1 cycle | 95°C | 10 min. |
| 40 cycle | 95°C | 30 Sec, |
| | 50°C | 1 min, |
| | 72°C | 30 Sec. |

Optionally, a dissociation profile is added to evaluate the make-up of the ROX-labeled and the SYBR Green I-labeled amplicon:

55°C to 95°C,
81 cycles with 0.5°C increase per step and 30 sec to 1 min per plateau.

In these reactions, dUTP was used in place of dTTP along with uracil-N Glycosylase (UNG) to help eliminate any carry over contamination. The initial 50°C hold in the thermal profile is required for the activation of UNG prior to amplification.

Real-time fluorescence data for PCR are collected at the annealing step using the "end 3" setting on the Stratagene Mx 4000. Data are acquired at each step of the dissociation curve using the above settings. The relevant filter sets used are (excitation/emission): SYBR/SYBR (referred to as SYBR) and SYBR/ROX (referred to as FRET). The ratio of signals collected at the annealing step, SYBR/FRET, for each amplification step is then calculated.

One 79bp CCR2 PCR amplicon was used as template along with the two primer sets described above to produce two amplicons of the same size (68bp) with varying numbers of dCTP incorporation sites. Each primer set was run in a reaction with 0% labeled Rox-dCTP and 1% Rox-dCTP.

The 0% reactions were used to determine the normal SYBR signal for the amplicon without FRET and the small fluorescence contribution of SYBR signal to the FRET channel. This SYBR leakage into the FRET channel can be subtracted from the FRET signals for the samples using Rox-dCTP to yield a more accurate ratio. The SYBR leakage adjustment was found to be negligible and was not used in the analysis of the data shown in Figure 1.

The SYBR and FRET signals for all replicates in the 1% Rox-dCTP reactions were used to calculate the average SYBR/FRET ratios for each amplicon. The difference in the SYBR/FRET ratios for each amplicon was expected to be equal to the fractional difference in the number of dCTP incorporation sites within each amplicon. Amplicon #1 has 15 dCTP sites, Amplicon #2 has 25 dCTP sites, for a difference of 10 dCTP sites. The fractional difference in dCTP sites for these amplicons is (25-15)/25 = 40%. The SYBR signal from a QPCR sample is proportional to the amount of amplicon in that sample. The FRET signal from incorporated Rox-dCTP excited by the SYBR fluorescence is proportional to both the SYBR fluorescence and the number of dCTP incorporation sites. The ratio of SYBR signal to the FRET signal is therefore inversely proportional to the number of incorporation sites.

The data in Figure 1 show the SYBR/FRET ratios for these two amplicons. After cycle 26 the fractional difference of incorporation sites in the two amplicons measured using the SYBR/FRET ratio agreed with the expected value. Figure 1A shows a graph of the values of the SYBR/FRET ratios for Amplicon #s 1 and 2. Amplicon #2 has 10 more dCTP incorporation sites than Amplicon #1, and therefore has a greater FRET signal, producing a lower SYBR/FRET ratio, than Amplicon #1, as can be seen in the graph. Figure 1B shows the data graphed in Figure 1A. Specifically, the average SYBR/FRET value from Amplicon #1 at cycle 30 was 3.0158, and the average SYBR/FRET value from Amplicon #2 at cycle 30 was 1.8067. The measured fractional difference in the number of incorporation sites is therefore

$$\frac{\frac{1}{1.8067} - \frac{1}{3.0158}}{\frac{1}{1.8067}} = 0.4009 = 40.09\%$$ which is remarkably close to the expected 40% fractional difference.

The data used to produce these results are from the dR (background subtracted) fluorescence data for each optical path collected for each sample. An adaptive baseline algorithm was used to treat all samples individually when plotting the fluorescence data.

Three replicate reactions were run for each sample and averaged. The dR fluorescence data for each optical path was collected (SYBR and FRET) at each cycle of the reaction and averaged for each replicate. (see columns C through E and L through N in Figure 1). The data obtained after Ct (threshold cycle) are relevant (about cycle 26).

**Example 2.** Determination of Color Ratios Using Rox-dNTP in a SNP Model

In this example, two 79bp PCR products are used as template for the amplification reaction, each representing a different allele for the CCR2 gene. Both PCR products were produced using the CCR2-79 Fwd and Rev primer set described in Example 1. The first PCR product consists of a region of interest corresponding to the wildtype genotype and the second PCR product consists of the same region of interest except it has a single sequence variation that corresponds to the variant genotype. One set of primers without tags is used to amplify both templates. These primers bind to the same interior region of the 79bp CCR2 amplicon described above in Example 1. This primer set amplifies both PCR product templates with equal efficiency.

The primer designs are listed below:

**FWD** 48bp CCR2 Primer 5' TTTTGTGGGCAACATGC 3' <u>(SEQ ID NO: 25)</u>
**REV** 48bp CCR2 Primer 5' TTTTTGCAGTTTATTAAGATGAGG 3' <u>(SEQ ID NO: 26)</u>

PCR is performed as described in Example 1 with 0% labeled Rox-dCTP and 1% Rox-dCTP reactions run for both alleles except the final SYBR Green concentration was 0.125X (1:80,000 dilution of "10,000X" stock solution).

Fluorescence data for PCR are collected at the annealing step as described above in Example 1 and the SYBR/FRET ratio is calculated for each replicate. The average SYBR/FRET ratios for each allele are then compared to determine if the difference is equal to the fractional difference in the number of dCTP incorporation sites within each amplicon 15: 14, wildtype to variant. The fractional difference in the number of dCTP incorporation sites between these amplicons is (15-14) / 15 = 6.667% from wildtype to variant.

The data in Figure 2 after cycle 30 show that the experimental fractional difference in dCTP incorporation sites measured using SYBR/FRET ratios for these two amplicons agreed with the expected value. Figure 2A shows a graph of the values of the SYBR/FRET ratios for the Wildtype amplicon (15 dCTP sites) and Variant amplicon (14 dCTP sites). The Variant amplicon has fewer dCTP incorporation sites than the Wildtype amplicon, thus having a lower FRET signal and a higher SYBR/FRET ratio, as can be seen in the graph. Figure 2B shows the data graphed in Figure 2A. Specifically, the average SYBR/FRET value from Amplicon #1 at cycle 30 was 3.5583, and the average SYBR/FRET value from Amplicon #2 at cycle 30 was 3.7947. Using the analysis explained in Example 1 the measured fractional difference in the number of incorporation sites is therefore $\dfrac{\frac{1}{3.5583} - \frac{1}{3.7947}}{\frac{1}{3.5583}} = 0.06230 = 6.230\%$, which is close to the expected 6.667% difference.

**Example 3** Effect of BHQ-10-dUTP on SYBR Green Fluorescence

In this example only one 79bp PCR product corresponding to the variant genotype for the CCR2 gene was used as template. The primers used in Example 2 were used in this example to amplify the same 48bp amplicon. PCR is performed as described in Example 1 except 125 fg of PCR product was used as template, SYBR Green was used at a final concentration of 0.10X (1:100,000 dilution of "10,000X" stock solution), BHQ-10-dUTP is used in place of Rox-dCTP and was titrated from 0-5% of the total concentration of dUTP (20uM final) used in the reaction.

Fluorescence data for PCR are collected at the annealing step as described in Example 1. The average SYBR Green signal for each set of replicates at each concentration of BHQ-10-dUTP is compared to determine the effect of BHQ-10-dUTP when incorporated into the amplicon. The data shown in Figure 3 indicate that as the concentration of BHQ-10-dUTP in the reaction increases the SYBR fluorescence decreases.

**Example 4.** The use of Modified Allele Specific Primers with a Rox-dNTP

In this example the two 79bp CCR2 PCR products that differ in their nucleotide sequence by a single base, as described in Example 2, were used as template. A set of allele specific primers was used that bind to an interior region of the 79bp CCR2 amplicons that amplify a 42bp region starting at position 254 and ending at position 295 in the CCR2 reference sequence Accession #NM_00648. Each forward primer contains a different base at the 3' end that is complementary to the nucleotide found in each of the two alleles for the SNP found at position 270 in the CCR2 reference sequence listed above. Each forward primer has a different 10bp tag region appended to the 5' end. The tag regions are non-complementary to the sequence upstream of the primer binding sites and do not play a role in binding to the CCR2 amplicon template. Once the tagged primers bind and extend, the tag regions become part of the amplicon and are replicated in subsequent amplification rounds. The final PCR amplicon is a 52bp product including the incorporated tag regions.

The forward wildtype specific primer has 5 dGTP bases included in the tag region, which will add a total of 5 sites for dCTP incorporation during amplification. The other forward primer specific to the variant allele has no dGTP bases included in the tag region and will not add any additional sites for dCTP incorporation during amplification. Both allele specific forward primers are used together with a non-modified, non-allele specific reverse primer. This three primer mix creates a competition reaction between the two forward primers, which yields high specificity for the correct forward primer/template combination.

■ The primer designs are listed below with the Tag regions and allele specific base in Bold Italic:

**Fwd** CCR2 Wildtype **5G** Mod Primer   5' ***GGATGGACAG***GGGCAACATGCTGGTC***G*** 3' <u>(SEQ ID NO: 27)</u>
**Fwd** CCR2 Variant **0G** Mod Primer   5' ***CCAACTCTAAG***GGCAACATGCTGGTC***A*** 3' <u>(SEQ ID NO: 28)</u>
**Rev** 48bp CCR2 Primer   5' TTTTTGCAGTTTATTAAGATGAGG 3' <u>(SEQ ID NO: 26)</u>

The Fwd 5G primer, and the Fwd 0G primer were used along with the Rev CCR2 primer to obtain allele specific amplicons with 18 and 12 dCTP incorporation sites respectively.

PCR is performed as described in Example 1 except 125 fg of template was used for each reaction, both forward allele specific primers are added to each reaction, SYBR Green was used at a final concentration of 0.10X (1: 100,000 dilution), the concentration of Rox-dCTP was 2% of the total dCTP (0.2uM final), and the annealing temperature was 62°C. A dissociation curve was appended to the end of the thermal profile to verify the specificity of the forward primers.

Fluorescence data for PCR are collected at the annealing step as described above in Example 1 and the SYBR/FRET ratio is calculated for each replicate. The average SYBR/FRET ratios for each allele are then compared to determine if the difference is significant enough to differentiate the two alleles. The average SYBR/FRET ratios were also used to verify the specificity of the forward primers.

The data in Figure 4 (graphed in Figure 4A, data table in Figure 4B) indicate that the competition reaction between the forward allele specific primers yields high specificity for the correct primer/template combination. The data also indicate the SYBR/FRET ratios are significantly different for each allele and that the coefficient of variance is small for each set of replicates. This suggests that the method has the accuracy and sensitivity necessary for genotyping SNPs with a single reaction.

**Example 5** Effect of Rox-dNTP and Quencher-dNTP on Color Ratio in a SNP Model

In this reaction the template and primers described in Example 2 were used to amplify the 48bp CCR2 amplicon from both alleles.

PCR is performed as described in Example 1 except 125 fg of template was used in each reaction, SYBR Green was used at a final concentration of 0.10X (1:100,000X dilution), 2% Rox-dUTP (0.4uM final) was used in place of Rox-dCTP, and BHQ-10-dUTP was added at 3% of the total concentration of dUTP (0.6uM final).

Rox labeled dUTP was used instead of Rox-dCTP, as the number of dUTP incorporation sites (13 to 12, variant to wildtype) is lower than the number of dCTP sites in both amplicons. This lower number of incorporation sites provides a slightly larger expected fractional difference between the two alleles. The expected fractional difference in the number of dUTP incorporation sites for these amplicons is (13-12) / 13 = 7.692%

Fluorescence data for PCR are collected at the annealing step as described above in Example 1 and the SYBR/FRET ratio is calculated for each replicate. The average SYBR/FRET ratios for each allele are then compared to determine if the addition of BHQ-10-dUTP has an effect on the difference between the two alleles.

The data at cycle 25 in Figure 5 show that the fractional difference in the number of dUTP incorporation sites for the two alleles measured using the SYBR/FRET ratios agreed closely with the expected value when 2% Rox-dUTP and 0% BHQ-10-dUTP were added to the reaction (see data table in Figure 5C). The fractional difference calculation using SYBR/FRET ratios was performed as explained in Example 1. The data also show that the difference in SYBR/FRET ratios for the two alleles increases when 3% BHQ-10-dUTP is added (see Figure 5A and 5B). The data obtained after Ct (threshold cycle) are relevant (about cycle 25).

OTHER EMBODIMENTS

The foregoing examples demonstrate experiments performed and contemplated by the present inventors in making and carrying out the invention. It is believed that these examples include a disclosure of techniques which serve to both apprise the art of the practice of the invention and to demonstrate its usefulness.

**Claims**

1. A method for determining a sequence difference between a region of interest in a first polynucleotide and a corresponding region of interest in a second polynucleotide, said method comprising:

   a) incubating said first polynucleotide in a first reaction mixture comprising a primer and a nucleotide labeled with a detectable label to produce a first extended polynucleotide product comprising said primer from said first polynucleotide;
   b) determining an incorporation frequency of said labeled nucleotide for said first extended polynucleotide product, wherein said incorporation frequency is determined by

      i) measuring the detectable label incorporated into said extended polynucleotide product comprising said primer,
      ii) measuring the total amount of extended polynucleotide product comprising said primer, and
      iii) calculating a ratio between (i) and (ii), wherein said ratio provides said incorporation frequency; and

   c) comparing said incorporation frequency determined in step (b) with an incorporation frequency for said second polynucleotide,

   wherein a difference in the two incorporation frequencies is indicative of a sequence difference between the region of interest of said first polynucleotide and the corresponding region of interest of said second polynucleotide.

2. The method according to claim 1, wherein said nucleotide labeled with said detectable label for said first polynucleotide is also used for said second polynucleotide.

3. The method according to claim 1 or 2, wherein said first reaction mixture further comprises nucleotides dATP, dGTP, dTTP, and dCTP, at least one of which is labeled with said detectable label.

4. The method according to any of claims 1 to 3, wherein said first polynucleotide product is linked to a solid support.

5. The method according to any of claims 1 to 4, wherein said amount of the first and/or second polynucleotide product is measured by polynucleotide staining with a polynucleotide stain.

6. The method according to any of claims 1 to 5, wherein said first reaction mixture further comprises a set of oligonucleotide primers which flank the region of interest.

7. The method according to any of claims 1 to 6, wherein said first polynucleotide product is an amplified product.

8. The method according to any of claims 1 to 7, wherein said sequence difference is at a predetermined nucleotide position within the region of interest in said polynucleotide, or comprises a single nucleotide polymorphism or a tandem repeat.

9. A method for determining a sequence difference between a region of interest in a polynucleotide and a reference sequence, said method comprising:

a) incubating said polynucleotide in a reaction mixture comprising a primer and a nucleotide labeled with a detectable label to produce an extended polynucleotide product comprising said primer from said polynucleotide;

b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency; and

c) comparing said incorporation frequency determined in step (b) with a known frequency for said reference sequence,

wherein a difference in the two frequencies is indicative of a sequence difference between the region of interest of said polynucleotide and said reference sequence.

10. The method according to claim 9, wherein said sequence difference is at a predetermined nucleotide position within the region of interest in said polynucleotide, or comprises a single polymorphism or a tandem repeat.

11. A method for determining the presence of a mutation in a region of interest in a polynucleotide, said method comprising:

a) incubating said polynucleotide in a reaction mixture comprising a primer and a nucleotide labeled with a detectable label, to produce an extended polynucleotide product comprising said primer from said polynucleotide;
b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency; and

c) comparing said incorporation frequency determined in step (b) with a known frequency for a reference wild-type sequence,

wherein a difference in the two frequencies is indicative of the presence of a mutation in a region of interest in a polynucleotide.

12. A method for genotyping comprising:

a) incubating in a reaction mixture a first nucleic acid sample comprising a region of interest, said reaction mixture comprising a primer and a nucleotide labeled with a detectable label to produce an extended polynucleotide product comprising said primer from said nucleic acid sample; and
b) determining an incorporation frequency of said labeled nucleotide for said polynucleotide product, wherein said incorporation frequency is determined by

i) measuring the detectable label incorporated into said extended polynucleotide product comprising said primer,
ii) measuring the total amount of extended polynucleotide product comprising said primer, and
iii) calculating a ratio between (i) and (ii), wherein said ratio provides said incorporation frequency,

and wherein said incorporation frequency is indicative of the genotype of the organism from which said nucleic acid sample was obtained.

13. The method according to claim 12, wherein said incorporation frequency is indicative of whether said organism has a wild-type or a variant genotype.

14. The method according to any of claims 9 to 13, wherein said polynucleotide product is linked to a solid support.

**15.** The method according to claim 14, wherein said amount of the polynucleotide product is measured by polynucleotide staining with a polynucleotide stain.

**16.** The method according to any of claims 9 to 15, wherein said reaction mixture further comprises a set of oligonucleotide primers which flank the region of interest.

**17.** The method according to any of claims 9 to 16, wherein said reaction mixture further comprises nucleotides dATP, dGTP, dTTP, and dCTP, at least one of which is labeled with said detectable label.

**18.** The method according to any of claims 9 to 17, wherein said polynucleotide product is an amplified product.

**19.** The method according to any of claims 4 to 8 and 14 to 18, wherein the number of potential linkage sites on said solid support is substantially constant and said linked polynucleotide product saturates the potential linkage sites on said solid support.

**20.** The method according to any of claims 1 to 19, wherein said detectable label is one selected from the group consisting of: a fluorescent label, a fluorescence quencher, a colorimetric label, a chemiluminescent label, an isotope, a quantum dot label, an antigen, an affinity moiety, and a chemical label.

**21.** The method according to any of claims 1 to 20, wherein said labeled nucleotide comprises a signal generating moiety and a signal quenching moiety, wherein said signal quenching moiety quenches the signal from said signal generating moiety when both said moieties are present on said labeled nucleotide.

**22.** The method according to claim 21, wherein said signal quenching moiety is separated from said labeled nucleotide upon incorporation of said labeled nucleotide into said polynucleotide product.

**23.** The method according to claim 5 or 15, wherein SYBR is used for said polynucleotide staining , and ROX is used to label said labeled nucleotide.

**24.** The method according to claim 23, wherein a detectable signal is generated by ROX on said labeled nucleotide incorporated into the polynucleotide product.

**25.** The method according to any of claims 1 to 24, wherein said reaction mixture comprises a mixture of said nucleotide labeled with a detectable label and the same nucleotide not labeled with a detectable label.

**26.** The method according to claim 25, wherein the amount of said labeled nucleotide is 0.01 % to 25%, preferably 0.5% to 25% of the total amount of said nucleotide, including labeled and unlabeled said nucleotide.

**27.** The method according to any of claims 1 to 26, wherein said reaction mixture comprises two different nucleotides labeled with different detectable labels.

**28.** The method according to any of claims 1 to 27, wherein said reaction is an amplification reaction.

**Patentansprüche**

**1.** Verfahren zur Bestimmung einer Sequenzdifferenz zwischen einer Region von Interesse in einem ersten Polynukleotid und einer entsprechenden Region von Interesse in einem zweiten Polynukleotid, wobei das Verfahren Folgendes umfasst:

a) Inkubieren des ersten Polynukleotids in einem ersten Reaktionsgemisch, das einen Primer und ein Nukleotid, das mit einem detektierbaren Marker markiert ist, umfasst, um ein erstes verlängertes Polynukleotidprodukt, das den Primer umfasst, von dem ersten Polynukleotid zu erzeugen;
b) Bestimmen einer Labeling-Effizienz des markierten Nukleotids für das erste verlängerte Polynukleotidprodukt, wobei die Labeling-Effizienz bestimmt wird durch:

i) Messen des detektierbaren Markers, der in das verlängerten Polynukleotidprodukt eingearbeitet ist, das den Primer umfasst;

ii) Messen der Gesamtmenge des verlängerten Polynukleotidprodukts, das den Primer umfasst, und
iii) Berechnen eines Verhältnisses zwischen (i) und (ii), wobei das Verhältnis die Labeling-Effizienz liefert; und

c) Vergleichen der Labeling-Effizienz, die in Schritt (b) bestimmt wurde, mit einer Labeling-Effizienz für das zweite Polynukleotid,

wobei eine Differenz in den zwei Labeling-Effizienzen eine Sequenzdifferenz zwischen der Region von Interesse des ersten Polynukleotids und der entsprechenden Region von Interesse des zweiten Polynukleotids angibt.

2. Verfahren nach Anspruch 1, wobei das Nukleotid, das mit dem detektierbaren Marker für das erste Polynukleotid markiert ist, auch für das zweite Polynukleotid verwendet wird.

3. Verfahren nach Anspruch 1 oder 2, wobei das erste Reaktionsgemisch des Weiteren Nukleotide dATP, dGTP, dTTP und dCTP umfasst, von welchen mindestens eines mit dem detektierbaren Marker markiert ist.

4. Verfahren nach einem der Ansprüche 1 bis 3, wobei das erste Polynukleotidprodukt mit einem festen Träger verbunden ist.

5. Verfahren nach einem der Ansprüche 1 bis 4, wobei die Menge des ersten und/oder zweiten Polynukleotidprodukts durch Polynukleotidfärbung mit einem Polynukleotidfarbstoff gemessen wird.

6. Verfahren nach einem der Ansprüche 1 bis 5, wobei das erste Reaktionsgemisch des Weiteren einen Satz von Oligonukleotid-Primern umfasst, die die Region von Interesse flankieren.

7. Verfahren nach einem der Ansprüche 1 bis 6, wobei das erste Polynukleotidprodukt ein amplifiziertes Produkt ist.

8. Verfahren nach einem der Ansprüche 1 bis 7, wobei die Sequenzdifferenz an einer vorbestimmten Nukleotidposition innerhalb der Region von Interesse in dem Polynukleotid vorhanden ist oder einen einzelnen Nukleotidpolymorphismus oder ein Tandem-Repeat umfasst.

9. Verfahren zur Bestimmung einer Sequenzdifferenz zwischen einer Region von Interesse in einem Polynukleotid und einer Referenzsequenz, wobei das Verfahren Folgendes umfasst:

a) Inkubieren des Polynukleotids in einem Reaktionsgemisch, das einen Primer und ein Nukleotid, das mit einem detektierbaren Marker markiert ist, umfasst, um ein verlängertes Polynukleotidprodukt, das den Primer umfasst, von dem Polynukleotid zu erzeugen;
b) Bestimmen einer Labeling-Effizienz des markierten Nukleotids für das Polynukleotidprodukt, wobei die Labeling-Effizienz bestimmt wird durch:

i) Messen des detektierbaren Markers, der in das verlängerte Polynukleotidprodukt eingearbeitet ist, das den Primer umfasst;
ii) Messen der Gesamtmenge des verlängerten Polynukleotidprodukts, das den Primer umfasst, und
iii) Berechnen eines Verhältnisses zwischen (i) und (ii), wobei das Verhältnis die Labeling-Effizienz liefert; und

c) Vergleichen der Labeling-Effizienz, die in Schritt (b) bestimmt wurde, mit einer bekannten Effizienz für die Referenzsequenz,

wobei eine Differenz in den zwei Effizienzen eine Sequenzdifferenz zwischen der Region von Interesse des Polynukleotids und der Referenzsequenz angibt.

10. Verfahren nach Anspruch 9, wobei die Sequenzdifferenz an einer vorbestimmten Nukleotidposition innerhalb der Region von Interesse in dem Polynukleotid vorhanden ist oder einen einzelnen Nukleotidpolymorphismus oder ein Tandem-Repeat umfasst.

11. Verfahren zur Bestimmung des Vorhandenseins einer Mutation in einer Region von Interesse in einem Polynukleotid, wobei das Verfahren Folgendes umfasst:

a) Inkubieren des Polynukleotids in einem Reaktionsgemisch, das einen Primer und ein Nukleotid, das mit einem detektierbaren Marker markiert ist, umfasst, um ein verlängertes Polynukleotidprodukt, das den Primer umfasst, von dem Polynukleotid zu erzeugen;

b) Bestimmen einer Labeling-Effizienz des markierten Nukleotids für das Polynukleotidprodukt, wobei die Labeling-Effizienz bestimmt wird durch:

i) Messen des detektierbaren Markers, der in das verlängerte Polynukleotidprodukt eingearbeitet ist, das den Primer umfasst;

ii) Messen der Gesamtmenge des verlängerten Polynukleotidprodukts, das den Primer umfasst, und

iii) Berechnen eines Verhältnisses zwischen (i) und (ii), wobei das Verhältnis die Labeling-Effizienz liefert; und

c) Vergleichen der Labeling-Effizienz, die in Schritt (b) bestimmt wurde, mit einer bekannten Effizienz für eine Wildtyp-Referenzsequenz,

wobei eine Differenz in den zwei Effizienzen das Vorhandensein einer Mutation in einer Region von Interesse in einem Polynukleotid angibt.

12. Verfahren zur Genotypisierung, umfassend:

a) Inkubieren einer ersten Nukleinsäureprobe, die eine Region von Interesse umfasst, in einem Reaktionsgemisch, wobei das Reaktionsgemisch einen Primer und ein Nukleotid umfasst, das mit einem detektierbaren Marker markiert ist, um ein verlängertes Polynukleotidprodukt, das den Primer umfasst, von der Nukleinsäureprobe zu erzeugen;

b) Bestimmen einer Labeling-Effizienz des markierten Nukleotids für das Polynukleotidprodukt, wobei die Labeling-Effizienz bestimmt wird durch:

i) Messen des detektierbaren Markers, der in das verlängerte Polynukleotidprodukt eingearbeitet ist, das den Primer umfasst;

ii) Messen der Gesamtmenge des verlängerten Polynukleotidprodukts, das den Primer umfasst, und

iii) Berechnen eines Verhältnisses zwischen (i) und (ii), wobei das Verhältnis die Labeling-Effizienz liefert; und

und wobei die Labeling-Effizienz den Genotyp des Organismus angibt, von dem die Nukleinsäureprobe erhalten wurde.

13. Verfahren nach Anspruch 12, wobei die Labeling-Effizienz angibt, ob der Organismus einen Wildtyp- oder einen Varianten-Genotyp aufweist.

14. Verfahren nach einem der Ansprüche 9 bis 13, wobei das Polynukleotidprodukt mit einem festen Träger verbunden ist.

15. Verfahren nach Anspruch 14, wobei die Menge des Polynukleotidprodukts durch Polynukleotidfärbung mit einem Polynukleotidfarbstoff gemessen wird.

16. Verfahren nach einem der Ansprüche 9 bis 15, wobei das Reaktionsgemisch des Weiteren einen Satz von Oligonukleotid-Primern umfasst, die die Region von Interesse flankieren.

17. Verfahren nach einem der Ansprüche 9 bis 16, wobei das Reaktionsgemisch des Weiteren Nukleotide dATP, dGTP, dTTP und dCTP umfasst, von welchen mindestens eines mit dem detektierbaren Marker markiert ist.

18. Verfahren nach einem der Ansprüche 9 bis 17, wobei das Polynukleotidprodukt ein amplifiziertes Produkt ist.

19. Verfahren nach einem der Ansprüche 4 bis 8 und 14 bis 18, wobei die Anzahl möglicher Bindungsstellen auf dem festen Träger im Wesentlichen konstant ist und das gebundene Polynukleotidprodukt die möglichen Bindungsstellen auf dem festen Träger sättigt.

20. Verfahren nach einem der Ansprüche 1 bis 19, wobei der detektierbare Marker ausgewählt ist aus der Gruppe bestehend aus: einem fluoreszierenden Marker, einem Fluoreszenzquencher, einem kolorimetrischen Marker, einem chemilumineszenten Marker, einem Isotop, einem Quantenpunktmarker, einem Antigen, einer Affinitätsgruppe und einem chemischen Marker.

**21.** Verfahren nach einem der Ansprüche 1 bis 20, wobei das markierte Nukleotid eine Signalerzeugungsgruppe und eine Signal-Quenching-Gruppe umfasst, wobei die Signal-Quenching-Gruppe das Signal von der Signalerzeugungsgruppe unterdrückt, wenn beide Gruppen auf dem markierten Nukleotid vorhanden sind.

**22.** Verfahren nach Anspruch 21, wobei die Signal-Quenching-Gruppe von dem markierten Nukleotid beim Einarbeiten des markierten Nukleotids in das Polynukleotidprodukt getrennt ist.

**23.** Verfahren nach Anspruch 5 oder 15, wobei SYBR für die Polynukleotidfärbung verwendet wird, und ROX für die Markierung des markierten Nukleotids verwendet wird.

**24.** Verfahren nach Anspruch 23, wobei ein detektierbares Signal von ROX auf dem markierten Nukleotid erzeugt wird, das in das Polynukleotidprodukt eingearbeitet ist.

**25.** Verfahren nach einem der Ansprüche 1 bis 24, wobei das Reaktionsgemisch ein Gemisch aus dem Nukleotid, das mit einem detektierbaren Marker markiert ist, und demselben Nukleotid, das nicht mit einem detektierbaren Marker markiert ist, umfasst.

**26.** Verfahren nach Anspruch 25, wobei die Menge des markierten Nukleotids 0,01% bis 25%, vorzugsweise 0,5% bis 25% der Gesamtmenge des Nukleotids beträgt, einschließlich des markierten und nicht markierten Nukleotids.

**27.** Verfahren nach einem der Ansprüche 1 bis 26, wobei das Reaktionsgemisch zwei verschiedenen Nukleotide umfasst, die mit verschiedenen detektierbaren Markern markiert sind.

**28.** Verfahren nach einem der Ansprüche 1 bis 27, wobei die Reaktion eine Amplifikationsreaktion ist.

**Revendications**

**1.** Procédé de détermination d'une différence de séquence entre une région d'intérêt dans un premier polynucléotide et une région d'intérêt correspondante dans un second polynucléotide, ledit procédé comprenant :

a) l'incubation dudit premier polynucléotide dans un premier mélange réactionnel comprenant une amorce et un nucléotide marqué avec un marqueur détectable pour produire un premier produit polynucléotidique allongé comprenant ladite amorce dudit premier polynucléotide ;
b) la détermination d'une fréquence d'incorporation dudit nucléotide marqué pour ledit premier produit polynucléotidique allongé, ladite fréquence d'incorporation étant déterminée par

i) la mesure du marqueur détectable incorporé dans ledit produit polynucléotidique allongé comprenant ladite amorce,
ii) la mesure de la quantité totale de produit polynucléotidique allongé comprenant ladite amorce, et
iii) le calcul d'un rapport entre (i) et (ii), ledit rapport fournissant ladite fréquence d'incorporation ; et

c) la comparaison de ladite fréquence d'incorporation déterminée dans l'étape (b) à une fréquence d'incorporation pour ledit second polynucléotide,

une différence entre les deux fréquences d'incorporation indiquant une différence de séquence entre la région d'intérêt dudit premier polynucléotide et la région d'intérêt correspondante dudit second polynucléotide.

**2.** Procédé selon la revendication 1, dans lequel ledit nucléotide marqué avec ledit marqueur détectable pour ledit premier polynucléotide est également utilisé pour ledit second polynucléotide.

**3.** Procédé selon la revendication 1 ou 2, dans lequel ledit premier mélange réactionnel comprend en outre les nucléotides dATP, dGTP, dTTP, et dCTP, au moins l'un d'entre eux étant marqué avec ledit marqueur détectable.

**4.** Procédé selon l'une quelconque des revendications 1 à 3, dans lequel ledit premier produit polynucléotidique est lié à un support solide.

**5.** Procédé selon l'une quelconque des revendications 1 à 4, dans lequel ladite quantité du premier et/ou du second produit polynucléotidique est mesurée par coloration de polynucléotides avec un colorant pour polynucléotides.

**6.** Procédé selon l'une quelconque des revendications 1 à 5, dans lequel ledit premier mélange réactionnel comprend en outre un jeu d'amorces oligonucléotidiques qui flanquent la région d'intérêt.

**7.** Procédé selon l'une quelconque des revendications 1 à 6, dans lequel ledit premier produit polynucléotidique est un produit amplifié.

**8.** Procédé selon l'une quelconque des revendications 1 à 7, dans lequel ladite différence de séquence est à une position de nucléotide prédéterminée à l'intérieur de la région d'intérêt dans ledit polynucléotide, ou comprend un polymorphisme mononucléotidique ou une répétition en tandem.

**9.** Procédé de détermination d'une différence de séquence entre une région d'intérêt dans un polynucléotide et une séquence de référence, ledit procédé comprenant :

a) l'incubation dudit polynucléotide dans un mélange réactionnel comprenant une amorce et un nucléotide marqué avec un marqueur détectable pour produire un produit polynucléotidique allongé comprenant ladite amorce dudit polynucléotide ;
b) la détermination d'une fréquence d'incorporation dudit nucléotide marqué pour ledit produit polynucléotidique, ladite fréquence d'incorporation étant déterminée par :

i) la mesure du marqueur détectable incorporé dans ledit produit polynucléotidique allongé comprenant ladite amorce,
ii) la mesure de la quantité totale de produit polynucléotidique allongé comprenant ladite amorce, et
iii) le calcul d'un rapport entre (i) et (ii), ledit rapport fournissant ladite fréquence d'incorporation ; et

c) la comparaison de ladite fréquence d'incorporation déterminée dans l'étape (b) à une fréquence connue pour ladite séquence de référence,

une différence entre les deux fréquences indiquant une différence de séquence entre la région d'intérêt dudit polynucléotide et ladite séquence de référence.

**10.** Procédé selon la revendication 9, dans lequel ladite différence de séquence est à une position de nucléotide prédéterminée à l'intérieur de la région d'intérêt dans ledit polynucléotide, ou comprend un seul polymorphisme ou une répétition en tandem.

**11.** Procédé de détermination de la présence d'une mutation dans une région d'intérêt dans un polynucléotide, ledit procédé comprenant :

a) l'incubation dudit polynucléotide dans un mélange réactionnel comprenant une amorce et un nucléotide marqué avec un marqueur détectable, pour produire un produit polynucléotidique allongé comprenant ladite amorce dudit polynucléotide ;
b) la détermination d'une fréquence d'incorporation dudit nucléotide marqué pour ledit produit polynucléotidique, ladite fréquence d'incorporation étant déterminée par

i) la mesure du marqueur détectable incorporé dans ledit produit polynucléotidique allongé comprenant ladite amorce,
ii) la mesure de la quantité totale de produit polynucléotidique allongé comprenant ladite amorce, et
iii) le calcul d'un rapport entre (i) et (ii), ledit rapport fournissant ladite fréquence d'incorporation ; et

c) la comparaison de ladite fréquence d'incorporation déterminée dans l'étape (b) à une fréquence connue pour une séquence de référence de type sauvage,

une différence entre les deux fréquences indiquant la présence d'une mutation dans une région d'intérêt dans un polynucléotide.

12. Procédé de génotypage comprenant :

a) l'incubation dans un mélange réactionnel d'un premier échantillon d'acide nucléique comprenant une région d'intérêt, ledit mélange réactionnel comprenant une amorce et un nucléotide marqué avec un marqueur détectable pour produire un produit polynucléotidique allongé comprenant ladite amorce dudit échantillon d'acide nucléique ; et
b) la détermination d'une fréquence d'incorporation dudit nucléotide marqué pour ledit produit polynucléotidique, ladite fréquence d'incorporation étant déterminée par

i) la mesure du marqueur détectable incorporé dans ledit produit polynucléotidique allongé comprenant ladite amorce,
ii) la mesure de la quantité totale de produit polynucléotidique allongé comprenant ladite amorce, et
iii) le calcul d'un rapport entre (i) et (ii), ledit rapport fournissant ladite fréquence d'incorporation,

et ladite fréquence d'incorporation indiquant le génotype de l'organisme duquel ledit échantillon d'acide nucléique a été obtenu.

13. Procédé selon la revendication 12, dans lequel ladite fréquence d'incorporation indique si ledit organisme a un génotype de type sauvage ou variant.

14. Procédé selon l'une quelconque des revendications 9 à 13, dans lequel ledit produit polynucléotidique est lié à un support solide.

15. Procédé selon la revendication 14, dans lequel ladite quantité du produit polynucléotidique est mesurée par coloration de polynucléotides avec un colorant pour polynucléotides.

16. Procédé selon l'une quelconque des revendications 9 à 15, dans lequel ledit mélange réactionnel comprend en outre un jeu d'amorces oligonucléotidiques qui flanquent la région d'intérêt.

17. Procédé selon l'une quelconque des revendications 9 à 16, dans lequel ledit mélange réactionnel comprend en outre les nucléotides dATP, dGTP, dTTP, et dCTP, au moins l'un d'entre eux étant marqué avec ledit marqueur détectable.

18. Procédé selon l'une quelconque des revendications 9 à 17, dans lequel ledit produit polynucléotidique est un produit amplifié.

19. Procédé selon l'une quelconque des revendications 4 à 8 et 14 à 18, dans lequel le nombre de sites de liaison potentiels sur ledit support solide est substantiellement constant et ledit produit polynucléotidique lié sature les sites de liaison potentiels sur ledit support solide.

20. Procédé selon l'une quelconque des revendications 1 à 19, dans lequel ledit marqueur détectable est choisi dans le groupe constitué par : un marqueur fluorescent, un extincteur de fluorescence, un marqueur colorimétrique, un marqueur chimiluminescent, un isotope, un marqueur point quantique, un antigène, une fraction d'affinité, et un marqueur chimique.

21. Procédé selon l'une quelconque des revendications 1 à 20, dans lequel ledit nucléotide marqué comprend une fraction de génération de signal et une fraction d'extinction de signal, ladite fraction d'extinction de signal éteignant le signal de ladite fraction de génération de signal lorsque lesdites deux fractions sont présentes sur ledit nucléotide marqué.

22. Procédé selon la revendication 21, dans lequel ladite fraction d'extinction de signal est séparée dudit nucléotide marqué lors de l'incorporation dudit nucléotide marqué dans ledit produit polynucléotidique.

23. Procédé selon la revendication 5 ou 15, dans lequel le SYBR est utilisé pour ladite coloration de polynucléotides, et le ROX est utilisé pour marquer ledit nucléotide marqué.

**24.** Procédé selon la revendication 23, dans lequel un signal détectable est généré par ROX sur ledit nucléotide marqué incorporé dans le produit polynucléotidique.

**25.** Procédé selon l'une quelconque des revendications 1 à 24, dans lequel ledit mélange réactionnel comprend un mélange dudit nucléotide marqué avec un marqueur détectable et du même nucléotide non marqué avec un marqueur détectable.

**26.** Procédé selon la revendication 25, dans lequel la quantité dudit nucléotide marqué est de 0,01 % à 25 %, de préférence de 0,5 % à 25 % de la quantité totale dudit nucléotide, comprenant ledit nucléotide marqué et non marqué.

**27.** Procédé selon l'une quelconque des revendications 1 à 26, dans lequel ledit mélange réactionnel comprend deux nucléotides marqués différents avec différents marqueurs détectables.

**28.** Procédé selon l'une quelconque des revendications 1 à 27, dans lequel ladite réaction est une réaction d'amplification.

FIGURE 1A

**Amplicon #1**
**Average Sybr/Average Fret Values for Each Replicate**

| Cycle | Rep 1 | Rep 2 | Rep 3 | AVERAGE | STDEV | CV |
|---|---|---|---|---|---|---|
| 1 | 5.907768 | 3.16512 | -35.25125 | -8.726122298 | 23.01233 | -263.72% |
| 2 | 6.414971 | -0.541527 | 1.552669 | 2.475371057 | 3.568859 | 144.17% |
| 3 | -0.027092 | 3.376997 | 2.789458 | 2.046454241 | 1.819614 | 88.92% |
| 4 | 5.987016 | 0.096768 | 3.471689 | 3.185157824 | 2.955559 | 92.79% |
| 5 | -32.09976 | 41.38178 | -8.445496 | 0.278841979 | 37.50959 | 13451.92% |
| 6 | 7.692093 | 15.67809 | 9.867979 | 11.07938736 | 4.128519 | 37.26% |
| 7 | -0.840165 | -0.987716 | -0.492785 | -0.773555469 | 0.2541 | -32.85% |
| 8 | -1.355295 | 1.075552 | 1.00831 | 0.242855833 | 1.384447 | 570.07% |
| 9 | -5.02327 | -1.674362 | -0.517733 | -2.405121728 | 2.339973 | -97.29% |
| 10 | -7.378379 | 13.83619 | 6.846169 | 4.434659992 | 10.81092 | 243.78% |
| 11 | 3.158956 | -5.560976 | 13.50194 | 3.699972418 | 9.542966 | 257.92% |
| 12 | -2.717492 | 1.226412 | 0.516726 | -0.324784641 | 2.102308 | -647.29% |
| 13 | -1.854698 | 1.920325 | 7.001473 | 2.355700092 | 4.444109 | 188.65% |
| 14 | -1.066265 | 8.095033 | 8.813187 | 5.280651673 | 5.508308 | 104.31% |
| 15 | 8.938763 | -1.490911 | -13.36954 | -1.973895347 | 11.16199 | -565.48% |
| 16 | -9.892304 | 3.546254 | -1.025165 | -2.457071835 | 6.83275 | -278.09% |
| 17 | 1.488823 | 5.393558 | 1.162577 | 2.681652551 | 2.354237 | 87.79% |
| 18 | -1.608382 | 0.711024 | 18.2456 | 5.782746649 | 10.85527 | 187.72% |
| 19 | -4.409081 | -7.000854 | 3.463456 | -2.648826564 | 5.449708 | -205.74% |
| 20 | -4.142625 | -7.08162 | 3.788005 | -2.478746644 | 5.622593 | -226.83% |
| 21 | 1.322835 | -11.94788 | 7.548627 | -1.025472373 | 9.95813 | -971.08% |
| 22 | 3.151938 | 7.402373 | 2.509072 | 4.354460735 | 2.659068 | 61.07% |
| 23 | 3.799452 | 4.017992 | 2.487014 | 3.434818961 | 0.828065 | 24.11% |
| 24 | 2.876862 | 2.732882 | 2.600454 | 2.736732643 | 0.138245 | 5.05% |
| 25 | 2.991252 | 3.095339 | 2.632639 | 2.906409949 | 0.242738 | 8.35% |
| 26 | 2.458727 | 2.657486 | 2.647947 | 2.588053203 | 0.112102 | 4.33% |
| 27 | 2.582879 | 2.907748 | 2.657972 | 2.716199549 | 0.170082 | 6.26% |
| 28 | 2.701211 | 3.032881 | 2.805907 | 2.846666544 | 0.16955 | 5.96% |
| 29 | 2.784131 | 2.94231 | 2.860271 | 2.862237323 | 0.079108 | 2.76% |
| 30 | 2.975815 | 3.118129 | 2.953497 | 3.015813671 | 0.089308 | 2.96% |
| 31 | 2.95876 | 3.218637 | 3.043047 | 3.073481515 | 0.132585 | 4.31% |
| 32 | 3.133262 | 3.316653 | 3.16935 | 3.206421655 | 0.097154 | 3.03% |
| 33 | 3.269876 | 3.412046 | 3.206954 | 3.296292256 | 0.105067 | 3.19% |
| 34 | 3.317869 | 3.355801 | 3.27773 | 3.317133503 | 0.039041 | 1.18% |
| 35 | 3.335932 | 3.466812 | 3.280148 | 3.36096383 | 0.095817 | 2.85% |
| 36 | 3.413004 | 3.535198 | 3.317431 | 3.421877879 | 0.109155 | 3.19% |
| 37 | 3.420388 | 3.638462 | 3.335305 | 3.464718417 | 0.156365 | 4.51% |
| 38 | 3.44951 | 3.703882 | 3.365825 | 3.506405366 | 0.176064 | 5.02% |
| 39 | 3.530846 | 3.681459 | 3.343151 | 3.51848536 | 0.169492 | 4.82% |
| 40 | 3.680848 | 3.834158 | 3.347505 | 3.620836831 | 0.248815 | 6.87% |

FIGURE 1B-1

**Amplicon #2**
**Average Sybr/Average Fret Values for Each Replicate**

| Cycle | Rep 1 | Rep 2 | Rep 3 | AVERAGE | STDEV | CV |
|---|---|---|---|---|---|---|
| 1 | 7.240895 | -5.123211 | 12.54861 | 4.888766186 | 9.067675 | 185.48% |
| 2 | -2.814144 | 1.482559 | 0.390696 | -0.31362962 | 2.233265 | -712.07% |
| 3 | 1.191457 | 3.128628 | -7.7884 | -1.156105113 | 5.824831 | -503.63% |
| 4 | 4.215119 | -0.001731 | 2.432925 | 2.215437863 | 2.116621 | 95.55% |
| 5 | -5.410478 | 7.912769 | 3.903078 | 2.135122921 | 6.835311 | 320.14% |
| 6 | 1.93062 | -0.482585 | 3.316544 | 1.588192957 | 1.922573 | 121.05% |
| 7 | 0.223919 | -1.461145 | 194.6484 | 64.47039429 | 112.7406 | 174.87% |
| 8 | 0.075058 | 1.259591 | 13.70379 | 5.012812473 | 7.549873 | 150.61% |
| 9 | 3.708652 | 1.958395 | 7.823346 | 4.496797752 | 3.010862 | 66.96% |
| 10 | -5.577799 | -8.769746 | 1.240077 | -4.369155825 | 5.113194 | -117.03% |
| 11 | 1.002942 | -0.592329 | 23.53811 | 7.982908209 | 13.49479 | 169.05% |
| 12 | -2.230881 | 2.902226 | 6.393173 | 2.35483933 | 4.338007 | 184.22% |
| 13 | 3.749643 | -0.35205 | 0.760684 | 1.386092214 | 2.121161 | 153.03% |
| 14 | 11.22515 | 155.3778 | 1.008108 | 55.87035887 | 86.32727 | 154.51% |
| 15 | 0.452404 | 6.454613 | 413.9962 | 140.3010642 | 237.0459 | 168.96% |
| 16 | ⊦0.84563 | 7.71461 | -6.633703 | 0.078425802 | 7.218651 | 9204.43% |
| 17 | 9.406157 | -5.241917 | 3.147 | 2.437080036 | 7.349796 | 301.58% |
| 18 | 2.034282 | 5.826035 | -2.768123 | 1.697398119 | 4.306972 | 253.74% |
| 19 | 0.823912 | 2.394956 | -0.230114 | 0.996251197 | 1.320994 | 132.60% |
| 20 | 3.742991 | -4.066856 | 83.37577 | 27.68396927 | 48.38834 | 174.79% |
| 21 | 0.726671 | 2.56852 | 0.080715 | 1.12530195 | 1.29092 | 114.72% |
| 22 | 0.649364 | 1.14899 | 1.181846 | 0.993399925 | 0.298396 | 30.04% |
| 23 | 1.16224 | 1.845211 | 1.251478 | 1.419643166 | 0.371243 | 26.15% |
| 24 | 1.252406 | 1.94681 | 1.329072 | 1.509429484 | 0.380718 | 25.22% |
| 25 | 1.792069 | 1.699725 | 1.280887 | 1.590893575 | 0.272415 | 17.12% |
| 26 | 1.580045 | 1.662281 | 1.493929 | 1.578751658 | 0.084184 | 5.33% |
| 27 | 1.607573 | 1.739522 | 1.543598 | 1.630230871 | 0.099908 | 6.13% |
| 28 | 1.706771 | 1.774332 | 1.614167 | 1.698423258 | 0.080408 | 4.73% |
| 29 | 1.710515 | 1.885357 | 1.636839 | 1.744237138 | 0.127645 | 7.32% |
| 30 | 1.776245 | 1.921143 | 1.722583 | 1.80665715 | 0.102714 | 5.69% |
| 31 | 1.856199 | 1.959404 | 1.794686 | 1.870096191 | 0.083233 | 4.45% |
| 32 | 1.894297 | 2.034529 | 1.850262 | 1.926362634 | 0.096228 | 5.00% |
| 33 | 1.948812 | 2.088568 | 1.8764 | 1.97125997 | 0.107851 | 5.47% |
| 34 | 2.055142 | 2.179001 | 1.902808 | 2.045650602 | 0.138341 | 6.76% |
| 35 | 2.155307 | 2.218981 | 1.937282 | 2.103856573 | 0.147729 | 7.02% |
| 36 | 2.214953 | 2.205164 | 1.949923 | 2.123346531 | 0.150269 | 7.08% |
| 37 | 2.251617 | 2.187937 | 1.949569 | 2.129707529 | 0.15922 | 7.48% |
| 38 | 2.336189 | 2.27784 | 1.965569 | 2.193199415 | 0.199281 | 9.09% |
| 39 | 2.330774 | 2.295424 | 1.988653 | 2.204950261 | 0.188151 | 8.53% |
| 40 | 2.345601 | 2.308908 | 1.995724 | 2.216744283 | 0.192286 | 8.67% |

FIGURE 1B-2

**Experimental Results for Ratio Differences**

Amplicon #1 to Amplicon #2

| |
|---|
| 156.0245% |
| 112.6700% |
| 156.4931% |
| 30.4450% |
| -665.7107% |
| 85.6653% |
| 8434.2949% |
| -1964.1104% |
| 286.9676% |
| 198.5229% |
| -115.7559% |
| 825.0464% |
| 41.1601% |
| -958.0202% |
| 7207.8269% |
| 103.1918% |
| 9.1202% |
| 70.6472% |
| 137.6110% |
| 1216.8535% |
| 209.7350% |
| 77.1866% |
| 58.6691% |
| 44.8456% |
| 45.2626% |
| 38.9985% |
| 39.9812% |
| 40.3364% |
| 39.0604% |
| 40.0939% |
| 39.1538% |
| 39.9217% |
| 40.1977% |
| 38.3308% |
| 37.4032% |
| 37.9479% |
| 38.5316% |
| 37.4516% |
| 37.3324% |
| 38.7781% |

FIGURE 1B-3

# FIGURE 2A

## CCR2 WildType & Variant Alleles 2% Rox-dCTP

EP 1 606 389 B1

**CCR2 Wildtype**
**Average Sybr/Average Fret Values for Each Replicate**

| Cycle | Rep 1 | Rep 2 | Rep 3 | AVERAGE | STDEV | CV |
|---|---|---|---|---|---|---|
| 1 | 0.667107 | 2.217445 | 38.29633 | 13.72695948 | 21.29181 | 155.11% |
| 2 | 0.146824 | 0.381992 | -15.73643 | -5.069205098 | 9.238837 | -182.25% |
| 3 | -0.119342 | 1.053067 | -2.704187 | -0.590153763 | 1.922365 | -325.74% |
| 4 | -2.15782 | 1.851634 | -0.634134 | -0.313439746 | 2.023874 | -645.70% |
| 5 | 8.156268 | 1.880859 | 0.425799 | 3.487642139 | 4.108083 | 117.79% |
| 6 | 3.216423 | 2.294206 | 22.78651 | 9.432378769 | 11.5742 | 122.71% |
| 7 | 3.132541 | 2.114741 | -3.934042 | 0.43774658 | 3.820128 | 872.68% |
| 8 | 14.51735 | 3.168735 | -1.980053 | 5.235344284 | 8.44063 | 161.22% |
| 9 | 11.38033 | -4.059303 | -1.759569 | 1.853818426 | 8.329945 | 449.34% |
| 10 | -0.338378 | -0.006337 | -0.15179 | -0.165501457 | 0.166445 | -100.57% |
| 11 | 1.640792 | 0.173039 | 0.430414 | 0.748081452 | 0.783747 | 104.77% |
| 12 | 1.03154 | 0.672875 | 1.078007 | 0.927474143 | 0.22171 | 23.90% |
| 13 | 2.084012 | 0.90832 | 1.326533 | 1.439621546 | 0.595948 | 41.40% |
| 14 | 2.494504 | 1.495494 | 1.288987 | 1.759661703 | 0.644714 | 36.64% |
| 15 | 101.874 | 2.614227 | 1.909203 | 35.4658135 | 57.51227 | 162.16% |
| 16 | -13.9123 | 5.071955 | 2.216244 | -2.208034013 | 10.23627 | -463.59% |
| 17 | -97.82619 | 6.795866 | 3.678618 | -29.11723551 | 59.52411 | -204.43% |
| 18 | 8.710339 | -14.62503 | 5.435509 | -0.159727108 | 12.63388 | -7909.66% |
| 19 | 5.24464 | -14.0147 | -4.198925 | -4.322995801 | 9.630271 | -222.77% |
| 20 | 3.694187 | -1.091677 | 0.625404 | 1.075971389 | 2.424537 | 225.33% |
| 21 | 2.027339 | 0.649596 | 1.265192 | 1.314042268 | 0.690169 | 52.52% |
| 22 | 11.55171 | 2.031121 | 1.93407 | 5.172299045 | 5.524942 | 106.82% |
| 23 | 3.576946 | 2.81418 | 2.492283 | 2.961136194 | 0.557064 | 18.81% |
| 24 | 3.327599 | 2.516112 | 2.812986 | 2.885565652 | 0.410583 | 14.23% |
| 25 | 3.043009 | 2.807799 | 2.985266 | 2.945357959 | 0.122578 | 4.16% |
| 26 | 3.166248 | 2.885493 | 3.153345 | 3.068362064 | 0.1585 | 5.17% |
| 27 | 3.214431 | 3.159819 | 3.382199 | 3.252149678 | 0.115889 | 3.56% |
| 28 | 3.337999 | 3.300253 | 3.481789 | 3.373346766 | 0.095791 | 2.84% |
| 29 | 3.430281 | 3.466913 | 3.612165 | 3.503119694 | 0.096196 | 2.75% |
| 30 | 3.487599 | 3.536821 | 3.650505 | 3.558308344 | 0.083552 | 2.35% |
| 31 | 3.498955 | 3.579661 | 3.68677 | 3.588461968 | 0.094217 | 2.63% |
| 32 | 3.490889 | 3.574434 | 3.665362 | 3.576895112 | 0.087263 | 2.44% |
| 33 | 3.473745 | 3.563309 | 3.659916 | 3.56565701 | 0.093108 | 2.61% |
| 34 | 3.423783 | 3.538434 | 3.622459 | 3.528225403 | 0.099731 | 2.83% |
| 35 | 3.388173 | 3.503768 | 3.577523 | 3.489821409 | 0.095442 | 2.73% |
| 36 | 3.33311 | 3.459902 | 3.521373 | 3.438128231 | 0.096001 | 2.79% |
| 37 | 3.293913 | 3.404311 | 3.473821 | 3.390681578 | 0.090725 | 2.68% |
| 38 | 3.23362 | 3.352961 | 3.441206 | 3.342595557 | 0.10418 | 3.12% |
| 39 | 3.183978 | 3.299849 | 3.404995 | 3.296273927 | 0.110552 | 3.35% |
| 40 | 3.138745 | 3.262627 | 3.385576 | 3.262316038 | 0.123416 | 3.78% |

## FIGURE 2B-1

**CCR2 Variant**
**Average Sybr/Average Fret Values for Each Replicate**

| Cycle | Rep 1 | Rep 2 | Rep 3 | AVERAGE | STDEV | CV |
|---|---|---|---|---|---|---|
| 1 | 1.88152 | 3.289289 | 1.835657 | 2.335488677 | 0.826334 | 35.38% |
| 2 | 1.8425 | 2.319443 | 0.557027 | 1.572989995 | 0.911594 | 57.95% |
| 3 | 2.018545 | 6.717596 | 0.539625 | 3.091921732 | 3.225823 | 104.33% |
| 4 | 1.344104 | 1.051469 | 0.211361 | 0.86897811 | 0.588008 | 67.67% |
| 5 | -7.43812 | 1.368881 | 0.050804 | -2.006144796 | 4.750168 | -236.78% |
| 6 | 2.694848 | 2.489626 | -0.458962 | 1.575170793 | 1.764596 | 112.03% |
| 7 | 2.77556 | 2.41081 | -2.147867 | 1.012834254 | 2.743317 | 270.86% |
| 8 | 2.944435 | 3.632256 | 1.864269 | 2.813653384 | 0.89122 | 31.67% |
| 9 | 2.411161 | 2.495122 | 0.897715 | 1.934665773 | 0.899007 | 46.47% |
| 10 | 0.355718 | 0.77769 | 0.855837 | 0.663081467 | 0.269037 | 40.57% |
| 11 | -1.63318 | 1.666406 | 0.278152 | 0.103792299 | 1.656689 | 1596.16% |
| 12 | 3.155954 | 0.8921 | -0.500012 | 1.182680663 | 1.845223 | 156.02% |
| 13 | 2.854295 | 0.528998 | -3.241911 | 0.047127102 | 3.076537 | 6528.17% |
| 14 | 5.556324 | 0.977395 | 6.449667 | 4.327795356 | 2.935711 | 67.83% |
| 15 | 4.07138 | 2.514512 | 2.840811 | 3.142234086 | 0.821037 | 26.13% |
| 16 | 2.586758 | 4.930101 | 2.610793 | 3.375884048 | 1.346045 | 39.87% |
| 17 | 1.96841 | 5.926294 | 1.455402 | 3.116702128 | 2.446661 | 78.50% |
| 18 | 1.629912 | 3.60884 | 2.732033 | 2.656928319 | 0.9916 | 37.32% |
| 19 | 2.494506 | 3.674855 | 1.683585 | 2.617648569 | 1.001331 | 38.25% |
| 20 | 1.262759 | -0.583484 | 1.432482 | 0.703919321 | 1.118149 | 158.85% |
| 21 | 1.500242 | 1.165522 | -1.209515 | 0.485416643 | 1.477364 | 304.35% |
| 22 | 2.647097 | 3.160163 | 6.037189 | 3.948149655 | 1.827258 | 46.28% |
| 23 | 2.48421 | 4.561198 | 1.968805 | 3.004737995 | 1.372347 | 45.67% |
| 24 | 3.428694 | 4.569681 | 2.067096 | 3.355156996 | 1.252912 | 37.34% |
| 25 | 3.353462 | 4.251713 | 2.227133 | 3.277436033 | 1.014429 | 30.95% |
| 26 | 3.346317 | 3.865984 | 2.713645 | 3.308648695 | 0.577092 | 17.44% |
| 27 | 3.419246 | 3.722702 | 3.007049 | 3.382999137 | 0.3592 | 10.62% |
| 28 | 3.590777 | 3.755398 | 3.252784 | 3.532986466 | 0.256242 | 7.25% |
| 29 | 3.744311 | 3.865461 | 3.424337 | 3.67803628 | 0.227907 | 6.20% |
| 30 | 3.870369 | 3.961713 | 3.552167 | 3.794749737 | 0.21499 | 5.67% |
| 31 | 3.929575 | 3.989519 | 3.682048 | 3.867047318 | 0.162994 | 4.21% |
| 32 | 3.94644 | 3.976112 | 3.715942 | 3.879497907 | 0.142419 | 3.67% |
| 33 | 3.921082 | 3.917344 | 3.711986 | 3.850137346 | 0.119658 | 3.11% |
| 34 | 3.880509 | 3.856603 | 3.659714 | 3.798941843 | 0.121166 | 3.19% |
| 35 | 3.844622 | 3.808916 | 3.617741 | 3.75709287 | 0.121996 | 3.25% |
| 36 | 3.807089 | 3.781557 | 3.571605 | 3.720083661 | 0.129218 | 3.47% |
| 37 | 3.782058 | 3.748889 | 3.533953 | 3.688299913 | 0.134693 | 3.65% |
| 38 | 3.764801 | 3.691086 | 3.483472 | 3.646452908 | 0.145879 | 4.00% |
| 39 | 3.748526 | 3.625236 | 3.43737 | 3.603710491 | 0.156691 | 4.35% |
| 40 | 3.742665 | 3.55626 | 3.38568 | 3.561534917 | 0.178551 | 5.01% |

FIGURE 2B-2

**Experimental Results for Ratio Differences**

% Difference Between Alleles

| |
|---|
| -487.76% |
| 422.27% |
| 119.09% |
| 136.07% |
| 273.85% |
| -498.82% |
| 56.78% |
| -86.07% |
| 4.18% |
| 124.96% |
| -620.75% |
| 21.58% |
| -2954.76% |
| 59.34% |
| -1028.68% |
| 165.41% |
| 1034.23% |
| 106.01% |
| 265.15% |
| -52.85% |
| -170.70% |
| -31.01% |
| 1.45% |
| 14.00% |
| 10.13% |
| 7.26% |
| 3.87% |
| 4.52% |
| 4.76% |
| 6.23% |
| 7.20% |
| 7.80% |
| 7.39% |
| 7.13% |
| 7.11% |
| 7.58% |
| 8.07% |
| 8.33% |
| 8.53% |
| 8.40% |

FIGURE 2B-3

Sybrgreen Amplification Plots of BHQ-10-dUTP Titration Reaction from 0%-5%

FIGURE 3

FIGURE 4A

**CCR2 WildType Allele Replicates**
**Average Sybr/Average Fret Values for Each Replicate**

| Cycle | Rep 1 | Rep 2 | Rep 3 | AVERAGE | STDEV | CV |
|---|---|---|---|---|---|---|
| 1 | -4.169718 | -3.322128 | -6.812611 | -4.76815251 | 1.820566 | -38.18% |
| 2 | -1.788977 | -1.2719 | -3.97053 | -2.34380233 | 1.432315 | -61.11% |
| 3 | -1.507806 | -0.57017 | -2.760112 | -1.61269601 | 1.098732 | -68.13% |
| 4 | -0.613769 | 0.181669 | -0.927927 | -0.45334228 | 0.571929 | -126.16% |
| 5 | 0.299785 | 0.714617 | 0.892181 | 0.63552797 | 0.304014 | 47.84% |
| 6 | 1.34928 | 1.103428 | 2.100032 | 1.51758016 | 0.519181 | 34.21% |
| 7 | 2.28066 | 1.295086 | 2.823307 | 2.13301766 | 0.774735 | 36.32% |
| 8 | 3.748601 | 1.002913 | 2.576498 | 2.44267079 | 1.377727 | 56.40% |
| 9 | 2.58884 | -1.52239 | 51.69746 | 17.5879718 | 29.61112 | 168.36% |
| 10 | 3.020007 | 7.085075 | 5.586285 | 5.23045585 | 2.055762 | 39.30% |
| 11 | 4.07612 | 4.368412 | 5.560921 | 4.66848444 | 0.78657 | 16.85% |
| 12 | 4.358759 | 3.78151 | 5.575561 | 4.57194322 | 0.915828 | 20.03% |
| 13 | 4.333559 | 3.640381 | 5.798848 | 4.59092961 | 1.102009 | 24.00% |
| 14 | 0.80683 | 3.766916 | -4986.276 | -1660.5673 | 2880.148 | -173.44% |
| 15 | 4.449863 | 10.23758 | 2.905231 | 5.86422594 | 3.86538 | 65.91% |
| 16 | 4.554141 | 4.200055 | 3.955843 | 4.23667981 | 0.300826 | 7.10% |
| 17 | 4.72848 | 4.53105 | 4.337371 | 4.5323002 | 0.195557 | 4.31% |
| 18 | 4.815434 | 4.672385 | 4.484777 | 4.65753197 | 0.165828 | 3.56% |
| 19 | 4.91949 | 4.816735 | 4.591006 | 4.77574395 | 0.168035 | 3.52% |
| 20 | 4.953867 | 4.898582 | 4.669322 | 4.84059033 | 0.150877 | 3.12% |
| 21 | 4.986207 | 4.981393 | 4.727993 | 4.89853117 | 0.14771 | 3.02% |
| 22 | 4.991256 | 4.992834 | 4.756762 | 4.91361734 | 0.135843 | 2.76% |
| 23 | 4.990282 | 4.99998 | 4.774411 | 4.92155773 | 0.127525 | 2.59% |
| 24 | 4.974268 | 4.984066 | 4.770696 | 4.90967674 | 0.120461 | 2.45% |
| 25 | 4.953809 | 4.956599 | 4.769533 | 4.89331379 | 0.107206 | 2.19% |
| 26 | 4.93261 | 4.928543 | 4.749246 | 4.870133 | 0.104711 | 2.15% |
| 27 | 4.915535 | 4.898882 | 4.720769 | 4.84506201 | 0.107963 | 2.23% |
| 28 | 4.884613 | 4.861991 | 4.691601 | 4.81273469 | 0.105513 | 2.19% |
| 29 | 4.847951 | 4.829436 | 4.663489 | 4.78029184 | 0.101577 | 2.12% |
| 30 | 4.810327 | 4.786459 | 4.615344 | 4.73737667 | 0.106355 | 2.25% |
| 31 | 4.763353 | 4.742306 | 4.570414 | 4.69202439 | 0.105843 | 2.26% |
| 32 | 4.714624 | 4.691036 | 4.519207 | 4.64162249 | 0.106669 | 2.30% |
| 33 | 4.667368 | 4.645027 | 4.461875 | 4.59142344 | 0.112747 | 2.46% |
| 34 | 4.614867 | 4.58666 | 4.406129 | 4.53588541 | 0.113254 | 2.50% |
| 35 | 4.563989 | 4.531222 | 4.354341 | 4.48318399 | 0.112778 | 2.52% |
| 36 | 4.515904 | 4.477483 | 4.297943 | 4.43044332 | 0.116345 | 2.63% |
| 37 | 4.463314 | 4.423232 | 4.249492 | 4.37867916 | 0.11366 | 2.60% |
| 38 | 4.415284 | 4.370619 | 4.201336 | 4.3290798 | 0.112861 | 2.61% |
| 39 | 4.370762 | 4.32225 | 4.151831 | 4.28161443 | 0.114983 | 2.69% |
| 40 | 4.322731 | 4.281644 | 4.097435 | 4.23393669 | 0.119986 | 2.83% |

## FIGURE 4B-1

CCR2 Variant Allele Replicates
Average Sybr/Average Fret Values for Each Replicate

| Cycle | Rep 1 | Rep 2 | Rep 3 | AVERAGE | STDEV | CV |
|---|---|---|---|---|---|---|
| 1 | -3.16248 | -6.918808 | -5.670828 | -5.250705344 | 1.91308 | -36.43% |
| 2 | -1.753588 | -3.297283 | -4.783254 | -3.278041644 | 1.514925 | -46.21% |
| 3 | -1.109105 | -2.045965 | -2.785771 | -1.980280209 | 0.840261 | -42.43% |
| 4 | -0.284741 | -0.588873 | -0.835292 | -0.569635633 | 0.275779 | -48.41% |
| 5 | 0.586501 | 0.55108 | 0.637121 | 0.591567241 | 0.043244 | 7.31% |
| 6 | 1.20481 | 1.248464 | 1.479066 | 1.310779935 | 0.147365 | 11.24% |
| 7 | 2.029066 | 2.248292 | 1.978441 | 2.085266522 | 0.143435 | 6.88% |
| 8 | 2.751075 | 2.958533 | 1.500938 | 2.403515175 | 0.788507 | 32.81% |
| 9 | 0.519978 | 2.870439 | 0.147524 | 1.179313661 | 1.47635 | 125.19% |
| 10 | 7.161238 | 2.827505 | -11.54708 | -0.519445668 | 9.792949 | -1885.27% |
| 11 | 4.804491 | 2.268758 | 4.678061 | 3.917103589 | 1.428908 | 36.48% |
| 12 | 5.078069 | 2.652867 | 4.100653 | 3.943863148 | 1.22018 | 30.94% |
| 13 | 5.987094 | 2.746104 | 3.302113 | 4.011770416 | 1.733123 | 43.20% |
| 14 | 4.996641 | 2.602215 | 2.984583 | 3.527812853 | 1.28633 | 36.46% |
| 15 | 5.063729 | 2.120786 | 2.051664 | 3.078726426 | 1.71941 | 55.85% |
| 16 | 5.255232 | 1.02748 | -3.343396 | 0.979772108 | 4.299512 | 438.83% |
| 17 | 5.778037 | 38.10286 | 8.842373 | 17.57442447 | 17.84405 | 101.53% |
| 18 | 5.685796 | 7.358988 | 6.354408 | 6.466397523 | 0.842199 | 13.02% |
| 19 | 5.679116 | 6.180073 | 5.90154 | 5.92024267 | 0.251002 | 4.24% |
| 20 | 5.749381 | 6.07534 | 5.726741 | 5.850487294 | 0.195057 | 3.33% |
| 21 | 5.806716 | 6.006144 | 5.740774 | 5.851211352 | 0.138167 | 2.36% |
| 22 | 5.817832 | 5.986198 | 5.732633 | 5.845554167 | 0.129035 | 2.21% |
| 23 | 5.814741 | 5.921536 | 5.729226 | 5.821834376 | 0.096351 | 1.66% |
| 24 | 5.786308 | 5.863608 | 5.701171 | 5.783695842 | 0.08125 | 1.40% |
| 25 | 5.733352 | 5.77996 | 5.666308 | 5.726539987 | 0.057131 | 1.00% |
| 26 | 5.690259 | 5.731547 | 5.621696 | 5.681167166 | 0.055487 | 0.98% |
| 27 | 5.633206 | 5.667379 | 5.560704 | 5.620429593 | 0.054473 | 0.97% |
| 28 | 5.58881 | 5.615071 | 5.504572 | 5.56948399 | 0.057729 | 1.04% |
| 29 | 5.524624 | 5.54324 | 5.447334 | 5.505066203 | 0.050857 | 0.92% |
| 30 | 5.473837 | 5.487112 | 5.381293 | 5.447414253 | 0.057646 | 1.06% |
| 31 | 5.415489 | 5.428708 | 5.315589 | 5.386595308 | 0.061847 | 1.15% |
| 32 | 5.35615 | 5.368234 | 5.25767 | 5.327351286 | 0.060648 | 1.14% |
| 33 | 5.297076 | 5.306531 | 5.198105 | 5.267237152 | 0.060056 | 1.14% |
| 34 | 5.249007 | 5.259978 | 5.130224 | 5.213069476 | 0.071956 | 1.38% |
| 35 | 5.200461 | 5.204547 | 5.081522 | 5.162176819 | 0.069879 | 1.35% |
| 36 | 5.152012 | 5.147657 | 5.028561 | 5.109409964 | 0.070051 | 1.37% |
| 37 | 5.105926 | 5.096322 | 4.976897 | 5.059715034 | 0.071883 | 1.42% |
| 38 | 5.062046 | 5.050942 | 4.931084 | 5.014690651 | 0.072618 | 1.45% |
| 39 | 5.020751 | 5.008517 | 4.894743 | 4.974670224 | 0.069489 | 1.40% |
| 40 | 4.984154 | 4.973614 | 4.847086 | 4.934951379 | 0.076276 | 1.55% |

FIGURE 4B-2

**Experimental Results for Ratio Differences**

% Difference Between Alleles

| |
|---|
| 9.19% |
| 28.50% |
| 18.56% |
| 20.42% |
| -7.43% |
| -15.78% |
| -2.29% |
| -1.63% |
| -1391.37% |
| 1106.93% |
| -19.18% |
| -15.93% |
| -14.44% |
| 47170.73% |
| -90.48% |
| -332.41% |
| 74.21% |
| 27.97% |
| 19.33% |
| 17.26% |
| 16.28% |
| 15.94% |
| 15.46% |
| 15.11% |
| 14.55% |
| 14.28% |
| 13.80% |
| 13.59% |
| 13.17% |
| 13.03% |
| 12.89% |
| 12.87% |
| 12.83% |
| 12.99% |
| 13.15% |
| 13.29% |
| 13.46% |
| 13.67% |
| 13.93% |
| 14.21% |

FIGURE 4B-3

CCR2 Variant and WildType Alleles with 2% Rox-dUTP and 3% BHQ-10-dUTP

FIGURE 5A

FIGURE 5B

EP 1 606 389 B1

2% ROX dUTP 0% BHQ-10-dUTP Variant Allele
· Average SYBR/FRET Ratio For Each Replicate

| Cycle | Rep 1 | Rep 2 | Rep 3 | AVERAGE | ·STDEV | CV |
|---|---|---|---|---|---|---|
| 1 | -0.371988 | -0.955155 | -0.295964 | -0.541035845 | 0.3606468 | -66.66% |
| 2 | -0.059014 | -0.199159 | 0.082554 | -0.058539826 | 0.1408573 | -240.62% |
| 3 | -0.034803 | 0.103155 | 0.14749 | 0.071947506 | 0.0950692 | 132.14% |
| 4 | 0.321925 | 0.668331 | 0.551819 | 0.514024956 | 0.1762687 | 34.29% |
| 5 | 0.359972 | 2.441679 | 0.716456 | 1.172702235 | 1.1133266 | 94.94% |
| 6 | 0.432743 | -2.898435 | -40.2907 | -14.25212919 | 22.611488 | -158.65% |
| 7 | 0.069664 | -0.599367 | -1.084331 | -0.538011291 | 0.5794369 | -107.70% |
| 8 | 0.539409 | 0.192439 | -0.061497 | 0.223450166 | 0.3016508 | 135.00% |
| 9 | 0.658127 | 0.755346 | 1.333784 | 0.915752451 | 0.3652747 | .39.89% |
| 10 | 0.017483 | 0.701942 | 1.006127 | 0.575183927 | 0.5063646 | 88.04% |
| 11 | 0.639564 | 0.708949 | 1.532108 | 0.960207328 | 0.4964944 | 51.71% |
| 12 | 0.650585 | 0.969183 | 1.029819 | 0.88319546 | 0.2037154 | 23.07% |
| 13 | 0.907481 | 1.066203 | 0.977448 | 0.983710901 | 0.079546 | 8.09% |
| 14 | 0.560839 | 1.547044 | 1.010334 | 1.039405509 | 0.4937448 | 47.50% |
| 15 | 1.003867 | -6.355341 | 1.017057 | -1.444805528 | 4.2526534 | -294.34% |
| 16 | 0.31668 | 0.459456 | 0.584895 | 0.453677006 | 0.1342008 | 29.58% |
| 17 | 0.783224 | 1.03071 | 1.01967 | 0.944534448 | 0.1398082 | 14.80% |
| 18 | 1.12521 | 1.262192 | 1.128592 | 1.171998085 | 0.0781285 | 6.67% |
| 19 | 1.37048 | 1.446523 | 1.370422 | 1.395808538 | 0.0439204 | 3.15% |
| 20 | 1.49324 | 1.523558 | 1.528765 | 1.515187727 | 0.0191845 | 1.27% |
| 21 | 1.621594 | 1.634786 | 1.699962 | 1.652113858 | 0.0419593 | 2.54% |
| 22 | 1.746918 | 1.736878 | 1.821633 | 1.768476098 | 0.0463079 | 2.62% |
| 23 | 1.855781 | 1.829355 | 1.909871 | 1.865002258 | 0.0410423 | 2.20% |
| 24 | 1.921636 | 1.899671 | 1.969171 | 1.93015916 | 0.0355253 | 1.84% |
| 25 | 1.952334 | 1.920777 | 1.995942 | 1.956351348 | 0.0377433 | 1.93% |
| 26 | 1.950736 | 1.917313 | 1.992734 | 1.953594365 | 0.0377918 | 1.93% |
| 27 | 1.938776 | 1.896872 | 1.978305 | 1.937984387 | 0.0407226 | 2.10% |
| 28 | 1.912017 | 1.8769 | 1.951364 | 1.913427024 | 0.0372518 | 1.95% |
| 29 | 1.895004 | 1.853527 | 1.929903 | 1.892811498 | 0.038235 | 2.02% |
| 30 | 1.875691 | 1.831792 | 1.901926 | 1.869802817 | 0.0354359 | 1.90% |
| 31 | 1.855049 | 1.796388 | 1.874342 | 1.841926363 | 0.0406001 | 2.20% |
| 32 | 1.828237 | 1.772728 | 1.852383 | 1.817782358 | 0.0408437 | .2.25% |
| 33 | 1.802771 | 1.755036 | 1.831779 | 1.796528739 | 0.0387502 | 2.16% |
| 34 | 1.786187 | 1.744984 | 1.817803 | 1.782991303 | 0.0365148 | 2.05% |
| 35 | 1.770911 | 1.729641 | 1.79859 | · 1.766380571 | 0.0346969 | 1.96% |
| 36 | 1.757457 | 1.711256 | 1.787263 | 1.751992303 | 0.038297 | 2.19% |
| 37 | 1.740549 | 1.700643 | 1.770589 | 1.737260615 | 0.0350886 | 2.02% |
| 38 | 1.724974 | 1.689324 | 1.761174 | 1.72515763 | 0.0359253 | 2.08% |
| 39 | 1.71063 | 1.678084 | 1.747461 | 1.712058188 | 0.0347109 | 2.03% |
| 40 | 1.696832 | 1.664296 | 1.743091 | 1.701406207 | 0.0395963 | 2.33% |

FIGURE 5C-1

2% ROX dUTP 0% BHQ-10-dUTP WildType Allele
Average SYBR/FRET Ratio For Each Replicate

| Cycle | Rep 1 | Rep 2 | Rep 3 | AVERAGE | STDEV | CV |
|---|---|---|---|---|---|---|
| 1 | -0.395946 | -0.129268 | -0.370227 | -0.298480155 | 0.147105 | -49.28% |
| 2 | -0.065077 | 0.054987 | -0.1173 | -0.042463307 | 0.088342 | -208.04% |
| 3 | -0.012756 | 0.069089 | -0.044556 | 0.003925564 | 0.05863 | 1493.55% |
| 4 | 0.106302 | 0.202259 | 0.108005 | 0.138855032 | 0.054916 | 39.55% |
| 5 | 0.159863 | 0.222944 | -0.389033 | -0.002075483 | 0.336596 | -16217.73% |
| 6 | 0.260349 | 0.193763 | 0.007463 | 0.153858635 | 0.131081 | 85.20% |
| 7 | 1.362048 | 0.548042 | -0.167334 | 0.580918612 | 0.765221 | 131.73% |
| 8 | 0.042019 | 0.153202 | -0.429786 | -0.078188197 | 0.309525 | -395.87% |
| 9 | 0.154415 | 0.360978 | -0.573009 | -0.019205655 | 0.490602 | -2554.47% |
| 10 | 0.291766 | 0.613253 | -1.560277 | -0.21841926 | 1.173147 | -537.11% |
| 11 | 0.291182 | 0.560608 | 0.194171 | 0.348653592 | 0.189858 | 54.45% |
| 12 | 0.334628 | 0.633419 | 1.430576 | 0.799541214 | 0.566545 | 70.86% |
| 13 | 0.335501 | 0.53879 | 2.302695 | 1.058995482 | 1.081861 | 102.16% |
| 14 | -0.289909 | -0.467614 | -6.895171 | -2.550897929 | 3.7633 | -147.53% |
| 15 | 2.104546 | 2.998773 | 1.141837 | 2.081718811 | 0.928678 | 44.61% |
| 16 | 1.67009 | 1.895402 | 1.643547 | 1.736346357 | 0.138384 | 7.97% |
| 17 | 1.53619 | 1.628108 | 1.593211 | 1.585836185 | 0.046401 | 2.93% |
| 18 | 1.637426 | 1.562517 | 1.604308 | 1.601417002 | 0.037538 | 2.34% |
| 19 | 1.780744 | 1.659375 | 1.736872 | 1.725663728 | 0.061456 | 3.56% |
| 20 | 1.964498 | 1.787557 | 1.901138 | 1.884397863 | 0.08965 | 4.76% |
| 21 | 2.082676 | 1.920149 | 2.058211 | 2.020345375 | 0.08763 | 4.34% |
| 22 | 2.147879 | 1.999813 | 2.110416 | 2.086036003 | 0.076985 | 3.69% |
| 23 | 2.163426 | 2.049976 | 2.145961 | 2.119787426 | 0.061086 | 2.88% |
| 24 | 2.1514 | 2.057237 | 2.147151 | 2.118596037 | 0.053181 | 2.51% |
| 25 | 2.11067 | 2.048396 | 2.151178 | 2.103414884 | 0.051774 | 2.46% |
| 26 | 2.080106 | 2.021072 | 2.130322 | 2.077166562 | 0.054684 | 2.63% |
| 27 | 2.048693 | 1.993664 | 2.106285 | 2.049547169 | 0.056316 | 2.75% |
| 28 | 2.031053 | 1.967876 | 2.068662 | 2.022530403 | 0.050931 | 2.52% |
| 29 | 2.006662 | 1.938949 | 2.032401 | 1.992670635 | 0.048271 | 2.42% |
| 30 | 1.985403 | 1.914065 | 1.997831 | 1.965766247 | 0.045204 | 2.30% |
| 31 | 1.958404 | 1.887245 | 1.97377 | 1.939806386 | 0.046163 | 2.38% |
| 32 | 1.935285 | 1.865494 | 1.948623 | 1.916467032 | 0.044645 | 2.33% |
| 33 | 1.911359 | 1.84816 | 1.928668 | 1.896062477 | 0.042378 | 2.24% |
| 34 | 1.901097 | 1.834504 | 1.907361 | 1.880987023 | 0.040377 | 2.15% |
| 35 | 1.889166 | 1.81882 | 1.892258 | 1.866748017 | 0.041536 | 2.23% |
| 36 | 1.877307 | 1.800941 | 1.878145 | 1.852130642 | 0.044334 | 2.39% |
| 37 | 1.860921 | 1.772495 | 1.867151 | 1.833522091 | 0.052943 | 2.89% |
| 38 | 1.845802 | 1.758885 | 1.852139 | 1.818942144 | 0.052107 | 2.86% |
| 39 | 1.834066 | 1.741647 | 1.83599 | 1.803900993 | 0.053922 | 2.99% |
| 40 | 1.823893 | 1.742213 | 1.819273 | 1.795126143 | 0.045883 | 2.56% |

FIGURE 5C-2

Experimental Results for Ratio Differences

2% ROX dUTP 3% BHQ-10-dUTP Variant
Average SYBR/FRET Ratio For Each Rep

| % Difference Between Alleles | Cycle | Rep 1 | Rep 2 | Rep 3 |
|---|---|---|---|---|
| -81.26% | 1 | -0.300908597 | -0.228357031 | -0.421495 |
| -37.86% | 2 | 0.165973375 | 0.072029505 | -0.114362 |
| -1732.79% | 3 | 0.229416552 | 0.228191366 | -0.194956 |
| -270.19% | 4 | 0.489424949 | 0.333456516 | 0.459009 |
| 66602.62% | 5 | 2.058527359 | 0.561221624 | 0.546645 |
| 9363.13% | 6 | 0.376370815 | 0.3262914 | 0.654986 |
| 192.61% | 7 | -1.736498747 | 0.177608127 | -2.542664 |
| 385.79% | 8 | -4.688639623 | 0.343898383 | 0.042311 |
| 4868.14% | 9 | 0.639090258 | 0.533564708 | 0.537086 |
| 363.34% | 10 | -1.237043121 | 0.521387735 | 0.819193 |
| -175.40% | 11 | 1.106607555 | 0.85073645 | 0.761178 |
| -10.46% | 12 | 0.811725428 | 0.718341211 | 0.602761 |
| 7.11% | 13 | 0.697523722 | 4.009307877 | 0.298491 |
| 140.75% | 14 | 1.327693303 | -3.413248323 | 0.329545 |
| 169.40% | 15 | -0.441069971 | -1.030664369 | 1.214223 |
| 73.87% | 16 | 0.199150048 | 0.217459646 | 0.348639 |
| 40.44% | 17 | 0.541274762 | 0.710418508 | 0.552928 |
| 26.81% | 18 | 0.803820037 | 0.796915339 | 0.781178 |
| 19.11% | 19 | 1.033651393 | 0.934123474 | 0.968136 |
| 19.59% | 20 | 1.189204225 | 1.093522848 | 1.142027 |
| 18.23% | 21 | 1.312702685 | 1.265384161 | 1.304627 |
| 15.22% | 22 | 1.449043186 | 1.419605461 | 1.451797 |
| 12.02% | 23 | 1.581191643 | 1.557695256 | 1.590743 |
| 8.89% | 24 | 1.675889706 | 1.653619895 | 1.709669 |
| 6.99% | 25 | 1.750626076 | 1.71038834 | 1.778821 |
| 5.95% | 26 | 1.778447735 | 1.739471972 | 1.807285 |
| 5.44% | 27 | 1.802083632 | 1.748304099 | 1.807429 |
| 5.39% | 28 | 1.803954102 | 1.748584844 | 1.802157 |
| 5.01% | 29 | 1.807107498 | 1.733543524 | 1.790652 |
| 4.88% | 30 | 1.796720298 | 1.723258013 | 1.777346 |
| 5.05% | 31 | 1.780788986 | 1.702904002 | 1.753054 |
| 5.15% | 32 | 1.763497466 | 1.687686513 | 1.727228 |
| 5.25% | 33 | 1.745106152 | 1.663987863 | 1.705227 |
| 5.21% | 34 | 1.723162572 | 1.654022971 | 1.68764 |
| 5.38% | 35 | 1.694150569 | 1.637611274 | 1.673402 |
| 5.41% | 36 | 1.671679957 | 1.62679054 | 1.658713 |
| 5.25% | 37 | 1.652774471 | 1.601493574 | 1.643801 |
| 5.16% | 38 | 1.635978794 | 1.580317238 | 1.627389 |
| 5.09% | 39 | 1.616823844 | 1.554627425 | 1.60886 |
| 5.22% | 40 | 1.596544851 | 1.535714532 | 1.591453 |

FIGURE 5C-3

**ı Allele
plicate**

2% ROX dUTP 3% BHQ-10-dUTP WII
Average SYBR/FRET Ratio For Each

| AVERAGE | STDEV | CV |
|---|---|---|
| -0.316920256 | 0.09756 | -30.76% |
| 0.041213765 | 0.142685 | 346.21% |
| 0.217521469 | 0.019551 | 8.99% |
| 0.427296953 | 0.082679 | 19.35% |
| 1.055464661 | 0.868708 | 82.31% |
| 0.452549481 | 0.177095 | 39.13% |
| -1.367118106 | 1.397345 | -102.21% |
| -1.434143469 | 2.822507 | -196.81% |
| 0.569913708 | 0.059935 | 10.52% |
| 0.859208025 | 0.359502 | 41.84% |
| 0.906173958 | 0.179264 | 19.78% |
| 0.710942578 | 0.104678 | 14.72% |
| 1.668440981 | 2.037044 | 122.09% |
| -0.585336602 | 2.499378 | -427.00% |
| -0.085837136 | 1.16384 | -1355.87% |
| 0.255082988 | 0.081538 | 31.97% |
| 0.601540319 | 0.094471 | 15.70% |
| 0.793970996 | 0.011605 | 1.46% |
| 0.978637105 | 0.050588 | 5.17% |
| 1.141584791 | 0.047842 | 4.19% |
| 1.29423797 | 0.025312 | 1.96% |
| 1.440148705 | 0.017844 | 1.24% |
| 1.576543336 | 0.017007 | 1.08% |
| 1.679726329 | 0.028221 | 1.68% |
| 1.746611967 | 0.034393 | 1.97% |
| 1.775068362 | 0.034033 | 1.92% |
| 1.785938757 | 0.032702 | 1.83% |
| 1.784898531 | 0.031461 | 1.76% |
| 1.777100876 | 0.038609 | 2.17% |
| 1.765774878 | 0.038074 | 2.16% |
| 1.745582471 | 0.039476 | 2.26% |
| 1.726137267 | 0.037917 | 2.20% |
| 1.704773664 | 0.040561 | 2.38% |
| 1.688275093 | 0.034574 | 2.05% |
| 1.668388044 | 0.028601 | 1.71% |
| 1.652394624 | 0.023102 | 1.40% |
| 1.632689657 | 0.027387 | 1.68% |
| 1.614561751 | 0.029966 | 1.86% |
| 1.59343694 | 0.033845 | 2.12% |
| 1.574570769 | 0.033747 | 2.14% |

| Cycle | Rep 1 | Rep 2 | Rep 3 |
|---|---|---|---|
| 1 | -0.128783928 | 0.031136 | 0.087781 |
| 2 | 0.298059719 | 0.238341 | 0.208352 |
| 3 | 0.249423753 | 0.18647 | 0.244531 |
| 4 | 0.397223982 | 0.180089 | 0.26025 |
| 5 | 0.189017589 | -0.057433 | 0.45552 |
| 6 | 0.33533412 | -1.956141 | 0.701017 |
| 7 | 0.648523456 | 0.887642 | -2.140368 |
| 8 | 0.699415361 | 0.683966 | 0.071457 |
| 9 | 0.972944143 | 0.551557 | 0.317321 |
| 10 | 1.045303517 | 0.520702 | 0.45339 |
| 11 | 0.550975211 | 0.398671 | 0.46048 |
| 12 | 0.781585149 | 0.639785 | 0.353172 |
| 13 | 0.624368764 | 1.446692 | 0.217491 |
| 14 | -0.296336741 | -0.170564 | 0.058407 |
| 15 | 0.83451981 | 0.636928 | -0.137196 |
| 16 | 0.923760386 | 0.937028 | -4.574337 |
| 17 | 1.186641053 | 1.150271 | 2.080109 |
| 18 | 1.217816038 | 1.324437 | 1.651063 |
| 19 | 1.388915588 | 1.480066 | 1.584742 |
| 20 | 1.552840477 | 1.660923 | 1.692445 |
| 21 | 1.74547336 | 1.818497 | 1.833652 |
| 22 | 1.877331386 | 1.920027 | 1.954225 |
| 23 | 1.960622719 | 1.97652 | 2.010566 |
| 24 | 1.986148543 | 2.000849 | 2.038052 |
| 25 | 1.995345319 | 1.992916 | 2.041548 |
| 26 | 1.983007263 | 1.979639 | 2.045576 |
| 27 | 1.970784076 | 1.95719 | 2.033447 |
| 28 | 1.946282701 | 1.948494 | 2.016883 |
| 29 | 1.920900518 | 1.926128 | 1.988827 |
| 30 | 1.90439089 | 1.914989 | 1.960554 |
| 31 | 1.885225298 | 1.886113 | 1.935953 |
| 32 | 1.875450021 | 1.865803 | 1.915509 |
| 33 | 1.856996535 | 1.835118 | 1.894878 |
| 34 | 1.845315124 | 1.818446 | 1.877351 |
| 35 | 1.823092827 | 1.795575 | 1.856834 |
| 36 | 1.812391975 | 1.777248 | 1.834425 |
| 37 | 1.798891635 | 1.749443 | 1.816111 |
| 38 | 1.791411983 | 1.732152 | 1.804392 |
| 39 | 1.778843469 | 1.710948 | 1.797652 |
| 40 | 1.765952744 | 1.701563 | 1.792703 |

FIGURE 5C-4

IdType Allele
I Replicate                                                Experimental Results for Ratio Differences

| AVERAGE | STDEV | CV | | % Difference Between Alleles |
|---|---|---|---|---|
| -0.00328897 | 0.112312 | -3414.79% | | -9535.85% |
| 0.24825079 | 0.045668 | 18.40% | | 83.40% |
| 0.22680844 | 0.03502 | 15.44% | | 4.09% |
| 0.2791876 | 0.109799 | 39.33% | | -53.05% |
| 0.19570152 | 0.256542 | 131.09% | | -439.32% |
| -0.30659681 | 1.440201 | -469.74% | | 247.60% |
| -0.20140099 | 1.683446 | -835.87% | | -578.80% |
| 0.48494597 | 0.358176 | 73.86% | | 395.73% |
| 0.61394061 | 0.332234 | 54.11% | | 7.17% |
| 0.67313197 | 0.324062 | 48.14% | | -27.64% |
| 0.47004197 | 0.076601 | 16.30% | | -92.79% |
| 0.59151403 | 0.218247 | 36.90% | | -20.19% |
| 0.76285063 | 0.626192 | 82.09% | | -118.71% |
| -0.13616456 | 0.179856 | -132.09% | | -329.87% |
| 0.4447506 | 0.513572 | 115.47% | | 119.30% |
| -0.90451639 | 3.178165 | -351.37% | | 128.20% |
| 1.47234016 | 0.526657 | 35.77% | | 59.14% |
| 1.39777202 | 0.225741 | 16.15% | | 43.20% |
| 1.48457446 | 0.097991 | 6.60% | | 34.08% |
| 1.63540303 | 0.073218 | 4.48% | | 30.20% |
| 1.79920755 | 0.047148 | 2.62% | | 28.07% |
| 1.9171945 | 0.038525 | 2.01% | | 24.88% |
| 1.98256968 | 0.025515 | 1.29% | | 20.48% |
| 2.00834993 | 0.026753 | 1.33% | | 16.36% |
| 2.00993625 | 0.027403 | 1.36% | | 13.10% |
| 2.00274073 | 0.037135 | 1.85% | | 11.37% |
| 1.98714038 | 0.040675 | 2.05% | | 10.13% |
| 1.97055327 | 0.040138 | 2.04% | | 9.42% |
| 1.94528524 | 0.037799 | 1.94% | | 8.65% |
| 1.92664448 | 0.02984 | 1.55% | | 8.35% |
| 1.90243023 | 0.029035 | 1.53% | | 8.24% |
| 1.88558727 | 0.026358 | 1.40% | | 8.46% |
| 1.86233105 | 0.030235 | 1.62% | | 8.46% |
| 1.84703733 | 0.02949 | 1.60% | | 8.60% |
| 1.8251674 | 0.030682 | 1.68% | | 8.59% |
| 1.80802161 | 0.028838 | 1.60% | | 8.61% |
| 1.7881487 | 0.034608 | 1.94% | | 8.69% |
| 1.7759854 | 0.038511 | 2.17% | | 9.09% |
| 1.76248134 | 0.045609 | 2.59% | | 9.59% |
| 1.75340599 | 0.046848 | 2.67% | | 10.20% |

FIGURE 5C-5

**EP 1 606 389 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4963663 A
- US 5411859 A
- US 5175082 A
- US 4683202 A
- US 4800159 A
- US 5468613 A
- US 5604099 A, Mullis, K.
- US 6013431 A
- WO 9102087 A
- WO 9009455 A
- WO 8910414 A
- US 6322980 B
- US 6287778 B
- WO 0011221 A
- WO 9606187 A
- WO 0194546 A2
- US 4683195 A
- US 5770716 A
- US 4883867 A, Lee
- US 1989 A
- US 5582977 A, Yue
- US 1996 A

- US 5321130 A, Yue
- US 1994 A
- US 5410030 A, Yue
- US 1995 A
- US 5436134 A, Haugland
- US 5658751 A, Yue
- US 1997 A
- US 5863753 A, Haugland
- US 1999 A
- US 5710028 A
- US 5945283 A
- US 5856092 A
- US 5846710 A
- US 5888819 A
- US 6004744 A
- WO 9216657 A
- WO 0132887 A
- US 5525711 A, Hawkins
- US 10436231 B
- US 60452481 B
- EP 04718119 A

**Non-patent literature cited in the description**

- **SOUTHERN, E. M.** *J. Mol. Biol.,* 1975, vol. 98 (3), 503-507
- **SCHUMM et al.** *American Journal of Human Genetics,* 1988, vol. 42, 143-159
- **WYMAN, A. ; WHITE, R.** *Proc. Natl. Acad. Sci, U.S.A.,* 1980, vol. 77, 6754-6758
- **NAKAMURA Y. et al.** *Science,* 1987, vol. 235, 1616-1622
- **JEFFREYS et al.** *Nature,* 1985, vol. 314, 67-73
- **JEFFREYS et al.** *Nature,* 1985, vol. 316, 76-79
- **MIZUTANI et al.** *J Hum Genet,* 2001, vol. 46, 448-55
- **SPRECHER et al.** *Biotechniques,* 1996, vol. 20, 266-76
- **HAAF et al.** *Nat. Genet.,* 1996, vol. 12, 183-5
- **WOOSTER et al.** *Nat. Genet.,* 1994, vol. 6, 152-6
- **VERGNAUD.** *Polynucleotides Res.,* 1989, vol. 17, 7623-30
- **PERTL et al.** *Hum. Genet.,* 2000, vol. 106, 45-9

- **DENG et al.** *Biotechniques,* 2000, vol. 29, 298-304
- **HOHOFF ; BRINKMANN.** *Mol. Biotechnol.,* 1999, vol. 13, 123-136
- **SHERLOCK et al.** *Ann. Hum. Genet.,* 1998, vol. 62, 9-23
- **KASAI K et al.** *Journal Forensic Science,* 1990, vol. 35 (5), 1196-1200
- **KOMHER, J. S. et al.** *Nucl. Acids. Res.,* 1989, vol. 17, 7779-7784
- **SOKOLOV, B. P.** *Nucl. Acids Res.,* 1990, vol. 18, 3671
- **SYVANEN, A.-C. et al.** *Genomics,* 1990, vol. 8, 684-692
- **KUPPUSWAMY, M.N. et al.** *Proc. Natl. Acad. Sci. (U.S.A.,* 1991, vol. 88, 1143-1147
- **PREZANT, T.R. et al.** *Hum. Mutat.,* 1992, vol. 1, 159-164
- **UGOZZOLI, L et al.** *GATA,* 1992, vol. 9, 107-112
- **NYREN, P. et al.** *Anal. Biochem.,* 1993, vol. 208, 171-175

74

- **SYVANEN, A.C. et al.** *Amer. J. Hum. Genet.,* 1993, vol. 52, 46 59
- **INNIS et al.** Molecular Cloning, A Laboratoy Manual. Academic Press, Inc, 1989
- **AUSUBEL et al.** Current Protocols in Molecular Biology. John Weley & Sons, Inc, 1997
- PCR Technology : Principles and Applications for DNA Amplificatioh. Freeman Press, 1992
- PCR Protocols : A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **WU ; WALLACE.** *Genomies,* 1989, vol. 4, 560
- **LANDEGREN et al.** *Science,* 1988, vol. 241, 1077
- **KWOH et al.** *Proc. Natl. Acad. Sci. USA,* 1989, vol. 86, 1173
- **GUATELLI et al.** *Proc. Natl. Acad. Sci. USA,* 1990, vol. 87, 1874
- **SOOKNANAN, R. ; MALEK, L.** *Bio Technology,* 1995, vol. 13, 563-65
- **LIZARDI et al.** *Nature Genet.,* 1998, vol. 19, 225-232
- **SCHWEITZER et al.** *Proc. Nat'l. Acad. Sci. USA,* 2000, vol. 97, 10113-10119
- **WILLIAMS et al.** *Nucl. Acids. Res.,* 1990, vol. 18, 6531-6535
- **WELSH ; MCCLELLAND.** *Nucl. Acids. Res.,* 1990, vol. 18, 7213-7218
- Handbook of Fluorescent Probes and Research Chemicals. Molecular Probes, Inc, 1996
- **LEE et al.** *Polynucleotides Research,* 1997, vol. 25, 2816
- **GLAZER et al.** *Current Opinion in Biotechnology,* 1997, vol. 8, 94-102
- Fluorescence Spectroscopy. Marcel Dekker, 1971
- **WHITE et al.** Fluorescence Analysis : A Practical Approach. Marcel Dekker, 1970
- **BERLMAN.** Handbook of Fluorescence Spectra of Aromatic Molecules. Academic Press, 1971
- **GRIFFITHS.** Colour and Constitution of Organic Molecules. Academic Press, 1976
- Indicators. Pergamon Press
- Handbook of Fluorescent Probes and Research Chemicals. **HAUGLAND.** Molecular Probes. 1992
- Handbook of Fluorescent Probes and Research Chemicals. **HAUGLAND, RP.** Molecular Probes. Inc. Eugene OR, 1996
- Fluorescein Hapten: An Immunological Probe
- **MESSING.** *Methods Enzymol.,* 1983, vol. 101, 20-78
- **NARANG et al.** *Meth. Enzymol.,* 1979, vol. 68, 90
- **BEAUCAGE et al.** *Tetrahedron Letters,* 1981, vol. 22, 1859-1862
- **CARUTHERS, M. H. et al.** *Methods in Enzymology,* 1988, vol. 154, 287-314
- Synthesis and Applications of DNA and RNA. Academic Press, 1987
- **BEAUCAGE ; CARUTHERS.** *Tetrahedron Letts.,* 1981, vol. 22 (20), 1859-1862
- **NEEDHAM-VANDEVANTER et al.** *Polynucleotides Res.,* 1984, vol. 12, 6159-6168
- **PEARSON ; REGNIER.** *J. Chrom.,* 1983, vol. 255, 137-149
- **MAXAM ; GILBERT.** Methods in Enzymology. Academic Press, 1980, vol. 65, 499-560
- Oligonucleotide Synthesis: A Practical Approach. IRL Press, 1984
- **W. H. A. KUIJPERS.** *Polynucleotides Research,* 1994, vol. 18 (17), 5197
- **K. L. DUEHOLM.** *J. Org. Chem.,* 1994, vol. 59, 5767-5773
- Methods in Molecular Biology. 1993, vol. 20
- overview of principles of hybridization and the strategy of polynucleotide probe assays. **TIJSSEN.** Laboratory Techniques in biochemistry and molecular biology--hybridization with polynucleotide probes. Elsevier, 1993
- **SOMERS et al.** *Biochimica et Biophysica Acta,* 1998, vol. 1379, 42-52
- **NIKIFOROV et al.** *PCR Methods and Applications,* 1994, vol. 3, 285-291
- **HIGUCHI ; OCHMAN.** *Nucleic Acids Research,* 1989, vol. 17, 5865
- **STRAUS ; ZAGURSKY.** *Biotechniques,* 1991, vol. 10, 376-384
- **LUNDBERG et al.** *Gene,* 1991, vol. 108, 1
- **HINNISDAELS et al.** *Biotechniques,* 1996, vol. 20, 186-8
- **MYERS ; GELFAND.** *Biochemistry,* 1991, vol. 30, 7661
- **STENESH ; MCGOWAN.** *Biochim Biophys Acta,* 1977, vol. 475, 32
- **CARIELLO et al.** *Polynucleotides Res,* 1991, vol. 19, 4193
- **DIAZ ; SABINO.** *Braz J. Med. Res,* 1998, vol. 31, 1239
- **CHIEN et al.** *J. Bacteoriol,* 1976, vol. 127, 1550
- **TAKAGI et al.** *Appl. Environ. Microbiol.,* 1997, vol. 63, 4504
- **JUNCOSA-GINESTA et al.** *Biotechniques,* 1994, vol. 16, 820
- **LECOMTE ; DOUBLEDAY.** *Polynucleotides Res.,* 1983, vol. 11, 7505
- **NORDSTROM et al.** *J. Biol. Chem.,* 1981, vol. 256, 3112

- **CANN et al.** *Proc Natl Acad Sci U S A,* 1998, vol. 95, 14250-5
- Current Protocols in Molecular Biology, Current Protocols. Greene Publishing Associates, Inc. and John Wiley & Sons, Inc, 1996
- **ARNHEIM ; LEVINSON.** *The Journal Of NIH Research,* 1990, vol. 3, 81-94
- **LOMELL et al.** *J. Clin. Chem,* 1989, vol. 35, 1826
- **LANDEGREN et al.** *Science,* 1988, vol. 241, 1077-1080
- **VAN BRUNT.** *Biotechnology,* 1990, vol. 8, 291-294
- **WU ; WALLACE.** *Gene,* 1989, vol. 4, 560
- **BARRINGER et al.** *Gene,* 1990, vol. 89, 117
- **SOOKNANAN ; MALEK.** *Biotechnology,* 1995, vol. 13, 563-564